(19) Europäisches Patentamt / European Patent Office / Office européen des brevets

(11) EP 2 789 347 A1

(12) **EUROPEAN PATENT APPLICATION**
published in accordance with Art. 153(4) EPC

(43) Date of publication:
**15.10.2014 Bulletin 2014/42**

(21) Application number: **12854422.8**

(22) Date of filing: **28.11.2012**

(51) Int Cl.:
***A61K 45/00*** *(2006.01)* ***A61K 31/421*** *(2006.01)*
***A61K 31/505*** *(2006.01)* ***A61P 3/04*** *(2006.01)*
***A61P 43/00*** *(2006.01)* ***C07D 239/47*** *(2006.01)*
***C07D 263/24*** *(2006.01)* ***C12N 15/09*** *(2006.01)*

(86) International application number:
**PCT/JP2012/080731**

(87) International publication number:
**WO 2013/080999 (06.06.2013 Gazette 2013/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.11.2011 JP 2011260334**

(71) Applicant: **Kowa Company, Ltd.**
**Nagoya-shi, Aichi 460-8625 (JP)**

(72) Inventors:
- **MURAKAMI, Takeshi**
  **Higashimurayama-shi**
  **Tokyo 189-0022 (JP)**
- **MURAKAMI, Kentaro**
  **Higashimurayama-shi**
  **Tokyo 189-0022 (JP)**
- **OHGIYA, Tadaaki**
  **Higashimurayama-shi**
  **Tokyo 189-0022 (JP)**
- **SHIBUYA, Kimiyuki**
  **Higashimurayama-shi**
  **Tokyo 189-0022 (JP)**

(74) Representative: **Blodig, Wolfgang**
**Wächtershäuser & Hartz**
**Patentanwaltspartnerschaft mbB**
**Weinstrasse 8**
**80333 München (DE)**

# (54) AGENT FOR INHIBITING EXPRESSION OF NPC1L1 AND/OR LIPG MRNA AND AGENT FOR PREVENTING AND/OR TREATING OBESITY

(57) Provided is an agent for treating and/or preventing obesity. An agent for suppressing expression of NPC1L1 (Niemann-Pick disease, type C1, gene-like 1) and/or LIPG (Lipase, endothelial) mRNA, and an agent for preventing and/or treating obesity, each comprising, as an active ingredient, a compound having CETP inhibitory activity, or a salt thereof, or a solvate thereof.

[Figure 1]

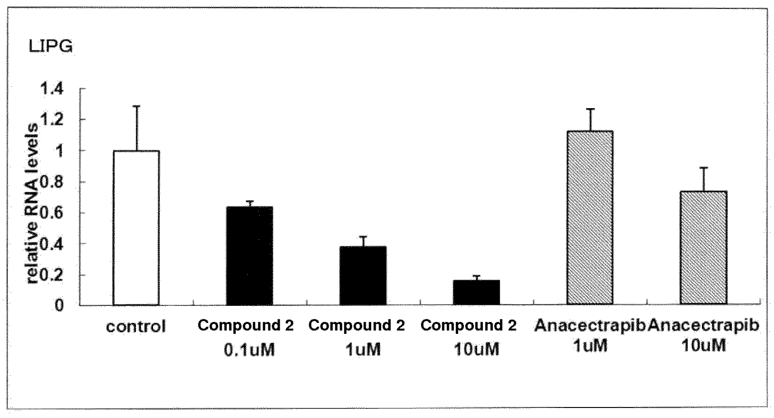


EP 2 789 347 A1

## Description

[Technical Field]

The present invention relates to an agent for preventing and/or treating obesity. In addition, the present invention relates to an agent for suppressing expression of NPC1L1 (Niemann-Pick disease, type C1, gene-like 1) and/or LIPG (Lipase, endothelial) mRNA.

[Background Art]

In recent years, with a change in Japanese dietary habits from Japanese-style food to Western-style food, Japanese people have taken in excessive energy (an increase in the intake of fat and sucrose). The lack of exercise, as well as the aforementioned intake of excessive energy, has resulted in an increase in lifestyle-related diseases such as obesity and diabetes. According to the reports by the Organization for Economic Cooperation and Development, an increase in obese people is not a problem characteristic for Japan, but a global-scale problem regarding public health, including the United States of America, European countries such as England and Austria, and other major countries such as China and India. It has been known that the life time of severe obese patients is shorter than that of healthy people with normal body weights by 8 to 10 years and that the annual medical costs of obese patients are higher than those of healthy people with normal body weights by 25% (Non Patent Literature 1). The latest data from the Centers for Disease Control and Prevention and the National Center for Health Statistics, U.S.A. have reported that "overweight" (BMI: less than 29.9) constitutes 58.0% of the adult population, "obesity" (BMI: 30-39.9) constitutes 35.7% thereof, and "excessive obesity" (BMI: 40.0 or greater) constitutes 6.3% thereof. Obesity is associated with an increase in the total amount of fat tissues (i.e. body fat), and in particular, fat tissues accumulated in abdomen, and it is a chronic disease directly causing metabolic vascular anomaly including hyperglycemia, hyperlipidemia, hypertension, fatty liver disease and various vascular disorders, and also causing many dangerous comorbidities such as inflammatory disease, progeria and several forms of cancers. Moreover, as a result of an increase in clinical grounds, the best method for regulating type 2 diabetes has been found to be weight reduction. Since suppression of obesity leads to prevention of various diseases that would be developed subsequently, it has been expected to establish an effective method for preventing obesity.

NPC1L1 (Niemann-Pick disease, type C1, gene-like 1) is expressed at a high level in lung, pancreas and liver (GenBank Accession No.: NM_013389). The NPC1L1 protein encoded by this gene is an N-glycosylated protein containing a four-amino acid motif that functions as a trans-Golgi network for plasma membrane transport signal (Non Patent Literatures 2, 3, 4 and 5). The tissue distribution of such NPC1L1 protein is restricted, and this protein is abundantly present in the stomach and bowl. In addition, this protein has homology of 42% at the amino acid sequence level with human NPC1 (Non Patent Literature 6). The NPC1 protein has cholesterol transport ability. Thus, it has been known that if this protein were lost, Niemann-Pick disease would be developed, which is congenital lipid metabolism abnormality and causes accumulation of low-density lipoprotein (LDL)-derived non-esterified cholesterol in lysosome (Non Patent Literature 7). Moreover, it has also been reported that NPC1L1 is a protein that is a key for cholesterol absorption via small intestine and has an effect on a decrease in the LDL value in plasma, and that the NPC1L1 is also a target protein of ezetimibe, and the polymorphism of this gene leads to a difference in reactivity with ezetimibe (Non Patent Literature 8). Furthermore, NPC1L1 has been known to have various biological activities. It has been reported that, when NPC1L1-knockout mice were fed with high-fat diet without cholesterol, an increase in body weight was not observed in the knockout mice, in comparison with wild-type mice which had no change in eating and fat absorption via small intestine (Non Patent Literature 9). Accordingly, it is expected that NPC1L1 can be used to develop an effective anti-obesity agent based on the suppression of the expression of this gene.

LIPG (Lipase, endothelial) is a gene encoding endothelial lipase (EL), and it has been known that the polymorphism of this gene is associated with plasma HDL-C level (GenBank Accession No.: NM_006033). It has also been known that endothelial lipase is an enzyme which HDL or other lipoproteins are hydroxylated with, and is widely distributed in a body (Non Patent Literatures 10 and 11). The mRNA level of endothelial lipase is accelerated in HUVEC and coronary artery endothelial cells by inflammatory cytokine assumed to be involved in vascular diseases or by blood vessel remodeling. In short, it has been suggested that endothelial lipase be complicatedly involved in lipid metabolism in blood vessels, and that such endothelial lipase be also involved in vascular diseases such as atheroma (Non Patent Literature 12). A method which utilizes the antisense of this gene as a therapeutic agent for atheroma has also been reported (Patent Literature 6). It has been found that, in mice in which endothelial lipase has been knocked out, the plasma HDL-C level increases, and the levels of apolipoprotein A-1 and E increase. Thus, with the results that human LIPG polymorphism correlates with HDL-C, it has been demonstrated that endothelial lipase is a principal factor regarding the concentration, form and metabolism of HDL in human (Non Patent Literatures 13 and 14). Endothelial lipase has been known to have various biological activities. It has been reported that the value of endothelial lipase in plasma positively

correlates with HDL-C value, BMI, waist circumference, insulin resistance and inflammatory marker, and that it promotes metabolic syndrome (Non Patent Literature 15). In addition, in mice in which EL has been knocked out, the survival rate increases after injection of bacterial toxin, lipopolysaccharide (LPS), and thus, it is expected that anti-inflammatory action can be obtained by suppressing expression level of EL (Non Patent Literature 16).

Cholesteryl ester transfer protein (CETP) is an extremely highly hydrophobic protein which transfers cholesteryl ester from HDL cholesterol to LDL cholesterol, very low density lipoprotein (VLDL) cholesterol or the like. It is possible to increase HDL cholesterol by inhibiting such transfer by CETP. Hence, it is expected that a CETP inhibitor will be used as a therapeutic agent for diseases such as lipid disorder (hyperlipidemia), arteriosclerosis, atherosclerosis, peripheral vascular disease, hyper-LDL cholesterolemia, hypo-HDL cholesterolemia, hypercholesterolemia, hypertriglyceridemia, familial hypercholesterolemia, cardiovascular disturbance, angina pectoris, ischemia, cardiac ischemia, thrombosis, myocardial infarction, reperfusion injury, angioplasty restenosis, or hypertension. As compounds which inhibit such cholesteryl ester transfer protein (CETP) activity, Torcetrapib (Patent Literature 3), Anacetrapib (Patent Literature 4), Dalcetrapib (Patent Literature 5) and the like have been known, for example.

On the other hand, it has not yet been known that the CETP inhibitor is useful for suppressing expression of NPC1L1 or LIPG mRNA, or preventing and/or treating obesity.

[Citation List]

[Patent Literature]

[Patent Literature 1] JP-A-2004-505637
[Patent Literature 2] International Publication No. WO2007/037299
[Patent Literature 3] International Publication No. WO2000/017164
[Patent Literature 4] International Publication No. WO2006/014357
[Patent Literature 5] International Publication No. WO1998/035937
[Patent Literature 6] International Publication No. WO2004/055162

[Non Patent Literature]

[Non Patent Literature 1] OECD, the Organization for Economic Cooperation and Development, "Himan to Yobo no Keizaigaku (Economics for Obesity and Prevention Thereof): FIT, NOT FAT," [online], September 23, 2010, [searched on March 18, 2011] Internet, <URL: http://www.oecdtokyo2.org/pdf/theme_pdf/health_pdf/20100923fit.pdf>
[Non Patent Literature 2] EMBO J., 12: 2219-28 (1993)
[Non Patent Literature 3] J. Cell. Biol., 120: 1123-35 (1993)
[Non Patent Literature 4] J. Cell. Biol., 125: 253-268 (1994)
[Non Patent Literature 5] Science, 272: 227-34 (1996)
[Non Patent Literature 6] Science, 277: 228-31 (1997)
[Non Patent Literature 7] Genomics, 65 (2): 137 (2000)
[Non Patent Literature 8] J. Atheroscler. Thromb., 17 (4): 356 (2010)
[Non Patent Literature 9] J. Lipid Res., 51 (10) 3024-33 (2010)
[Non Patent Literature 10] J. Biol. Chem., 274, 14170 (1999)
[Non Patent Literature 11] Nat. Genet., 21, 424 (1999)
[Non Patent Literature 12] Biochem. Biophys. Res. Commun., 272, 90 (2000)
[Non Patent Literature 13] Proc Natl Acad Sci USA 2003, 100 (5): 2748
[Non Patent Literature 14] J Clin Invest 2003, 111 (3): 347
[Non Patent Literature 15] Atherosclerosis Supplement, Vol. 10, Issue 2, Abs: 612 (2009)
[Non Patent Literature 16] J. Lipid Res., Vol. 52 (1):57 (2011)

[Summary of Invention]

[Technical Problem]

It is an object of the present invention to provide an agent for preventing and/or treating obesity. In addition, it

is another object of the present invention to provide an agent for suppressing expression of NPC1L1 mRNA and/or LIPG mRNA.

[Solution to Problem]

As a result of intensive studies directed towards achieving the aforementioned objects, the present inventors have found that a compound represented by the following general formula (I) or general formula (II), or a salt thereof, or a solvate thereof, which has been known as an inhibitor of cholesteryl ester transfer protein (CETP), has an excellent action to suppress the expression of NPC1L1 and LIPG mRNAs, and that these substances also have action to suppress NPC1L1 and LIPG protein production. As a result, the inventors have found that these substances are also useful as agents for preventing and/or treating obesity, thereby completing the present invention. Specifically, the present invention includes the following examples [1] to [40].

[1] An agent for suppressing expression of NPC1L1 and/or LIPG mRNA, comprising, as an active ingredient, a compound having activity of inhibiting cholesteryl ester transfer protein, or a salt thereof, or a solvate thereof.

[2] The agent for suppressing expression according to [1] above, wherein the compound having activity of inhibiting cholesteryl ester transfer protein, or a salt thereof, or a solvate thereof is a compound represented by the following formula (I) or (II), or a salt thereof, or a solvate thereof:

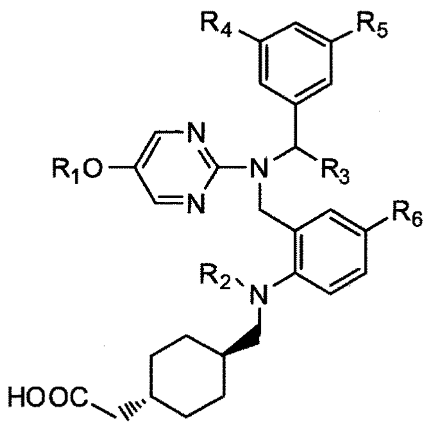

(I)

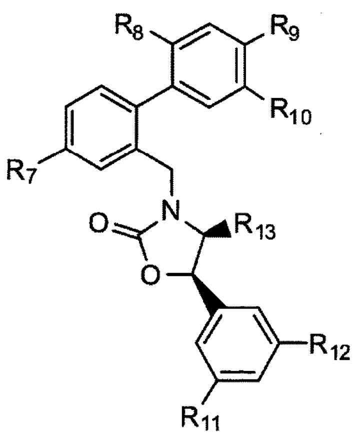

(II)

wherein, in the formula (I),

$R_1$ represents a $C_{1-6}$ alkylthio $C_{1-6}$ alkyl group, a $C_{1-6}$ alkylsulfinyl $C_{1-6}$ alkyl group or a $C_{1-6}$ alkylsulfonyl $C_{1-6}$ alkyl group,
$R_2$ represents a hydrogen atom or an optionally halogen atom-substituted $C_{1-6}$ alkyl group,
$R_3$ represents an optionally halogen atom-substituted $C_{1-6}$ alkyl group,
$R_4$ and $R_5$, which are the same or different, each represent a hydrogen atom, a halogen atom, an optionally halogen atom-substituted $C_{1-6}$ alkyl group, an optionally halogen atom-substituted $C_{1-6}$ alkoxy group, or a cyano group, and
$R_6$ represents a hydrogen atom, a halogen atom, an optionally halogen atom-substituted $C_{1-6}$ alkyl group, or an optionally halogen atom-substituted $C_{1-6}$ alkoxy group, and
in the formula (II),
$R_7$, $R_8$, $R_9$, $R_{10}$, $R_{11}$, $R_{12}$ and $R_{13}$, which are the same or different, each represent a hydrogen atom, a hydroxy group, a halogen atom, an optionally halogen atom-substituted $C_{1-6}$ alkyl group, or an optionally halogen atom-substituted $C_{1-6}$ alkoxy group.

[3] The agent for suppressing expression according to [1] above, comprising, as an active ingredient, at least one compound selected from the group consisting of the following compounds, or a salt thereof, or a solvate thereof: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

[4] The agent for suppressing expression according to [1] above, comprising, as an active ingredient, (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one, or a salt thereof, or a solvate thereof.

[5] An agent for suppressing NPC1L1 and/or LIPG protein production, comprising, as an active ingredient, a compound having activity of inhibiting cholesteryl ester transfer protein, or a salt thereof, or a solvate thereof.

[6] The agent for suppressing protein production according to [5] above, wherein the compound having activity of inhibiting cholesteryl ester transfer protein, or a salt thereof, or a solvate thereof is the compound represented by the above formula (I) or (II), or a salt thereof, or a solvate thereof.

[7] The agent for suppressing protein production according to [5] above, comprising, as an active ingredient, at least one compound selected from the group consisting of the following compounds, or a salt thereof, or a solvate thereof: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

[8] The agent for suppressing protein production according to [5] above, comprising, as an active ingredient, (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one, or a salt thereof, or a solvate thereof.

[9] An agent for preventing and/or treating obesity, comprising, as an active ingredient, a compound represented by the following formula (I), or a salt thereof, or a solvate thereof:

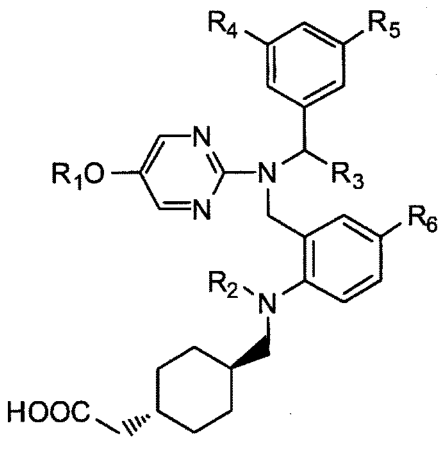

(I)

wherein $R_1$ to $R_6$ are the same as those described above.

[10] The agent for preventing and/or treating obesity according to [9] above, comprising, as an active ingredient, at least one compound selected from the group consisting of the following compounds, or a salt thereof, or a solvate thereof:

trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

[11] A compound having activity of inhibiting cholesteryl ester transfer protein, or a salt thereof, or a solvate thereof,

for suppressing expression of NPC1L1 and/or LIPG mRNA.

[12] The compound according to [11] above, or a salt thereof, or a solvate thereof, wherein the compound having activity of inhibiting cholesteryl ester transfer protein, or a salt thereof, or a solvate thereof is the compound represented by the above formula (I) or (II), or a salt thereof, or a solvate thereof.

[13] The compound according to [11] above, or a salt thereof, or a solvate thereof, wherein the compound having activity of inhibiting cholesteryl ester transfer protein is at least one compound selected from the group consisting of: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

[14] The compound according to [11] above, or a salt thereof, or a solvate thereof, wherein the compound having activity of inhibiting cholesteryl ester transfer protein is (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one.

[15] A compound having activity of inhibiting cholesteryl ester transfer protein, or a salt thereof, or a solvate thereof, for suppressing NPC1L1 and/or LIPG protein production.

[16] The compound according to [15] above, or a salt thereof, or a solvate thereof, wherein the compound having activity of inhibiting cholesteryl ester transfer protein, or a salt thereof, or a solvate thereof is the compound represented by the above formula (I) or (II), or a salt thereof, or a solvate thereof.

[17] The compound according to [15] above, or a salt thereof, or a solvate thereof, wherein the compound having activity of inhibiting cholesteryl ester transfer protein is at least one compound selected from the group consisting of: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}-acetic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

[18] The compound according to [15] above, or a salt thereof, or a solvate thereof, wherein the compound having activity of inhibiting cholesteryl ester transfer protein is (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one.

[19] The compound represented by the above formula (I), or a salt thereof, or a solvate thereof, for preventing and/or treating obesity.

[20] The compound according to [19] above, or a salt thereof, or a solvate thereof, wherein the compound having activity of inhibiting cholesteryl ester transfer protein is at least one compound selected from the group consisting of the following compounds, or a salt thereof, or a solvate thereof: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

[21] Use of a compound having activity of inhibiting cholesteryl ester transfer protein, or a salt thereof, or a solvate thereof, for manufacturing an agent for suppressing expression of NPC1L1 and/or LIPG mRNA.

[22] The use according to [21] above, wherein the compound having activity of inhibiting cholesteryl ester transfer protein, or a salt thereof, or a solvate thereof is the compound represented by the above formula (I) or (II), or a salt thereof, or a solvate thereof.

[23] The use according to [21] above, wherein the compound having activity of inhibiting cholesteryl ester transfer protein is at least one compound selected from the group consisting of:

trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

[24] The use according to [21] above, wherein the compound having activity of inhibiting cholesteryl ester transfer protein is (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one.

[25] Use of a compound having activity of inhibiting cholesteryl ester transfer protein, or a salt thereof, or a solvate

thereof, for manufacturing an agent for suppressing NPC1L1 and/or LIPG protein production.
[26] The use according to [25] above, wherein the compound having activity of inhibiting cholesteryl ester transfer protein, or a salt thereof, or a solvate thereof is the compound represented by the above formula (I) or (II), or a salt thereof, or a solvate thereof.
[27] The use according to [25] above, wherein the compound having activity of inhibiting cholesteryl ester transfer protein is at least one compound selected from the group consisting of:

trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

[28] The use according to [25] above, wherein the compound having activity of inhibiting cholesteryl ester transfer protein is (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one.
[29] Use of the compound represented by the above formula (I), or a salt thereof, or a solvate thereof, for manufacturing an agent for preventing and/or treating obesity.
[30] The use according to [29] above, wherein the compound represented by the formula (I) is at least one compound selected from the group consisting of:

trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

[31] A method for suppressing expression of NPC1L1 and/or LIPG mRNA, comprising administering a compound having activity of inhibiting cholesteryl ester transfer protein, or a salt thereof, or a solvate thereof.
[32] The method for suppressing expression according to [31] above, wherein the compound having activity of inhibiting cholesteryl ester transfer protein, or a salt thereof, or a solvate thereof is a compound represented by the above formula (I) or (II), or a salt thereof, or a solvate thereof.
[33] The method for suppressing expression according to [31] above, wherein the method comprises administering at least one compound selected from the group consisting of the following compounds, or a salt thereof, or a solvate thereof:

trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

[34] The method for suppressing expression according to [31] above, wherein the method comprises administering (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one, or a salt thereof, or a solvate thereof.
[35] A method for suppressing NPC1L1 and/or LIPG protein production, comprising administering a compound having activity of inhibiting cholesteryl ester transfer protein, or a salt thereof, or a solvate thereof.
[36] The method for suppressing expression according to [35] above, wherein the compound having activity of inhibiting cholesteryl ester transfer protein, or a salt thereof, or a solvate thereof is a compound represented by the above formula (I) or (II), or a salt thereof, or a solvate thereof.
[37] The method for suppressing protein production according to [35] above, wherein the method comprises administering at least one compound selected from the group consisting of the following compounds, or a salt thereof, or a solvate thereof:

trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

[38] The method for suppressing protein production according to [35] above, wherein the method comprises administering (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one, or a salt thereof, or a solvate thereof.
[39] A method for preventing and/or treating obesity, comprising administering the compound represented by the above formula (I), or a salt thereof, or a solvate thereof.
[40] The method for preventing and/or treating obesity according to [39] above, wherein the method comprises administering at least one compound selected from the group consisting of the following compounds, or a salt thereof, or a solvate thereof:

trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

The compound (III), trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and the compound (IV), (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one, are more preferable as specific compounds represented by the general formula (I) or (II).

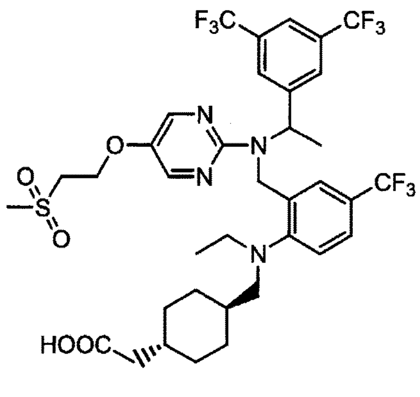

(III)

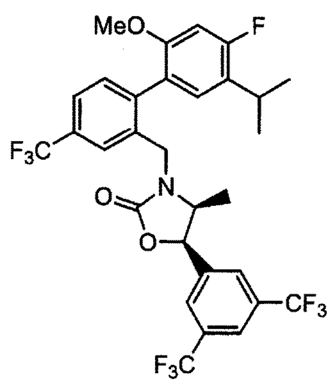

(IV)

[Advantageous Effects of Invention]

As specifically described in the after-mentioned Examples, the compounds having CETP inhibitory activity according to the present invention have a strong action to suppress the expression of NPC1L1 and LIPG mRNAs. In addition, since these compounds have an action to suppress NPC1L1 and LIPG protein production, they can be effectively used for preventing and/or treating obesity.

[Brief Description of Drawings]

[Figure 1] Figure 1 is a view showing the relative LIPG mRNA expression levels in cells, to which Compound 2 and Anacetrapib were each added.

[Figure 2] Figure 2 is a view showing the relative NPC1L1 mRNA expression levels in cells, to which Compound 2 and Anacetrapib were each added.

[Description of Embodiments]

The active ingredient of the pharmaceutical of the present invention is a compound having activity of inhibiting CETP. Examples of such a compound include the compounds described in Patent Literatures 3 to 5, such as the above-mentioned Torcetrapib, Anacetrapib and Dalcetrapib, and the compounds described in WO99/041237, WO00/018721, WO05/097805, WO06/073973, WO08/079427, WO08/129951, WO08/156718, WO09/007259, Bioorganic & Medicinal Chemistry Letters, 6, 1951-1954 (1996), Bioorganic & Medicinal Chemistry Letters, 15, 3611-3614 (2005), Bioorganic & Medicinal Chemistry Letters, 17, 2608-2613 (2007), etc. Preferred examples of the aforementioned compound include the compound represented by the general formula (I) or (II), or a salt thereof, or a solvate thereof.

The compound represented by the general formula (I) is described in WO08/129951 (Compound 1 in Example 45), and it can be produced by the method described in the same publication as described above. In addition, the compound represented by the general formula (II) and including Anacetrapib is described in WO06/014357 (Anacetrapib in Example 73), and it can be produced by the method described in the same publication as described above.

In the present description, the "$C_{1-6}$ alkyl" in the phrase "a $C_{1-6}$ alkylthio $C_{1-6}$ alkyl group, a $C_{1-6}$ alkylsulfinyl $C_{1-6}$ alkyl group or a $C_{1-6}$ alkylsulfonyl $C_{1-6}$ alkyl group" means a linear or branched alkyl having 1 to 6 carbon atoms. Such $C_{1-6}$ alkyl is preferably "$C_{1-3}$ alkyl" and more preferably "methyl or ethyl". Examples of the "$C_1$-$C_6$ alkyl" include methyl, ethyl, n-propyl, isopropyl, n-butyl, isobutyl, sec-butyl, tert-butyl, n-pentyl, isopentyl, neopentyl, 2-methylbutyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 1-ethylbutyl, and 2-ethylbutyl.

In the present description, examples of the "$C_1$-$C_6$ alkylthio" in the phrase "$C_{1-6}$ alkylthio $C_{1-6}$ alkyl group" include methylthio, ethylthio, n-propylthio, isopropylthio, n-butylthio, isobutylthio, sec-butylthio, tert-butylthio, n-pentylthio, isopentylthio, neopentylthio, 2-methylbutylthio, 1-ethylpropylthio, n-hexylthio, isohexylthio, 3-methylpentylthio, 2-methylpentylthio, 1-methylpentylthio, 3,3-dimethylbutylthio, 2,2-dimethylbutylthio, 1,1-dimethylbutylthio, 1,2-dimethylbutylthio, 1,3-dimethylbutylthio, 2,3-dimethylbutylthio, 1-ethylbutylthio, and 2-ethylbutylthio.

In the present description, examples of the "$C_1$-$C_6$ alkylsulfinyl" in the phrase "$C_{1-6}$ alkylsulfinyl $C_{1-6}$ alkyl group" include methylsulfinyl, ethylsulfinyl, n-propylsulfinyl, isopropylsulfinyl, n-butylsulfinyl, isobutylsulfinyl, sec-butylsulfinyl, tert-butylsulfinyl, n-pentylsulfinyl, isopentylsulfinyl, neopentylsulfinyl, 2-methylbutylsulfinyl, 1-ethylpropylsulfinyl, n-hexylsulfinyl, isohexylsulfinyl, 3-methylpentylsulfinyl, 2-methylpentylsulfinyl, 1-methylpentylsulfinyl, 3,3-dimethylbutylsulfinyl, 2,2-dimethylbutylsulfinyl, 1,1-dimethylbutylsulfinyl, 1,2-dimethylbutylsulfinyl, 1,3-dimethylbutylsulfinyl, 2,3-dimethylbutylsulfinyl, 1-ethylbutylsulfinyl, and 2-ethylbutylsulfinyl.

In the present description, examples of the "$C_1$-$C_6$ alkylsulfonyl" in the phrase "$C_{1-6}$ alkylsulfonyl $C_{1-6}$ alkyl group" include methylsulfonyl, ethylsulfonyl, n-propylsulfonyl, isopropylsulfonyl, n-butylsulfonyl, isobutylsulfonyl, sec-butylsulfonyl, tert-butylsulfonyl, n-pentylsulfonyl, isopentylsulfonyl, neopentylsulfonyl, 2-methylbutylsulfonyl, 1-ethylpropylsulfonyl, n-hexylsulfonyl, isohexylsulfonyl, 3-methylpentylsulfonyl, 2-methylpentylsulfonyl, 1-methylpentylsulfonyl, 3,3-dimethylbutylsulfonyl, 2,2-dimethylbutylsulfonyl, 1,1-dimethylbutylsulfonyl, 1,2-dimethylbutylsulfonyl, 1,3-dimethylbutylsulfonyl, 2,3-dimethylbutylsulfonyl, 1-ethylbutylsulfonyl, and 2-ethylbutylsulfonyl.

In the present description, examples of the "halogen atom" include fluorine, chlorine, bromine, and iodine.

In the present description, the "optionally halogen atom-substituted $C_{1-6}$ alkyl group" means a linear or branched alkyl group having 1 to 6 carbon atoms, which is optionally substituted with a halogen atom. Examples of the optionally halogen atom-substituted $C_{1-6}$ alkyl group include methyl, ethyl, n-propyl, isopropyl, n-butyl, isobutyl, sec-butyl, tert-butyl, n-pentyl, isopentyl, neopentyl, 2-methylbutyl, 1-ethylpropyl, n-hexyl, isohexyl, trifluoromethyl, trichloromethyl, and trifluoroethyl. It is preferably an "optionally halogen atom-substituted $C_{1-3}$ alkyl group", and more preferably an "optionally halogen atom-substituted methyl or ethyl group".

In the present description, the "optionally halogen atom-substituted $C_{1-6}$ alkoxy group" means a linear or branched alkoxy group having 1 to 6 carbon atoms, which is optionally substituted with a halogen atom. Examples of the optionally halogen atom-substituted $C_{1-6}$ alkoxy group include methoxy, ethoxy, propoxy, isopropoxy, butoxy, isobutoxy, sec-butoxy, pentyloxy, hexyloxy, trifluoromethoxy, and trifluoroethoxy. It is preferably an "optionally halogen atom-substituted $C_{1-3}$ alkoxy group", and more preferably an "optionally halogen atom-substituted methoxy or ethoxy group".

As compounds represented by the general formula (I) or (II), any given stereoisomers or any given mixtures thereof, such as optically pure isomers or any given mixtures of the aforementioned isomers, racemic bodies, and any given geometric isomers or any given mixtures of the geometric isomers, may be used.

Examples of the stereoisomers of the compound represented by the general formula (I) include compounds represented by the following general formulae (I-1) and (I-2):

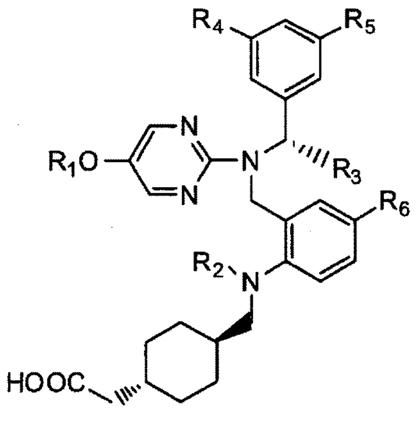

(I-1)

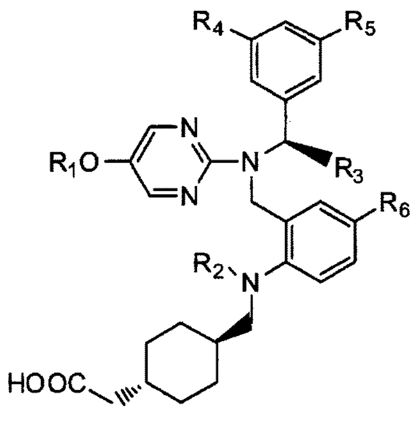

(I-2)

wherein $R_1$, $R_2$, $R_3$, $R_4$, $R_5$ and $R_6$ are the same as those described above.

The compound represented by the general formula (I) or (II) includes all of compounds which are metabolized *in vivo* and are converted to the compound represented by the general formula (I) or (II), namely, prodrugs. Examples of a group capable of forming a prodrug of the compound represented by the general formula (I) or (II) include the groups described in "Progress in Medicine," Lifescience Medica, 1985, Vol. 5, pp. 2157-2161, and the groups described in "Iyakuhin no Kaihatsu (Development of Medicines)" published by Hirokawa Shoten, 1990, Vol. 7, Bunshi Sekkei (Molecular Design), pp. 163-198.

Examples of the preferred compound in the present invention include:

trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid (Compound 1);
(S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid (Compound 2);
(R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid (Compound 3); and
(4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one (Compound 4). However, the scope of the present invention is not limited to these compounds.

Examples of a salt of the compound represented by the general formula (I) or (II) include acid-added salts and base-added salts. The salt is not particularly limited, as long as it is a pharmaceutically acceptable salt. Examples of the acid-added salts include: acid-added salts with inorganic acids, such as hydrochloride, hydrobromide, hydroiodide, sulfate, nitrate or phosphate; and acid-added salts with organic acids, such as benzoate, methanesulfonate, ethanesulfonate, benzenesulfonate, p-toluenesulfonate, maleate, fumarate, tartrate, citrate, or acetate. Examples of the base-added salts include: base-added salts with metals, such as sodium salts, potassium salts, lithium salts, calcium salts, or magnesium salts; amine salts such as ammonia, trimethylamine, triethylamine, pyridine, collidine, or lutidine; and base-added salts with organic bases such as lysine, or arginine.

Examples of a solvent used to form a solvate of the compound represented by the general formula (I) or (II) or a salt thereof include water and physiologically acceptable organic solvents such as ethanol, hexane or ethyl acetate, but the examples are not limited thereto. An example of the active ingredient of the pharmaceutical of the present invention is a hydrate, but the examples are not limited thereto.

The compound represented by the general formula (I-1) (e.g. Compound 2) or the general formula (I-2) (e.g. Compound 3), which is an enantiomer of the compound represented by the general formula (I) (e.g. Compound 1), can be produced from the compound represented by the general formula (I) (e.g. Compound 1) by a method using a chiral column, or from a derivative of the compound represented by the general formula (I) (e.g. Compound 1) by a method fractionation according to preferential crystallization or the like and then induction to the compound represented by the general formula (I-1) (e.g. Compound 2) or the general formula (I-2) (e.g. Compound 3), etc.

The pharmaceutical of the present invention includes, as an active ingredient, the above described compound represented by the general formula (I), or a salt thereof, or a solvate thereof. The aforementioned active ingredient may directly be administered as the pharmaceutical of the present invention. However, preferably, the active ingredient may be prepared into and administered as a pharmaceutical composition for oral administration or parenteral administration,

which can be produced according to a method well known to a person skilled in the art. Examples of a pharmaceutical composition suitable for oral administration include a tablet, a capsule, a powder, a fine granule, a granule, a liquid, and syrup. Examples of a pharmaceutical composition suitable for parenteral administration include injections such as intravenous injection or intramuscular injection, drops, suppository, inhalant, eye drops, nasal drops, a transdermal absorption agent and a transmucosal absorption agent, but the examples are not limited thereto.

The above described pharmaceutical composition can be produced by adding pharmacologically or pharmaceutically acceptable additives. Examples of such pharmacologically or pharmaceutically acceptable additives include an excipient, a binder, a thickener, a disintegrator, a surfactant, a lubricant, a dispersant, a buffer, a preservative, a corrigent, a perfume, a coating agent and a diluent, but the examples are not limited thereto.

The dosage of the pharmaceutical of the present invention is not particularly limited, and it can be selected, as appropriate, depending on the type of disease, preventive or therapeutic purpose, the type of an active ingredient, etc. Further, the dosage can be increased or decreased, as appropriate, depending on generally considerable various factors, such as the body weight and age of a patient, symptoms, and administration route. For example, in the case of oral administration, the compound according to the present invention can be used in the weight range of approximately 0.1 to 500 mg per adult per day. However, such dosage can be appropriately selected by a person skilled in the art, and it is not limited to the aforementioned range.

[Examples]

Hereinafter, the present invention will be further described in the following Examples. However, these examples are not intended to limit the scope of the present invention. It is to be noted that abbreviations used in the below-mentioned Examples have the following meanings.

s: Singlet
d: Doublet
t: Triplet
q: Quartet
m: Multiplet
br: Broad
J: Coupling constant
Hz: Hertz
$CDCl_3$: Deuterated chloroform
$^1H$-NMR: Proton nuclear magnetic resonance

Production Example 1

A compound produced according to the method disclosed in Example 45 of International Publication No. WO2008/129951 was used as Compound 1. In addition, as Compound 2 and Compound 3 which are both enantiomers of the Compound 1, those isolated from the Compound 1 by fractionation using a chiral column under the following conditions were used.

Column: CHIRALCEL OD-H (4.6 x 250 mm)
Flow rate: 1.0 mL/min
Detector: UV 242 nm
Temp.: 40°C
Mobile phase: hexane/EtOH/TFA = 90/10/0.1
Retention time: (R)-(+)-form: 21.3 min; and (S)-(-)-form: 23.7 min

Compound 2:

(S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid

$^1H$-NMR ($CDCl_3$) $\delta$: 0.80-0.96 (7H, m), 1.38 (1H, m), 1.47 (3H, d, J = 7.1 Hz), 1.65-1.77 (5H, m), 2.19 (2H, d, J = 6.8 Hz), 2.72 (1H, m), 2.81-2.91 (3H, m), 3.08 (3H, s), 3.45 (2H, t, J = 5.4 Hz), 4.44 (2H, t, J = 5.4 Hz), 4.62 (1H, d, J = 17.1 Hz), 4.86 (1H, d, J = 17.1 Hz), 6.21 (1H, q, J = 7.1 Hz), 7.13 (1H, d, J = 8.3 Hz), 7.19 (1H, s), 7.38 (1H, d, J = 8.3 Hz), 7.71 (1H, s), 7.73 (2H, s), 8.15 (2H, s).
$[\alpha]_D^{20}$ = -46.68 (c = 1.0, $CHCl_3$)

Compound 3:

(R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid

$^1$H-NMR ($CDCl_3$) $\delta$: the same as Compound 2
$[\alpha]_D^{20}$=+48.92 (c = 1.0, $CHCl_3$)

Production Example 2: Production of substantially optically pure (S)-form Compound 2 according to preferential crystallization

An outline of a method for producing a substantially optically pure (S)-form Compound 2 according to preferential crystallization, which was carried out by the present inventors, will be shown as Scheme 1 below.

The absolute configuration of each compound was determined on the basis of the absolute configuration of (R)-1-bromo-1-[3,5-bis(trifluoromethyl)phenyl]ethane confirmed by Step 1.

Moreover, the optical purity of the (S)-form Compound 2 obtained in Step 6, (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, was determined by chiral HPLC analysis under the conditions described in the above described Production Example 1.

Furthermore, the optical purity of each of 1-bromo-1-[3,5-bis(trifluoromethyl)phenyl]ethane obtained in Step 1 and trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid benzyl ester obtained in Steps 4 and 5 was determined by chiral HPLC analysis under the following conditions.

Conditions for chiral HPLC analysis of 1-bromo-1-[3,5-bis(trifluoromethyl)phenyl]ethane

Column: CHIRALPAK AS-RH
Mobile phase: ethanol/water = 60/40
Flow rate: 0.5 mL/min
Column temperature: 25°C
Detection wavelength: 220 nm
Retention time: first peak/21.8 min ((R)-form), second peak/26.0 min ((S)-form)

Conditions for chiral HPLC analysis of trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid benzyl ester

Column: CHIRALCEL OD-H
Mobile phase: hexane/ethanol = 80/20
Flow rate: 1.0 mL/min
Column temperature: 40°C
Detection wavelength: 242 nm
Retention time: first peak/11.3 min ((R)-form), second peak/13.0 min ((S)-form)

Scheme 1

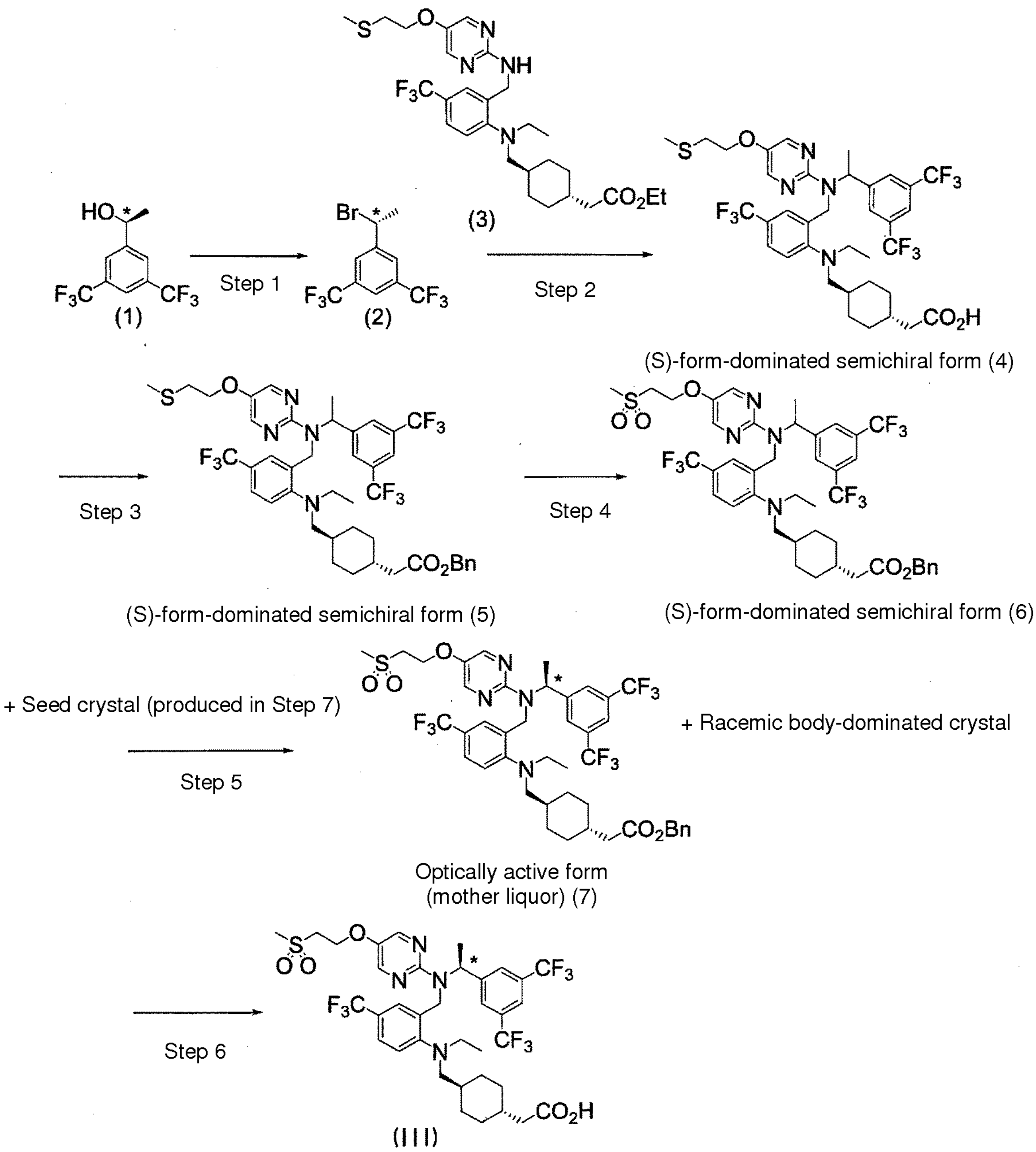


(1)
(2)
(3)
(S)-form-dominated semichiral form (4)
(S)-form-dominated semichiral form (5)
(S)-form-dominated semichiral form (6)
Optically active form (mother liquor) (7)
(III)

(In the scheme, Et indicates an ethyl group and Bn indicates a benzyl group.)

Step 1: Production of (R)-1-bromo-1-[3,5-bis(trifluoromethyl)phenyl]ethane

(R)-1-bromo-1-[3,5-bis(trifluoromethyl)phenyl]ethane was produced by the method described in 1-(a) below, and the absolute configuration thereof was then confirmed as follows. Specifically, the obtained (R)-1-bromo-1-[3,5-bis(trifluoromethyl)phenyl]ethane was induced to (S)-1-[3,5-bis(trifluoromethyl)phenyl]ethylamine. Thereafter, the absolute configuration of the thus induced compound was confirmed by comparing the compound with a commercially available standard product of (S)-1-[3,5-bis(trifluoromethyl)phenyl]ethylamine whose absolute configuration had been known, in terms of the sign in the measured value of specific optical rotation.

Moreover, (R)-1-bromo-1-[3,5-bis(trifluoromethyl)phenyl]ethane was also produced by the method described in 1-(b) below, separately.

1-(a): Production 1 of (R)-1-bromo-1-[3,5-bis(trifluoromethyl)phenyl]ethane

Under an argon atmosphere, 1,2-dibromo-1,1,2,2-tetrachloroethane (7.57 g, 23.2 mmol) was dissolved in toluene (12.5 mL), and triphenylphosphine (6.1g, 23.2 mmol) was then added to the obtained solution at 0°C, followed by stirring for 30 minutes. Thereafter, a toluene solution (12.5 mL) containing (S)-1-[3,5-bis(trifluoromethyl)phenyl]ethanol (1) (5.0 g, 19.4 mmol, > 99.5% ee) was added dropwise to the resulting solution at 0°C over 10 or more minutes. Thereafter, the temperature of the mixed solution was increased to a room temperature, and the solution was then stirred at the same temperature as described above for 1 hour. Subsequently, n-hexane (25 mL) was added to the reaction solution, followed by filtration with Celite. The filtrate was successively washed with water, saturated sodium bicarbonate water, and saturated saline solution, and it was then dried over sodium sulfate. Thereafter, it was distilled away under a reduced pressure. The obtained residue was subjected to distillation under a reduced pressure (56°C, 0.7 mmHg) to obtain 5.52g of (R)-1-bromo-1-[3,5-bis(trifluoromethyl)phenyl]ethane (2) in the form of a colorless oily product (yield: 88.6%).

Chiral HPLC analysis: optical purity > 99.5% ee (main peak: fist peak), invert ratio ≥ 99%
$[\alpha]_D^{25}$+59.1 (c = 1.03, $CHCl_3$)
$^1$H-NMR ($CDCl_3$) δ: 2.08 (3H, d, J = 7.1 Hz), 5.21 (1H, q, J = 7.1 Hz), 7.81 (1H, s), 7.87 (2H, s)

Confirmation of absolute configuration of (R)-1-bromo-1-[3,5-bis(trifluoromethyl)phenyl]ethane

Sodium azide (64.4 mg, 0.990 mmol) was added to an N,N-dimethylformamide solution (1 mL) containing (R)-1-bromo-1-[3,5-bis(trifluoromethyl)phenyl]ethane (2) (106 mg, 0.336 mmol, 99% ee) obtained in 1-(a) above, and the obtained mixture was then stirred at -18°C to -15°C for 4 hours. Thereafter, the reaction solution was extracted with ethyl acetate/n-hexane (1 : 1) and water. The organic layer was washed with saturated saline solution, and was then dried over anhydrous sodium sulfate, followed by concentration under a reduced pressure, to obtain 111.5 mg of 1-[3,5-bis(trifluoromethyl)phenyl]ethyl azide (crude product: 111.5 mg).
$^1$H-NMR ($CDCl_3$) δ: 1.61 (3H, d, J = 6.8 Hz), 4.79 (1H, q, J = 6.8 Hz), 7.78 (2H, s), 7.84 (1H, s)

The obtained 1-[3,5-bis(trifluoromethyl)phenyl]ethyl azide (crude product: 111.5 mg) was dissolved in methanol (6 mL), and palladium fibroin (18 mg) was then added to the obtained solution. Hydrogen substitution was carried out and the mixture was stirred at a room temperature for 1 hour. The reaction solution was filtrated with Celite, and the filtrate was then concentrated under a reduced pressure. The obtained residue was purified by silica gel column chromatography (chloroform : methanol = 50 : 1 to 5 : 1), to obtain 77.6 mg of 1-[3,5-bis(trifluoromethyl)phenyl]ethylamine in the form of a colorless oily product (yield: 91%, 2 steps).
$^1$H-NMR ($CDCl_3$) δ: 1.42 (3H, d, J = 6.8 Hz), 1.58 (2H, br-s), 4.30 (1H, q, J = 6.8 Hz), 7.75 (1H, s), 7.85 (2H, s)

The specific optical rotation of the obtained 1-[3,5-bis(trifluoromethyl)phenyl]ethylamine is the following.
$[\alpha]_D^{25}$-15.9 (c = 1.31, $CHCl_3$)

On the other hand, the specific rotation of a commercially available standard product of (S)-1-[3,5-bis(trifluoromethyl)phenyl]ethylamine (manufactured by Central Glass Co., Ltd.; Lot. 0102000; optical purity: 99% ee) is the following.
$[\alpha]_D^25$ -15.9 (c = 1.15, $CHCl_3$)

The sign in the measured value of the specific optical rotation was identical with that of the commercially available standard product. Thus, the obtained 1-[3,5-bis(trifluoromethyl)phenyl]ethylamine was confirmed to be an (S)-form. Since this amine was obtained from 1-bromo-1-[3,5-bis(trifluoromethyl)phenyl]ethane via the nucleophilic substitution reaction of azide ions, the 1-bromo-1-[3,5-bis(trifluoromethyl)phenyl]ethane obtained in 1-(a) above was confirmed to be an (R)-form.

1-(b): Production 2 of (R)-1-bromo-1-[3,5-bis(trifluoromethyl)phenyl]ethane

Under an argon atmosphere, phosphorus tribromide (157.3 g, 0.58 mol) was added dropwise to (S)-1-[3,5-bis(trifluoromethyl)phenyl]ethanol (1) (300g, 1.16 mol, 96% ee) at 20°C or lower in a water bath, and the obtained mixture was then stirred at 19°C to 22°C for 30 minutes. Thereafter, the reaction solution was cooled, and hydrogen bromide (30% acetic acid solution) (228 mL, 1.16 mol) was added dropwise thereto at 0°C or lower. The obtained mixture was stirred at 13°C to 15°C for 16 hours. Thereafter, the reaction solution was added to ice water, and was then extracted with n-hexane (3 L x 2). The organic layers were gathered. The gathered organic layer was successively washed with saturated sodium bicarbonate water (3 L) and saturated saline solution (3 L), and was then dried over anhydrous magnesium sulfate. The resultant was concentrated under a reduced pressure (90 to 100 mmHg) to obtain 389.2 g of a crude product. The obtained crude product was purified by column chromatography (900 g of silica gel, developing

solvent: n-hexane), to obtain 349.8 g of (R)-1-bromo-1-[3,5-bis(trifluoromethyl)phenyl]ethane (2) in the form of a colorless oily product (yield: 93.8%).

Since the first peak appeared as a main peak in the chiral HPLC analysis, as shown below, 1-bromo-1-[3,5-bis(trifluoromethyl)phenyl]ethane produced in 1-(b) above was also confirmed to be an (R)-form, as with the compound obtained in 1-(a) above.

Chiral HPLC analysis: optical purity > 93.9% ee (main peak: first peak); invert ratio: 97.8%
$^1$H-NMR ($CDCl_3$) $\delta$: 2.08 (3H, d, J = 7.1 Hz), 5.21 (1H, q, J = 7.1 Hz), 7.81 (1H, s), 7.87 (2H, s)

Step 2: Production of (S)-form-dominated semichiral form of trans-{4-[({2-[({1-[3,5-bis(trifluoromethyl)phenyl]ethyl}{5-[2-(methylthio)ethoxy]pyrimidin-2-yl}amino)methyl]-4-(trifluoromethyl)phenyl}(ethyl)amino)methyl]cyclohexyl}ac etic acid

Under an argon atmosphere, NaH (60% in oil, 119 g, 2.98 mol) was added to an anhydrous tetrahydrofuran (THF, 2.26 L) solution containing trans-[4-([(ethyl){2-[({5-[2-(methylthio)ethoxy]pyrimidin-2-yl}amino)methyl]-4-(trifluoromethyl)phenyl}amino]methyl)cyclohexyl]ethyl acetate (3) (565.4g, 0.99 mol) synthesized by the method described in Patent Literature 2 (International Publication No. WO2008/129951) under cooling on ice, and the obtained mixture was then stirred at a room temperature for 1 hour. Thereafter, the reaction solution was cooled to -30°C, and an anhydrous N,N-dimethylformamide (4.53 L) solution of (R)-1-bromo-1-[3,5-bis(trifluoromethyl)phenyl]ethane (2) (682 g, 1.99 mol, 93.9% ee) obtained in Step 1 was then added dropwise to the reaction solution, so that the temperature in the reaction system became -15°C or lower. The obtained mixture was stirred at -15°C to -1°C for 5 hours. Thereafter, the reaction solution was added to a mixed solution of ice water (35 L) and toluene (30 L). After that, citric acid was added to the mixed solution until the pH was adjusted to 6.9, and an organic layer was separated.

A water layer was extracted with toluene (20 L) twice, and the organic layers were then gathered. The gathered organic layer was dried over anhydrous magnesium sulfate, and was then concentrated under a reduced pressure to obtain a crude product. The crude product was dissolved in ethanol (8 L), and a 2 M NaOH aqueous solution (1.24 L, 2.48 mol) was added to the obtained solution under cooling on ice. The obtained mixture was stirred at 50°C for 3.5 hours. Under cooling on ice, a 1 M HCl aqueous solution was added to the reaction solution until the pH was adjusted to 5.4. The thus mixed solution was poured into water (25 L), and extraction was then carried out with ethyl acetate (22 L) twice. The organic layer was washed with saturated saline solution (12 L), and was then dried over anhydrous magnesium sulfate, followed by concentration under a reduced pressure. The obtained residue was purified by column chromatography (21 g of silica gel, developing solvent: heptane/acetone = 7/1 $\rightarrow$ 3/1), to obtain a semichiral form (4) of trans-{4-[({2-[({1-[3,5-bis(trifluoromethyl)phenyl]ethyl}{5-[2-(methylthio)ethoxy]pyrimidin-2-yl}amino)methyl]-4-(trifluoromethyl)phenyl}(ethyl)amino)methyl]cyclohexyl}ac etic acid (yellow oily product, 744.1 g, yield: 96%).

As described in Step 1 above, (R)-1-bromo-1-[3,5-bis(trifluoromethyl)phenyl]ethane (2), the absolute configuration of which had been confirmed, was used as a raw material, and a nucleophilic substitution reaction with amine (3) progressed. Thus, the (S)-form is dominated in the obtained semichiral form (4).
$^1$H-NMR ($CDCl_3$) $\delta$: 0.85-0.96 (7H, m), 1.35-1.45 (4H, m), 1.60-1.78 (5H, m), 2.18-2.21 (5H, m), 2.69 (1H, m), 2.81-2.91 (5H, m), 4.16 (2H, q, J = 6.8 Hz), 4.61 (1H, d, J = 17.1 Hz), 4.85 (1H, d, J = 17.1 Hz), 6.22 (1H, q, J = 6.8 Hz), 7.11 (1H, d, J = 8.6 Hz), 7.23 (1H, s), 7.37 (1H, d, J = 8.3 Hz), 7.70 (1H, s), 7.73 (2H, s), 8.14 (2H, s)

Step 3: Production of (S)-form-dominated semichiral form of trans-{4-[({2-[({1-[3,5-bis(trifluoromethyl)phenyl]ethyl}{5-[2-(methylthio)ethoxy]pyrimidin-2-yl}amino)methyl]-4-(trifluoromethyl)phenyl}(ethyl)amino)methyl]cyclohexyl}ac etic acid benzyl ester

Under an argon atmosphere, benzyl alcohol (113.1 g, 1.05 mol), WSC·HCl (200.5 g, 1.05 mol) and DMAP (11.9 g, 98 mmol) were added to an anhydrous dichloroethane (11.6 L) solution containing the (S)-form-dominated semichiral form (4) (744.1g, 0.95 mol) of trans-{4-[({2-[({1-[3,5-bis(trifluoromethyl)phenyl]ethyl}{5-[2-(methylthio)ethoxy]pyrimidin-2-yl}amino)methyl]-4-(trifluoromethyl)phenyl}(ethyl)amino)methyl]cyclohexyl}ac etic acid obtained in Step 2 under cooling on ice. The thus obtained mixture was stirred at a room temperature overnight. Thereafter, water (10 L) was added to the reaction solution, and extraction was then carried out with chloroform (19 L, 14 L). The organic layer was washed with saturated saline solution (12 L), and was then dried over anhydrous magnesium sulfate, followed by concentration under a reduced pressure. The obtained residue was purified by column chromatography (28 g of silica gel, developing solvent: heptane/ethyl acetate = 6/1), to obtain a semichiral form (5) of trans-{4-[({2-[({1-[3,5-bis(trifluoromethyl)phenyl]ethyl}{5-[2-(methylthio)ethoxy]pyrimidin-2-yl}amino)methyl]-4-(trifluoromethyl)phenyl}(ethyl)amino)methyl]cyclohexyl}ac etic acid benzyl ester (yellow oily product, 745.8 g, yield: 90%).

It is to be noted that (S)-form is dominated in the obtained semichiral form (5), as with the semichiral form (4).

$^1$H-NMR ($CDCl_3$) δ: 0.87-0.95 (7H, m), 1.37 (1H, m), 1.43 (3H, d, J = 7.1 Hz), 1.65-1.77 (5H, m), 2.20 (2H, d, J = 6.8 Hz), 2.22 (3H, s), 2.66-2.71 (2H, m), 2.82-2.91 (4H, m), 4.15 (2H, t, J = 6.6 Hz), 4.62 (1H, d, J = 17.1 Hz), 4.85 (1H, d, J = 17.1 Hz), 5.10 (2H, s), 6.21 (1H, q, J = 7.1 Hz), 7.10 (1H, d, J = 8.3 Hz), 7.22 (1H, s), 7.28-7.38 (6H, m), 7.70 (1H, s), 7.73 (2H, s), 8.14 (2H, s)

Step 4: Production of (S)-form-dominated semichiral form of trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}acetic acid benzyl ester

Under an argon atmosphere, tantalum pentachloride (31.3 g, 87.3 mmol) and 30% hydrogen peroxide water (496 mL, 4.38 mol) were added to a 2-propanol (15.2 L) solution containing (S)-form-dominated semichiral form (5) (745.8 g, 0.87 mol) of the trans-{4-[({2-[({1-[3,5-bis(trifluoromethyl)phenyl]ethyl}{5-[2-(methylthio)ethoxy]pyrimidin-2-yl}amino)methyl]-4-(trifluoromethyl)phenyl}(ethyl)amino)methyl]cyclohexyl}ac etic acid benzyl ester obtained in Step 3. The thus obtained mixture was stirred at a room temperature for 5 hours. Thereafter, the reaction solution was quenched with a saturated sodium hydrogen sulfite aqueous solution (3.1 L), and water (15 L) was then added to the reaction solution. The mixed solution was extracted with chloroform (14 L, 12 L). The organic layer was washed with saturated saline solution (20 L), and was then dried over anhydrous magnesium sulfate, followed by concentration under a reduced pressure. The obtained residue was purified by column chromatography (26 kg of silica gel, developing solvent: heptane/ethyl acetate = 3/1 → 2/1), so as to obtain a semichiral form (6) of the trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid benzyl ester (yellow amorphous product, 619.5 g, yield: 79%).

It is to be noted that (S)-form is dominated in the obtained semichiral form (6), as with the semichiral form (4) and the semichiral form (5).

Chiral HPLC analysis: optical purity 67.7% ee (main peak: second peak)
$^1$H-NMR ($CDCl_3$) δ: 0.87-0.96 (7H, m), 1.38 (1H, m), 1.45 (3H, d, J = 7.1 Hz), 1.65-1.80 (5H, m), 2.21 (2H, d, J = 6.6 Hz), 2.69 (1H, m), 2.81-2.91 (3H, m), 3.08 (3H, s), 3.44 (2H, t, J = 5.4 Hz), 4.44 (2H, t, J = 5.4 Hz), 4.64 (1H, d, J = 17.1 Hz), 4.86 (1H, d, J = 17.3 Hz), 5.10 (2H, s), 6.19 (1H, q, J = 6.9 Hz), 7.12 (1H, d, J = 8.3 Hz), 7.19 (1H, s), 7.30-7.39 (6H, m), 7.71 (1H, s), 7.72 (2H, s), 8.16 (2H, s)

Step 5: Production of substantially optically pure (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid benzyl ester

The (S)-form-dominated semichiral form (6) (111.7 g, 123.7 mmol, 67.7% ee) of trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid benzyl ester obtained in Step 4 was dissolved in ethanol (825 mL). Then, 2.0 mg of a seed crystal (a racemic crystal produced in Step 7 below), which had been prepared separately, was added to the above obtained solution at 15°C to 20°C. The obtained mixture was stirred at the same temperature as described above for 21 hours, and then at 0°C for 3 hours. Thereafter, the precipitate was separated by filtration, and was then washed with cold ethanol (165 mL), followed by concentration of mother liquor under a reduced pressure, to obtain substantially optically pure trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid benzyl ester (7) (yellow amorphous product, 66.38 g, yield: 59%).

It is to be noted that the obtained trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid benzyl ester (7) is an (S)-form because it was obtained by separating a racemic body-dominated crystal from the (S)-form-dominated semichiral form (6) by filtration.

Chiral HPLC analysis: optical purity > 99% ee (main peak: second peak)
$[\alpha]_D^{20}$-42.36 (c = 1.0 w/v%, $CHCl_3$)

As a result of the chiral HPLC analysis, the optical purity of the filtrated racemic body-dominated crystal was found to be 22% ee (43.39 g, yield: 39%).

Step 6: Production of substantially optically pure (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid

Under a nitrogen atmosphere, 10% Pd-C (wet) (3.4g) was added to an ethanol (340 mL) solution containing (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid benzyl ester (7) (34.2 g, 37.88 mmol, > 99% ee) obtained in Step 5. After hydrogen substitution was carried out, the reaction mixture was stirred at a room temperature for 2 hours. Thereafter, the reaction suspension was filtrated with Celite, and was then washed with ethanol (50 mL). After that, the wash liquid was concentrated under a reduced pressure to obtain substantially optically pure trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid (Compound 2) (white amorphous product, 31.78 g, yield: 100%).

As shown in the following specific optical rotation, the obtained compound was a levorotatory compound. In addition, since the compound was obtained by deprotecting ester from the benzyl ester (7) of an (S)-form, it is also an (S)-form.

Chiral HPLC analysis: optical purity > 99% ee (main peak: second peak)
$[\alpha]_D^{20}$-46.68 (c = 1.0, $CHCl_3$)
IR (ATR) $cm^{-1}$: 2921, 1706, 1479, 1279, 1134
$^1$H-NMR ($CDCl_3$) $\delta$: 0.80-0.96 (7H, m), 1.38 (1H, m), 1.47 (3H, d, J = 7.1 Hz), 1.65-1.77 (5H, m), 2.19 (2H, d, J = 6.8 Hz), 2.72 (1H, m), 2.81-2.91 (3H, m), 3.08 (3H, s), 3.45 (2H, t, J = 5.2 Hz), 4.44 (2H, q, J = 5.4 Hz), 4.62 (1H, d, J = 17.1 Hz), 4.86 (1H, d, J = 17.4 Hz), 6.21 (1H, q, J = 7.1 Hz), 7.13 (1H, d, J = 8.3 Hz), 7.19 (1H, s), 7.38 (1H, d, J = 6.6 Hz), 7.71 (1H, s), 7.73 (2H, s), 8.15 (2H, s)

Step 7: Production of racemic seed crystal of trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid benzyl ester

Under cooling on ice, benzyl alcohol (2.93 g, 27.07 mmol), DMAP (300 mg, 2.46 mmol) and WSC·HCl (5.19 g, 27.07 mmol) were added to an anhydrous dichloromethane (200 mL) solution containing trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid (racemic compound (I)) (20 g, 24.61 mmol) synthesized by the method described in Example 45 of Patent Literature 2 (International Publication No. WO2008/129951). The temperature of the thus obtained mixture was increased to a room temperature, and the mixture was then stirred for 16 hours. Thereafter, water (100 mL) was added to the reaction solution, and extraction was then carried out with chloroform (500 mL). The organic layer was washed with a 2 M hydrochloric acid aqueous solution (100 mL) and saturated saline solution (100 mL), and was then dried over anhydrous magnesium sulfate, followed by concentration under a reduced pressure. The obtained residue was purified by column chromatography (350 g of silica gel, developing solvent: N-hexane/ethyl acetate = 3/1 $\rightarrow$ 1/1), to obtain trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid benzyl ester (21.15 g, yield: 95.2%) in the form of a white amorphous product.

The obtained white amorphous trans-{4-[({2-[({1-[3,5-bis(trifluoromethyl)phenyl]ethyl}{5-[2-(methylthio)ethoxy]pyrimidin-2-yl}amino)methyl]-4-(trifluoromethyl)phenyl}(ethyl)amino)methyl]cyclohexyl}ac etic acid benzyl ester (7.9 g) was dissolved in ethanol (40 mL), and the obtained solution was then stirred at a room temperature for 15 hours. Thereafter, the obtained precipitate was collected by filtration, and it was washed with cold ethanol (20 mL) and was then dried at 60°C for 4 hours under a reduced pressure, so as to obtain a racemic crystal of trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid benzyl ester (white crystalline powder, 6.98g, recovery rate: 88.4%).

In the present Production Example 2, a compound having a middle level of optical purity obtained as a result of a reduction in the optical purity caused by partial racemization (a compound having an optical purity of approximately 50 to 90% ee, and preferably approximately 70 to 90% ee) is referred to as a "semichiral form." Furthermore, the semichiral form, in which a compound with S-configured asymmetric carbon atom(s) is present in an amount excessively larger than a compound with R-configured asymmetric carbon atom(s), is referred to as an "(S)-form-dominated semichiral form."

Test Example 1

A compound in a test compound group was added to cells of a human hepatocellular carcinoma cell line HepG2, and the cells were then cultured for 24 hours. Thereafter, the expression levels of NPC1L1 and LIPG mRNAs were measured by real-time RT-PCR. Specifically, the HepG2 cells were inoculated at $2 \times 10^5$ cells/well into a 24-well plate, and were then cultured overnight. Thereafter, Compound 2 was dissolved in dimethyl sulfoxide (DMSO) to give concentrations of 0.1 μM, 1 μM and 10 μM, and Anacetrapib was dissolved in DMSO to give concentrations of 1 μM and 10 μM. The thus prepared solution was added to the culture in an amount of 1/1000-fold of the culture fluid. The cells were cultured at 37°C for 24 hours in a $CO_2$ incubator. Thereafter, 500 μL of ISOGEN (Nippon Gene Co., Ltd., Catalog No. 31-02501) was added to the culture, and total RNA was then extracted. From the extracted total RNA, cDNA was synthesized using High Capacity cDNA Reverse Transcription kit (Applied Biosystems, Catalog No. 4368813). The expression levels of human NPC1L1 and LIPG mRNAs were measured, using human NPC1L1-specific primers (sense: 5'-CAGGTATGGTCGCCCGAAGCAC-3' (SEQ ID NO: 1); and antisense: 5'-TGCGGTTGTTCTGGAAATACTG-3' (SEQ ID NO: 2)), human LIPG-specific primers (sense: 5'-TCAACGATGTCTTGGGATCA-3' (SEQ ID NO: 3); and antisense: 5'-TGAAGCGATTGGAGTCAGTG-3' (SEQ ID NO: 4)), and Fast SYBR Green master mix (Applied Biosystems, Catalog No. 4385614). 7900HT Fast Realtime PCR system was used as a measurement device.

The measurement value was corrected with the expression level of β-Actin mRNA. The expression levels of LIPG and NPC1L1 mRNAs in cells, to which only DMSO had been added, were defined as 1, and the expression levels of LIPG and NPC1L1 mRNAs in cells, to which the test compound group (Compound 2 or Anacetrapib) had been added, were calculated as a relative value thereof. The results are shown in Figures 1 and 2.

Test Example 2

A compound of a test compound group was added to cells of a human hepatocellular carcinoma cell line HepG2, and the cells were then cultured for 24 hours. Thereafter, the gene expression profile was measured using the DNA microarray of Agilent. Specifically, the HepG2 cells were inoculated at a concentration of $2 \times 10^5$ cells/well into a 24-well plate, and were then cultured overnight. Thereafter, Compound 2 was dissolved in dimethyl sulfoxide (DMSO) to give concentrations of 1 μM and 10 μM, whereas Anacetrapib was dissolved in DMSO to give a concentration of 10 μM. The thus prepared solution was added to the culture fluid in an amount of 1/1000-fold of the culture fluid. The cells were cultured at 37°C for 24 hours in a $CO_2$ incubator. Thereafter, 500 μL of ISOGEN (Nippon Gene Co., Ltd., Catalog No. 31-02501) was added to the culture, and total RNA was then extracted. Cy3-labeled cRNA was synthesized from 200 ng of the RNA using Low Input Quick Amp Labeling Kit (Agilent, Catalog No. 5190-2308). The synthesized Cy3-labeled cRNA was hybridized to Whole Human Genome (4 x 44K) (Agilent, Catalog No. G4110F), using GE Hybridization Buffer HI-RPM (Agilent, Catalog No. 5190-0403). After completion of the hybridization, the DNA microarray was washed with Gene Expression Wash Buffer (Agilent, Catalog No. 5188-5327), and data were then scanned with a scanner (Agilent).

The analysis was carried out using GeneSpring (Agilent), and the change rate of the expression level of each gene was calculated.

(Change rate) = (Expression level of each gene in cells treated with compound) ÷ (Expression level of each gene in cells untreated with compound)

The results are shown in Table 1.

[Table 1]

| | Change rate (fold) | | |
|---|---|---|---|
| | Compound 2 (1 μM) | Compound 2 (10 μM) | Anacetrapib (10 μM) |
| NPC1L1 | -2.3 | -3.1 | -1.5 |
| LIPG | -2.5 | -6.2 | -1.9 |

From the above described pharmacological test results, it became clear that the compound represented by the

general formula (I) or (II) has a strong and sustained action to suppress the expression of NPC1L1 and LIPG mRNAs.

[Industrial Applicability]

A pharmaceutical comprising a compound having CETP inhibitory activity has a strong action to suppress the expression of NPC1L1 and LIPG mRNAs and is useful for preventing and/or treating obesity. Therefore such a pharmaceutical is industrially applicable.

SEQUENCE LISTING

<110> Genmab A/S

<120> Bispecific antibodies against HER2xCD3

<130> P/73.WO

<160> 255

<170> PatentIn version 3.5

<210> 1
<211> 121
<212> PRT
<213> homo sapiens

<400> 1

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Gly Ile Ser Trp Val Arg Gln Ala Pro Gly Gln Gly Leu Glu Trp Met
        35                  40                  45

Gly Trp Leu Ser Ala Tyr Ser Gly Asn Thr Ile Tyr Ala Gln Lys Leu
    50                  55                  60

Gln Gly Arg Val Thr Met Thr Thr Asp Thr Ser Thr Thr Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Arg Ile Val Val Arg Pro Asp Tyr Phe Asp Tyr Trp Gly
            100                 105                 110

Gln Gly Thr Leu Val Thr Val Ser Ser
        115                 120
```

<210> 2
<211> 8
<212> PRT
<213> homo sapiens

<400> 2

```
Gly Tyr Thr Phe Thr Asn Tyr Gly
1               5
```

<210> 3

<211> 8
<212> PRT
<213> homo sapiens

<400> 3

Leu Ser Ala Tyr Ser Gly Asn Thr
1 5

<210> 4
<211> 14
<212> PRT
<213> homo sapiens

<400> 4

Ala Arg Asp Arg Ile Val Val Arg Pro Asp Tyr Phe Asp Tyr
1 5 10

<210> 5
<211> 107
<212> PRT
<213> homo sapiens

<400> 5

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Tyr Asp Ala Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Arg Ser Asn Trp Pro Arg
85 90 95

Thr Phe Gly Gln Gly Thr Lys Val Glu Ile Lys
100 105

<210> 6
<211> 6
<212> PRT
<213> homo sapiens

<400> 6

Gln Ser Val Ser Ser Tyr
1               5

<210>  7
<211>  9
<212>  PRT
<213>  homo sapiens

<400>  7

Gln Gln Arg Ser Asn Trp Pro Arg Thr
1               5

<210>  8
<211>  119
<212>  PRT
<213>  homo sapiens

<400>  8

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Asn Trp Val Arg Gln Ala Pro Gly Lys Gly Leu Glu Trp Val
        35                  40                  45

Ser Ala Ile Ser Gly Arg Gly Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Ser Ser Leu Arg Ala Glu Asp Thr Ala Val Tyr Tyr Cys
                85                  90                  95

Ala Lys Ala Arg Ala Asn Trp Asp Tyr Phe Asp Tyr Trp Gly Gln Gly
            100                 105                 110

Thr Leu Val Thr Val Ser Ser
        115

<210>  9
<211>  8
<212>  PRT
<213>  homo sapiens

<400>  9

Gly Phe Thr Phe Ser Ser Tyr Ala
1               5

<210> 10
<211> 8
<212> PRT
<213> homo sapiens

<400> 10

Ile Ser Gly Arg Gly Gly Thr Thr
1 5

<210> 11
<211> 12
<212> PRT
<213> homo sapiens

<400> 11

Ala Lys Ala Arg Ala Asn Trp Asp Tyr Phe Asp Tyr
1 5 10

<210> 12
<211> 107
<212> PRT
<213> homo sapiens

<400> 12

Asp Ile Gln Met Thr Gln Ser Pro Ser Ser Val Ser Ala Ser Val Gly
1 5 10 15

Asp Arg Val Thr Ile Thr Cys Arg Ala Ser Gln Gly Ile Ser Ser Trp
20 25 30

Leu Ala Trp Tyr Gln His Lys Pro Gly Lys Ala Pro Lys Leu Leu Ile
35 40 45

Tyr Ala Ala Ser Ile Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Ala Asn Ser Phe Pro Ile
85 90 95

Thr Phe Gly Gln Gly Thr Arg Leu Glu Ile Lys
100 105

<210> 13
<211> 6
<212> PRT
<213> homo sapiens

<400> 13

Gln Gly Ile Ser Ser Trp
1 5

<210> 14
<211> 9
<212> PRT
<213> homo sapiens

<400> 14

Gln Gln Ala Asn Ser Phe Pro Ile Thr
1 5

<210> 15
<211> 121
<212> PRT
<213> homo sapiens

<400> 15

Gln Val Gln Leu Val Gln Ser Gly Ala Glu Val Lys Lys Pro Gly Ser
1 5 10 15

Ser Val Lys Val Ser Cys Lys Ala Ser Gly Gly Thr Phe Arg Thr Tyr
20 25 30

Ala Ile Asn Trp Val Arg Gln Ala Pro Gly Gln Gly Leu Glu Trp Met
35 40 45

Gly Arg Ile Asn Thr Val Leu Gly Ile Val Asn His Ala Gln Lys Phe
50 55 60

Gln Gly Arg Val Thr Ile Thr Ala Asp Lys Ser Thr Asn Thr Ala Tyr
65 70 75 80

Met Glu Leu Asn Ser Leu Arg Ser Glu Asp Thr Ala Val Tyr Tyr Cys
85 90 95

Ala Arg Glu Lys Gly Val Asp Tyr Tyr Tyr Gly Ile Glu Val Trp Gly
100 105 110

Gln Gly Thr Thr Val Thr Val Ser Ser
115 120

<210> 16
<211> 8
<212> PRT
<213> homo sapiens

<400> 16

```
Gly Gly Thr Phe Arg Thr Tyr Ala
1               5


<210>  17
<211>  8
<212>  PRT
<213>  homo sapiens

<400>  17

Ile Asn Thr Val Leu Gly Ile Val
1               5


<210>  18
<211>  14
<212>  PRT
<213>  homo sapiens

<400>  18

Ala Arg Glu Lys Gly Val Asp Tyr Tyr Tyr Gly Ile Glu Val
1               5                   10


<210>  19
<211>  107
<212>  PRT
<213>  homo sapiens

<400>  19

Asp Ile Gln Met Thr Gln Ser Pro Ser Ser Val Ser Ala Ser Val Gly
1               5                   10                  15


Asp Arg Val Thr Ile Thr Cys Arg Ala Ser Gln Gly Ile Ser Ser Trp
            20                  25                  30


Leu Ala Trp Tyr Gln His Lys Pro Gly Lys Ala Pro Lys Leu Leu Ile
        35                  40                  45


Tyr Val Ala Ser Thr Leu Gln Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Ala Asn Ser Phe Pro Leu
                85                  90                  95


Thr Phe Gly Gly Gly Thr Lys Val Glu Ile Lys
            100                 105


<210>  20
```

<211> 6
<212> PRT
<213> homo sapiens

<400> 20

Gln Gly Ile Ser Ser Trp
1 5

<210> 21
<211> 9
<212> PRT
<213> homo sapiens

<400> 21

Gln Gln Ala Asn Ser Phe Pro Leu Thr
1 5

<210> 22
<211> 120
<212> PRT
<213> homo sapiens

<400> 22

Gln Val Gln Leu Gln Gln Trp Gly Ala Gly Leu Leu Lys Pro Ser Glu
1 5 10 15

Thr Leu Ser Leu Thr Cys Ala Val Tyr Gly Gly Ser Phe Ser Asp Tyr
20 25 30

Tyr Trp Asn Trp Ile Arg Gln Pro Pro Gly Lys Gly Leu Glu Trp Ile
35 40 45

Gly Glu Ile His His Ser Gly Ser Thr Asn Tyr Asn Pro Ser Leu Lys
50 55 60

Ser Arg Val Thr Ile Ser Val Asp Thr Ser Lys Asn Gln Phe Ser Leu
65 70 75 80

Lys Leu Ser Ser Val Thr Ala Ala Asp Thr Ala Val Tyr Tyr Cys Ala
85 90 95

Arg Gly Tyr Tyr Asp Ser Gly Val Tyr Tyr Phe Asp Tyr Trp Ala Gln
100 105 110

Gly Thr Leu Val Thr Val Ser Ser
115 120

<210> 23
<211> 8
<212> PRT

<213> homo sapiens

<400> 23

Gly Gly Ser Phe Ser Asp Tyr Tyr
1 5

<210> 24
<211> 7
<212> PRT
<213> homo sapiens

<400> 24

Ile His His Ser Gly Ser Thr
1 5

<210> 25
<211> 14
<212> PRT
<213> homo sapiens

<400> 25

Ala Arg Gly Tyr Tyr Asp Ser Gly Val Tyr Tyr Phe Asp Tyr
1 5 10

<210> 26
<211> 107
<212> PRT
<213> homo sapiens

<400> 26

Asp Ile Gln Met Thr Gln Ser Pro Ser Ser Leu Ser Ala Ser Val Gly
1 5 10 15

Asp Arg Val Thr Ile Thr Cys Arg Ala Ser Gln Gly Ile Ser Arg Trp
20 25 30

Leu Ala Trp Tyr Gln Gln Lys Pro Glu Lys Ala Pro Lys Ser Leu Ile
35 40 45

Tyr Ala Ala Ser Ser Leu Arg Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Asn Ser Tyr Pro Ile
85 90 95

Thr Phe Gly Gln Gly Thr Arg Leu Glu Ile Lys
100 105

<210> 27
<211> 6
<212> PRT
<213> homo sapiens

<400> 27

Gln Gly Ile Ser Arg Trp
1 5

<210> 28
<211> 9
<212> PRT
<213> homo sapiens

<400> 28

Gln Gln Tyr Asn Ser Tyr Pro Ile Thr
1 5

<210> 29
<211> 120
<212> PRT
<213> homo sapiens

<400> 29

Gln Val Gln Leu Gln Gln Trp Gly Ala Gly Leu Leu Lys Pro Ser Glu
1 5 10 15

Thr Leu Ser Leu Thr Cys Ala Val Ser Gly Gly Ser Phe Ser Gly Tyr
20 25 30

Tyr Trp Thr Trp Ile Arg Gln Pro Pro Gly Lys Gly Leu Glu Trp Ile
35 40 45

Gly Glu Ile Tyr His Ser Gly Asp Thr Asn Tyr Asn Pro Ser Leu Lys
50 55 60

Ser Arg Val Thr Ile Ser Val Asp Thr Ser Lys Asn Gln Phe Ser Leu
65 70 75 80

Lys Leu Tyr Ser Val Thr Ala Ala Asp Thr Ala Val Tyr Tyr Cys Ala
85 90 95

Arg Leu Tyr Phe Gly Ser Gly Ile Tyr Tyr Leu Asp Tyr Trp Gly Gln
100 105 110

Gly Thr Leu Val Thr Val Ser Ser
115 120

<210> 30
<211> 8
<212> PRT
<213> homo sapiens

<400> 30

Gly Gly Ser Phe Ser Gly Tyr Tyr
1 5

<210> 31
<211> 14
<212> PRT
<213> homo sapiens

<400> 31

Ala Arg Leu Tyr Phe Gly Ser Gly Ile Tyr Tyr Leu Asp Tyr
1 5 10

<210> 32
<211> 107
<212> PRT
<213> homo sapiens

<400> 32

Asp Ile Gln Met Thr Gln Ser Pro Ser Ser Leu Ser Ala Ser Val Gly
1 5 10 15

Asp Arg Val Thr Ile Thr Cys Arg Ala Ser Gln Gly Ile Ser Ser Trp
20 25 30

Leu Val Trp Tyr Gln Gln Lys Pro Glu Lys Ala Pro Lys Ser Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Asn Ser Phe Pro Pro
85 90 95

Thr Phe Gly Gln Gly Thr Lys Val Glu Ile Lys
100 105

<210> 33
<211> 6
<212> PRT
<213> homo sapiens

<400> 33

Gln Gly Ile Ser Ser Trp
1               5

<210> 34
<211> 9
<212> PRT
<213> homo sapiens

<400> 34

Gln Gln Tyr Asn Ser Phe Pro Pro Thr
1               5

<210> 35
<211> 119
<212> PRT
<213> homo sapiens

<400> 35

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Thr Phe
            20                  25                  30

Ala Ile His Trp Val Arg Gln Ala Pro Gly Lys Gly Leu Glu Trp Val
        35                  40                  45

Ala Val Ile Ser Tyr Asp Gly Gly His Lys Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Met Tyr Tyr Cys
                85                  90                  95

Ala Arg Gly Leu Gly Val Trp Gly Ala Phe Asp Tyr Trp Gly Gln Gly
            100                 105                 110

Thr Leu Val Thr Val Ser Ser
        115

<210> 36
<211> 8
<212> PRT
<213> homo sapiens

<400> 36

Gly Phe Thr Phe Ser Thr Phe Ala

1 5

<210> 37
<211> 8
<212> PRT
<213> homo sapiens

<400> 37

Ile Ser Tyr Asp Gly Gly His Lys
1 5

<210> 38
<211> 12
<212> PRT
<213> homo sapiens

<400> 38

Ala Arg Gly Leu Gly Val Trp Gly Ala Phe Asp Tyr
1 5 10

<210> 39
<211> 106
<212> PRT
<213> homo sapiens

<400> 39

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Tyr Asp Ala Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Arg Ser Asn Trp Trp Thr
85 90 95

Phe Gly Gln Gly Thr Lys Val Glu Ile Lys
100 105

<210> 40
<211> 6
<212> PRT

<213> homo sapiens

<400> 40

Gln Ser Val Ser Ser Tyr
1 5

<210> 41
<211> 8
<212> PRT
<213> homo sapiens

<400> 41

Gln Gln Arg Ser Asn Trp Trp Thr
1 5

<210> 42
<211> 123
<212> PRT
<213> homo sapiens

<400> 42

Glu Val Gln Leu Val Gln Ser Gly Ala Glu Val Lys Lys Pro Gly Glu
1 5 10 15

Ser Leu Thr Ile Ser Cys Lys Gly Ser Gly Tyr Ser Phe Ser Ile Tyr
20 25 30

Trp Ile Gly Trp Val Arg Gln Met Pro Gly Lys Gly Leu Glu Trp Met
35 40 45

Gly Ile Ile Phe Pro Gly Asp Ser Asp Ile Arg Tyr Ser Pro Ser Phe
50 55 60

Gln Gly Gln Val Thr Ile Ser Ala Asp Lys Ser Ile Ser Thr Ala Tyr
65 70 75 80

Leu Gln Trp Ser Ser Leu Lys Ala Ser Asp Thr Ala Met Tyr Tyr Cys
85 90 95

Ala Arg Gln Pro Gly Asp Trp Ser Pro Arg His Trp Tyr Phe Asp Leu
100 105 110

Trp Gly Arg Gly Thr Leu Val Thr Val Ser Ser
115 120

<210> 43
<211> 8
<212> PRT
<213> homo sapiens

<400> 43

```
Gly Tyr Ser Phe Ser Ile Tyr Trp
1               5
```

<210> 44
<211> 8
<212> PRT
<213> homo sapiens

<400> 44

```
Ile Phe Pro Gly Asp Ser Asp Ile
1               5
```

<210> 45
<211> 16
<212> PRT
<213> homo sapiens

<400> 45

```
Ala Arg Gln Pro Gly Asp Trp Ser Pro Arg His Trp Tyr Phe Asp Leu
1               5                   10                  15
```

<210> 46
<211> 107
<212> PRT
<213> homo sapiens

<400> 46

```
Val Ile Trp Met Thr Gln Ser Pro Ser Leu Leu Ser Ala Ser Thr Gly
1               5                   10                  15

Asp Arg Val Thr Ile Ser Cys Arg Met Ser Gln Gly Ile Ser Ser Tyr
            20                  25                  30

Leu Ala Trp Tyr Gln Gln Lys Pro Gly Lys Ala Pro Glu Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Thr Ile Ser Tyr Leu Gln Ser
65                  70                  75                  80

Glu Asp Phe Ala Thr Tyr Tyr Cys Gln Gln Tyr Tyr Ser Phe Pro Leu
                85                  90                  95

Thr Phe Gly Gly Gly Thr Lys Val Glu Ile Lys
            100                 105
```

<210> 47
<211> 6
<212> PRT
<213> homo sapiens

<400> 47

Gln Gly Ile Ser Ser Tyr
1               5

<210> 48
<211> 9
<212> PRT
<213> homo sapiens

<400> 48

Gln Gln Tyr Tyr Ser Phe Pro Leu Thr
1               5

<210> 49
<211> 126
<212> PRT
<213> homo sapiens

<400> 49

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Asn Phe Thr Ser Tyr
            20                  25                  30

Trp Ile Gly Trp Val Arg Gln Met Pro Gly Lys Gly Leu Glu Trp Met
        35                  40                  45

Gly Ile Ile Tyr Pro Gly Asp Ser Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Trp Gly Thr Tyr Tyr Asp Ile Leu Thr Gly Tyr Phe Asn Trp
            100                 105                 110

Phe Asp Pro Trp Gly Gln Gly Thr Leu Val Thr Val Ser Ser
        115                 120                 125

<210> 50
<211> 8

<212> PRT
<213> homo sapiens

<400> 50

Gly Tyr Asn Phe Thr Ser Tyr Trp
1 5

<210> 51
<211> 8
<212> PRT
<213> homo sapiens

<400> 51

Ile Tyr Pro Gly Asp Ser Asp Thr
1 5

<210> 52
<211> 15
<212> PRT
<213> homo sapiens

<400> 52

Ala Arg Trp Gly Thr Tyr Tyr Asp Ile Leu Thr Gly Tyr Phe Asn
1 5 10 15

<210> 53
<211> 107
<212> PRT
<213> homo sapiens

<400> 53

Asp Ile Gln Met Thr Gln Ser Pro Ser Ser Leu Ser Ala Ser Val Gly
1 5 10 15

Asp Arg Val Thr Ile Thr Cys Arg Ala Ser Gln Gly Ile Ser Ser Trp
20 25 30

Leu Ala Trp Tyr Gln Gln Lys Pro Glu Lys Ala Pro Lys Ser Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Tyr Ile Tyr Pro Trp
85 90 95

Thr Phe Gly Gln Gly Thr Lys Val Glu Ile Lys

100 105

<210> 54
<211> 6
<212> PRT
<213> homo sapiens

<400> 54

Gln Gly Ile Ser Ser Trp
1 5

<210> 55
<211> 9
<212> PRT
<213> homo sapiens

<400> 55

Gln Gln Tyr Tyr Ile Tyr Pro Trp Thr
1 5

<210> 56
<211> 124
<212> PRT
<213> homo sapiens

<400> 56

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Asn Tyr
20 25 30

Gly Met Ser Trp Val Arg Gln Ala Pro Gly Lys Gly Leu Glu Trp Val
35 40 45

Ser Ala Ile Ser Gly Ser Ala Tyr Ser Thr Tyr Tyr Ala Asp Ser Val
50 55 60

Lys Gly Arg Phe Thr Ile Ser Arg Asp Asn Ser Lys Asn Thr Leu Trp
65 70 75 80

Leu Gln Met Asn Ser Leu Arg Ala Glu Asp Thr Ala Val Tyr Tyr Cys
85 90 95

Ala Lys Ala His Tyr His Gly Ser Gly Ser Tyr Tyr Thr Leu Phe Asp
100 105 110

Tyr Trp Gly Gln Gly Thr Leu Val Thr Val Ser Ser
115 120

<210> 57
<211> 8
<212> PRT
<213> homo sapiens

<400> 57

Gly Phe Thr Phe Ser Asn Tyr Gly
1 5

<210> 58
<211> 8
<212> PRT
<213> homo sapiens

<400> 58

Ile Ser Gly Ser Ala Tyr Ser Thr
1 5

<210> 59
<211> 17
<212> PRT
<213> homo sapiens

<400> 59

Ala Lys Ala His Tyr His Gly Ser Gly Ser Tyr Tyr Thr Leu Phe Asp
1 5 10 15

Tyr

<210> 60
<211> 107
<212> PRT
<213> homo sapiens

<400> 60

Asp Ile Gln Met Thr Gln Ser Pro Ser Ser Leu Ser Ala Ser Val Gly
1 5 10 15

Asp Arg Val Thr Ile Thr Cys Arg Ala Ser Gln Gly Ile Ser Ser Trp
20 25 30

Leu Ala Trp Tyr Gln Gln Lys Pro Glu Lys Ala Pro Lys Ser Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

```
Glu Asp Phe Ala Thr Tyr Tyr Cys Gln Gln Tyr Asn Ser Tyr Pro Tyr
                85                  90                  95

Thr Phe Gly Gln Gly Thr Lys Leu Glu Ile Lys
            100                 105


<210>  61
<211>  6
<212>  PRT
<213>  homo sapiens

<400>  61

Gln Gly Ile Ser Ser Trp
1               5


<210>  62
<211>  9
<212>  PRT
<213>  homo sapiens

<400>  62

Gln Gln Tyr Asn Ser Tyr Pro Tyr Thr
1               5


<210>  63
<211>  121
<212>  PRT
<213>  homo sapiens

<400>  63

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Val Ile His Trp Val Arg Gln Ala Pro Gly Lys Gly Leu Glu Trp Val
        35                  40                  45

Thr Val Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Ser Ala Glu Asp Thr Ala Met Tyr Tyr Cys
                85                  90                  95
```

Ala Arg Gly Gly Ile Thr Gly Thr Thr Gly Val Phe Asp Tyr Trp Gly
100 105 110

Gln Gly Thr Leu Val Thr Val Ser Ser
115 120

<210> 64
<211> 8
<212> PRT
<213> homo sapiens

<400> 64

Gly Phe Thr Phe Ser Asp Tyr Val
1 5

<210> 65
<211> 8
<212> PRT
<213> homo sapiens

<400> 65

Ile Ser Tyr Asp Gly Ser Asn Lys
1 5

<210> 66
<211> 14
<212> PRT
<213> homo sapiens

<400> 66

Ala Arg Gly Gly Ile Thr Gly Thr Thr Gly Val Phe Asp Tyr
1 5 10

<210> 67
<211> 107
<212> PRT
<213> homo sapiens

<400> 67

Asp Ile Gln Met Thr Gln Ser Pro Ser Ser Leu Ser Ala Ser Val Gly
1 5 10 15

Asp Arg Val Thr Ile Thr Cys Arg Ala Ser Gln Gly Ile Ser Ser Trp
20 25 30

Leu Ala Trp Tyr Gln Gln Lys Pro Glu Lys Ala Pro Lys Ser Leu Ile
35 40 45

Tyr Asp Ala Ser Ser Leu Gln Ser Gly Val Pro Ser Arg Phe Ser Gly
50 55 60

Ser Gly Tyr Gly Thr Asp Phe Ser Leu Thr Ile Ser Ser Leu Gln Pro
65 70 75 80

Glu Asp Phe Ala Ile Tyr Tyr Cys Gln Gln Tyr Lys Ser Tyr Pro Ile
85 90 95

Thr Phe Gly Gln Gly Thr Arg Leu Glu Ile Lys
100 105

<210> 68
<211> 6
<212> PRT
<213> homo sapiens

<400> 68

Gln Gly Ile Ser Ser Trp
1 5

<210> 69
<211> 9
<212> PRT
<213> homo sapiens

<400> 69

Gln Gln Tyr Lys Ser Tyr Pro Ile Thr
1 5

<210> 70
<211> 125
<212> PRT
<213> homo sapiens

<400> 70

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Gly Ile Ser Trp Val Arg Gln Ala Pro Gly Gln Gly Leu Glu Trp Met
35 40 45

Gly Trp Ile Ser Ala Tyr Asn Gly Asn Ser Asn Tyr Val Gln Lys Phe
50 55 60

Gln Gly Arg Val Thr Met Thr Thr Asp Thr Thr Thr Ser Thr Ala Tyr
65 70 75 80

Met Glu Leu Arg Ser Leu Thr Ser Asp Asp Thr Ala Val Tyr Tyr Cys

```
                85                  90                  95

Ala Arg Glu Tyr Ser Tyr Asp Ser Gly Thr Tyr Phe Tyr Tyr Gly Met
            100                 105                 110

Asp Val Trp Gly Gln Gly Thr Thr Val Thr Val Ser Ser
        115                 120                 125


<210>  71
<211>  8
<212>  PRT
<213>  homo sapiens

<400>  71

Gly Tyr Thr Phe Thr Ser Tyr Gly
1               5


<210>  72
<211>  8
<212>  PRT
<213>  homo sapiens

<400>  72

Ile Ser Ala Tyr Asn Gly Asn Ser
1               5


<210>  73
<211>  18
<212>  PRT
<213>  homo sapiens

<400>  73

Ala Arg Glu Tyr Ser Tyr Asp Ser Gly Thr Tyr Phe Tyr Tyr Gly Met
1               5                   10                  15

Asp Val


<210>  74
<211>  108
<212>  PRT
<213>  homo sapiens

<400>  74

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30
```

Leu Ala Trp Tyr Gln Gln Lys Pro Gly Gln Ala Pro Arg Leu Leu Ile
35 40 45

Tyr Asp Ala Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Arg Ser Asn Trp Pro Met
85 90 95

Tyr Thr Phe Gly Gln Gly Thr Lys Leu Glu Ile Lys
100 105

<210> 75
<211> 6
<212> PRT
<213> homo sapiens

<400> 75

Gln Ser Val Ser Ser Tyr
1 5

<210> 76
<211> 10
<212> PRT
<213> homo sapiens

<400> 76

Gln Gln Arg Ser Asn Trp Pro Met Tyr Thr
1 5 10

<210> 77
<211> 119
<212> PRT
<213> homo sapiens

<400> 77

Glu Val Gln Leu Leu Glu Ser Gly Gly Asp Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Arg Gly Gly Thr Thr Tyr Tyr Ala Asp Ser Val
50 55 60

Lys Gly Arg Phe Thr Ile Ser Arg Asp Asn Ser Lys Ser Thr Leu Cys
65 70 75 80

Leu Gln Met Asn Ser Leu Arg Ala Glu Asp Thr Ala Val Tyr Tyr Cys
85 90 95

Ala Lys Ala Arg Ala Asn Trp Asp Tyr Phe Asp Tyr Trp Gly Gln Gly
100 105 110

Thr Leu Val Thr Val Ser Ser
115

<210> 78
<211> 107
<212> PRT
<213> homo sapiens

<400> 78

Asp Ile Gln Met Thr Gln Ser Pro Ser Ser Val Ser Ala Ser Val Gly
1 5 10 15

Asp Arg Val Thr Ile Thr Cys Arg Ala Ser Gln Gly Ile Ser Ser Trp
20 25 30

Leu Ala Trp Tyr Gln His Lys Pro Gly Lys Ala Pro Lys Leu Leu Ile
35 40 45

Tyr Ala Ala Ser Ile Leu Gln Ser Gly Val Pro Ser Arg Phe Ser Gly
50 55 60

Ser Gly Ser Gly Thr Asp Phe Thr Leu Thr Ile Ser Ser Leu Arg Pro
65 70 75 80

Glu Asp Phe Ala Thr Tyr Tyr Cys Gln Gln Ala Asn Ser Phe Pro Ile
85 90 95

Thr Phe Gly Gln Gly Thr Arg Leu Glu Ile Lys
100 105

<210> 79
<211> 119
<212> PRT
<213> homo sapiens

<400> 79

Glu Val Gln Leu Leu Glu Ser Gly Gly Asp Leu Val Gln Pro Gly Gly
1 5 10 15

```
Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Arg Gly Gly Thr Thr Tyr Tyr Ala Asp Ser Val
    50                  55                  60

Lys Gly Arg Phe Thr Ile Ser Arg Asp Asn Ser Lys Ser Thr Leu Cys
65                  70                  75                  80

Leu Gln Met Asn Ser Leu Arg Ala Glu Asp Thr Ala Val Tyr Tyr Cys
                85                  90                  95

Ala Lys Ala Arg Ala Asn Trp Asp Tyr Phe Asp Tyr Trp Gly Gln Gly
            100                 105                 110

Thr Leu Val Thr Val Ser Ser
        115


<210>  80
<211>  107
<212>  PRT
<213>  homo sapiens

<400>  80

Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln His Lys Pro Gly Lys Ala Pro Lys Leu Leu Ile
        35                  40                  45

Tyr Ala Ala Ser Ile Leu Gln Ser Gly Val Pro Ser Arg Phe Ser Gly
    50                  55                  60

Ser Gly Ser Gly Thr Asp Phe Thr Leu Thr Ile Ser Ser Leu Arg Pro
65                  70                  75                  80

Glu Asp Phe Ala Thr Tyr Tyr Cys Gln Gln Ala Asn Ser Phe Pro Ile
                85                  90                  95

Thr Phe Gly Gln Gly Thr Arg Leu Glu Ile Lys
            100                 105
```

<210> 81
<211> 119
<212> PRT
<213> homo sapiens

<400> 81

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Asn Trp Val Arg Gln Ala Pro Gly Lys Gly Leu Glu Trp Val
        35                  40                  45

Ser Ala Ile Ser Gly Arg Gly Gly Thr Thr Tyr Tyr Ala Asp Ser Val
    50                  55                  60

Lys Gly Arg Phe Thr Ile Ser Arg Asp Asn Ser Lys Ser Thr Leu Cys
65                  70                  75                  80

Leu Gln Met Asn Ser Leu Arg Ala Glu Asp Thr Ala Val Tyr Tyr Cys
                85                  90                  95

Ala Lys Ala Arg Ala Asn Trp Asp Tyr Phe Asp Tyr Trp Gly Gln Gly
            100                 105                 110

Thr Leu Val Thr Val Ser Ser
        115

<210> 82
<211> 107
<212> PRT
<213> homo sapiens

<400> 82

Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln His Lys Pro Gly Lys Ala Pro Lys Leu Leu Ile
        35                  40                  45

Tyr Ala Ala Ser Ile Leu Gln Ser Gly Val Pro Ser Arg Phe Ser Gly
    50                  55                  60

Ser Gly Ser Gly Thr Asp Phe Thr Leu Thr Ile Ser Ser Leu Arg Pro
65 70 75 80

Glu Asp Phe Ala Thr Tyr Tyr Cys Gln Gln Ala Asn Ser Phe Pro Ile
85 90 95

Thr Phe Gly Gln Gly Thr Arg Leu Glu Ile Lys
100 105

<210> 83
<211> 121
<212> PRT
<213> homo sapiens

<400> 83

Gln Val Gln Leu Val Gln Ser Gly Ala Glu Val Lys Lys Pro Gly Ala
1 5 10 15

Ser Val Lys Val Ser Cys Lys Ala Ala Gly Tyr Thr Phe Thr Asn Tyr
20 25 30

Gly Ile Ser Trp Val Arg Gln Ala Pro Gly Gln Ala Leu Glu Trp Met
35 40 45

Gly Trp Ile Thr Thr Tyr Ser Ser Asn Thr Ile Tyr Ala Gln Lys Leu
50 55 60

Gln Gly Arg Val Thr Met Thr Thr Asp Thr Ser Thr Ser Thr Ala Tyr
65 70 75 80

Met Glu Leu Arg Ser Leu Arg Ser Asp Asp Thr Ala Val Tyr Tyr Cys
85 90 95

Ala Arg Asp Arg Val Val Val Arg Pro Asp Tyr Phe Asp Tyr Trp Gly
100 105 110

Gln Gly Thr Leu Val Thr Val Ser Ser
115 120

<210> 84
<211> 107
<212> PRT
<213> homo sapiens

<400> 84

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr

20 25 30

Leu Ala Trp Tyr Gln Gln Lys Pro Gly Gln Ala Pro Arg Leu Leu Ile
35 40 45

Tyr Asp Thr Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Arg Ser His Trp Pro Arg
85 90 95

Thr Phe Gly Gln Gly Thr Lys Val Glu Ile Lys
100 105

<210> 85
<211> 121
<212> PRT
<213> homo sapiens

<400> 85

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asn Tyr
20 25 30

Gly Ile Ser Trp Val Arg Gln Ala Pro Gly Gln Gly Leu Glu Trp Met
35 40 45

Gly Trp Leu Ser Ala Tyr Ser Gly Asn Thr Ile Tyr Ala Gln Lys Leu
50 55 60

Gln Gly Arg Val Thr Met Thr Thr Asp Thr Ser Thr Thr Thr Ala Tyr
65 70 75 80

Met Glu Leu Arg Ser Leu Arg Ser Asp Asp Thr Ala Val Tyr Tyr Cys
85 90 95

Ala Arg Asp Arg Ile Val Val Arg Pro Asp Tyr Phe Asp Tyr Trp Gly
100 105 110

Gln Gly Thr Leu Val Thr Val Ser Ser
115 120

<210> 86

<211> 107
<212> PRT
<213> homo sapiens

<400> 86

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Arg
                85                  90                  95

Thr Phe Gly Gln Gly Thr Lys Val Glu Ile Lys
            100                 105

<210> 87
<211> 121
<212> PRT
<213> homo sapiens

<400> 87

Gln Val Gln Leu Val Gln Ser Gly Ala Glu Val Lys Lys Pro Gly Ala
1               5                   10                  15

Ser Val Lys Val Ser Cys Lys Ala Ala Gly Tyr Thr Phe Thr Asn Tyr
            20                  25                  30

Gly Ile Ser Trp Val Arg Gln Ala Pro Gly Gln Gly Leu Glu Trp Met
        35                  40                  45

Gly Trp Ile Ile Thr Tyr Asn Gly Asn Thr Ile Tyr Ala Gln Arg Phe
    50                  55                  60

Gln Asp Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Arg Ile Ile Val Arg Pro Asp Tyr Phe Asp Tyr Trp Gly
100 105 110

Gln Gly Thr Leu Val Thr Val Ser Ser
115 120

<210> 88
<211> 107
<212> PRT
<213> homo sapiens

<400> 88

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Tyr Asp Ala Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Arg Ser Asn Trp Pro Arg
85 90 95

Thr Phe Gly Gln Gly Thr Lys Val Glu Ile Lys
100 105

<210> 89
<211> 120
<212> PRT
<213> homo sapiens

<400> 89

Gln Val Gln Leu Gln Gln Trp Gly Ala Gly Leu Leu Lys Pro Ser Glu
1 5 10 15

Thr Leu Ser Leu Thr Cys Ala Val Tyr Gly Gly Ser Phe Ser Gly Tyr
20 25 30

Tyr Trp Asn Trp Ile Arg Gln Pro Pro Gly Lys Gly Leu Glu Trp Ile
35 40 45

```
Gly Glu Ile Asn His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Gly Asn Tyr Gly Ser Gly Tyr Tyr Tyr Phe Asp Leu Trp Gly Arg
            100                 105                 110

Gly Thr Gln Val Thr Val Ser Ser
        115                 120


<210>  90
<211>  107
<212>  PRT
<213>  homo sapiens

<400>  90

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Glu Lys Ala Pro Lys Ser Leu Ile
        35                  40                  45

Phe Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ile Ser Phe Pro Ile
                85                  90                  95

Thr Phe Gly Gln Gly Thr Arg Leu Glu Ile Lys
            100                 105


<210>  91
<211>  120
<212>  PRT
<213>  homo sapiens

<400>  91
```

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Asn Trp Ile Arg Gln Pro Pro Gly Lys Gly Leu Glu Trp Ile
        35                  40                  45

Gly Glu Ile His His Ser Gly Ser Ala Asn Tyr Asn Pro Ser Leu Met
    50                  55                  60

Ser Arg Val Thr Ile Ser Val Asp Thr Ser Lys Asn Gln Phe Ser Leu
65                  70                  75                  80

Gln Leu Ser Ser Val Thr Ala Ala Asp Thr Ala Val Tyr Tyr Cys Ala
                85                  90                  95

Arg Gly Tyr Tyr Gly Ser Gly Tyr Tyr Tyr Phe Asp Tyr Trp Gly Gln
            100                 105                 110

Gly Thr Leu Val Thr Val Ser Ser
        115                 120

<210> 92
<211> 107
<212> PRT
<213> homo sapiens

<400> 92

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Glu Lys Ala Pro Lys Ser Leu Ile
        35                  40                  45

Tyr Ala Ala Ser Arg Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser Tyr Pro Ile
                85                  90                  95

Thr Phe Gly Gln Gly Thr Arg Leu Glu Ile Lys
100 105

<210> 93
<211> 120
<212> PRT
<213> homo sapiens

<400> 93

Gln Val Gln Leu Gln Gln Trp Gly Ala Gly Leu Leu Lys Pro Ser Glu
1 5 10 15

Thr Leu Ser Leu Thr Cys Ala Val Tyr Gly Gly Ser Phe Ser Asp Tyr
20 25 30

Tyr Trp Asn Trp Ile Arg Gln Pro Pro Gly Lys Gly Leu Glu Trp Ile
35 40 45

Gly Glu Ile His His Val Gly Ser Thr Asn Tyr Asn Pro Ser Leu Lys
50 55 60

Ser Arg Val Thr Ile Ser Val Asp Thr Ser Lys Ser Gln Phe Ser Leu
65 70 75 80

Lys Leu Ser Ser Val Thr Ala Ala Asp Thr Ala Val Tyr Tyr Cys Ala
85 90 95

Arg Gly Tyr Tyr Asp Ser Gly Val Tyr Tyr Phe Asp Tyr Trp Ala Gln
100 105 110

Gly Thr Leu Val Thr Val Ser Ser
115 120

<210> 94
<211> 107
<212> PRT
<213> homo sapiens

<400> 94

Asp Ile Gln Met Thr Gln Ser Pro Ser Ser Leu Ser Ala Ser Val Gly
1 5 10 15

Asp Arg Val Thr Ile Thr Cys Arg Ala Ser Gln Gly Ile Ser Arg Trp
20 25 30

Leu Ala Trp Tyr Gln Gln Lys Pro Glu Lys Ala Pro Lys Ser Leu Ile
35 40 45

Tyr Ala Ala Ser Ser Leu Arg Ser Gly Val Pro Ser Arg Phe Ser Gly

50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Asn Ser Tyr Pro Ile
85 90 95

Thr Phe Gly Gln Gly Thr Arg Leu Glu Ile Lys
100 105

<210> 95
<211> 120
<212> PRT
<213> homo sapiens

<400> 95

Gln Val Gln Leu Gln Gln Trp Gly Ala Gly Leu Leu Lys Pro Ser Glu
1 5 10 15

Thr Leu Ser Leu Thr Cys Ala Val Tyr Gly Gly Ser Phe Ser Asp Tyr
20 25 30

Tyr Trp Asn Trp Ile Arg Gln Pro Pro Gly Lys Gly Leu Glu Trp Ile
35 40 45

Gly Glu Ile His His Ser Gly Ser Thr Asn Tyr Asn Pro Ser Leu Lys
50 55 60

Ser Arg Val Thr Ile Ser Val Asp Thr Ser Lys Asn Gln Phe Ser Leu
65 70 75 80

Lys Leu Ser Ser Val Thr Ala Ala Asp Thr Ala Val Tyr Tyr Cys Ala
85 90 95

Arg Gly Tyr Tyr Ala Ser Gly Val Tyr Tyr Phe Asp Tyr Trp Gly Gln
100 105 110

Gly Thr Leu Val Thr Val Ser Ser
115 120

<210> 96
<211> 107
<212> PRT
<213> homo sapiens

<400> 96

Asp Ile Gln Met Thr Gln Ser Pro Ser Ser Leu Ser Ala Ser Val Gly
1 5 10 15

```
Asp Arg Val Thr Ile Thr Cys Arg Ala Ser Gln Gly Ile Ser Ser Trp
            20                  25                  30

Leu Ala Trp Tyr Gln Gln Lys Pro Glu Lys Ala Pro Lys Ser Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser Tyr Pro Ile
                85                  90                  95

Thr Phe Gly Gln Gly Thr Arg Leu Glu Ile Lys
            100                 105


<210>  97
<211>  120
<212>  PRT
<213>  homo sapiens

<400>  97

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Asp Tyr
            20                  25                  30

Phe Trp Asn Trp Ile Arg Gln Pro Pro Gly Lys Gly Leu Glu Trp Ile
        35                  40                  45

Gly Glu Ile His His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Asn Leu Ser Ser Val Thr Ala Ala Asp Thr Ala Val Tyr Tyr Cys Ala
                85                  90                  95

Arg Gly Leu Ile Gly Ser Gly Tyr Tyr Tyr Phe Asp Tyr Trp Asp Gln
            100                 105                 110

Gly Thr Leu Val Thr Val Ser Ser
        115                 120
```

```
<210>  98
<211>  107
<212>  PRT
<213>  homo sapiens

<400>  98

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Glu Lys Ala Pro Lys Ser Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser Tyr Pro Ile
                85                  90                  95

Thr Phe Gly Gln Gly Thr Arg Leu Glu Ile Lys
            100                 105


<210>  99
<211>  120
<212>  PRT
<213>  homo sapiens

<400>  99

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asn His Ser Gly Asp Thr Asn Tyr Asn Pro Ser Leu Thr
    50                  55                  60

Ser Arg Val Thr Ile Ser Val Asp Thr Ser Lys Asn Gln Phe Ser Leu
65                  70                  75                  80
```

Lys Leu Ser Ser Val Thr Ala Ala Asp Thr Ala Val Tyr Tyr Cys Ala
85 90 95

Arg Leu Phe Tyr Gly Ser Gly Ile Tyr Tyr Phe Asp Tyr Trp Gly Gln
100 105 110

Gly Thr Leu Val Thr Val Ser Ser
115 120

<210> 100
<211> 107
<212> PRT
<213> homo sapiens

<400> 100

Asp Ile Gln Met Thr Gln Ser Pro Ser Ser Leu Ser Ala Ser Val Gly
1 5 10 15

Asp Arg Val Thr Ile Thr Cys Arg Ala Ser Gln Gly Ile Ser Ser Trp
20 25 30

Leu Ala Trp Tyr Gln Gln Lys Pro Glu Lys Ala Pro Lys Ser Leu Ile
35 40 45

Tyr Ala Thr Phe Arg Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Asn Ser Phe Pro Pro
85 90 95

Thr Phe Gly Gln Gly Thr Lys Val Glu Ile Lys
100 105

<210> 101
<211> 120
<212> PRT
<213> homo sapiens

<400> 101

Gln Val Gln Leu Gln Gln Trp Gly Ala Gly Leu Leu Lys Pro Ser Glu
1 5 10 15

Thr Leu Ser Leu Thr Cys Ala Ile Tyr Gly Gly Ser Phe Ser Gly Tyr
20 25 30

```
Tyr Trp Ser Trp Ile Arg Gln Pro Pro Gly Lys Gly Leu Glu Trp Ile
        35                  40                  45

Gly Glu Ile Asn His Ser Gly Asp Thr Asn Tyr Asn Pro Ser Leu Thr
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Leu Phe Tyr Gly Ser Gly Ile Tyr Tyr Phe Asp Tyr Trp Gly Gln
            100                 105                 110

Gly Thr Leu Val Thr Val Ser Ser
        115                 120


<210>  102
<211>  107
<212>  PRT
<213>  homo sapiens

<400>  102

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Glu Lys Ala Pro Lys Ser Leu Ile
        35                  40                  45

Tyr Ala Thr Phe Arg Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser Phe Pro Pro
                85                  90                  95

Thr Phe Gly Gln Gly Thr Lys Val Glu Ile Lys
            100                 105


<210>  103
<211>  120
<212>  PRT
<213>  homo sapiens
```

<400> 103

Gln Val Gln Leu Gln Gln Trp Gly Ala Gly Leu Leu Lys Pro Ser Glu
1 5 10 15

Thr Leu Ser Leu Thr Cys Ala Val Tyr Gly Gly Ser Phe Ser Asp Tyr
20 25 30

Tyr Trp Ser Trp Ile Arg Gln Pro Pro Gly Lys Gly Leu Glu Trp Ile
35 40 45

Gly Glu Ile Asn His Ser Gly Ser Thr Asn Tyr Asn Pro Ser Leu Lys
50 55 60

Ser Arg Val Thr Ile Ser Val Asp Thr Ser Lys Asn Gln Phe Ser Leu
65 70 75 80

Lys Leu Ser Ser Val Thr Ala Ala Asp Thr Ala Val Tyr Tyr Cys Ala
85 90 95

Arg Leu Tyr Tyr Gly Ser Gly Thr Tyr Tyr Phe Asp Tyr Trp Gly Gln
100 105 110

Gly Thr Leu Val Thr Val Ser Ser
115 120

<210> 104
<211> 107
<212> PRT
<213> homo sapiens

<400> 104

Asp Ile Gln Met Thr Gln Ser Pro Ser Ser Leu Ser Ala Ser Val Gly
1 5 10 15

Asp Arg Val Thr Ile Thr Cys Arg Ala Ser Gln Gly Ile Ser Ser Trp
20 25 30

Leu Thr Trp Tyr Gln Gln Lys Pro Glu Lys Ala Pro Lys Ser Leu Ile
35 40 45

Tyr Ala Ala Ser Arg Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Asn Ser Phe Pro Pro

85 90 95

Thr Phe Gly Gln Gly Thr Lys Val Glu Ile Lys
100 105

<210> 105
<211> 120
<212> PRT
<213> homo sapiens

<400> 105

Gln Val Gln Leu Gln Gln Trp Gly Ala Gly Leu Leu Lys Pro Ser Glu
1 5 10 15

Thr Leu Ser Leu Thr Cys Ala Val Ser Gly Gly Ser Phe Ser Gly Tyr
20 25 30

Tyr Trp Thr Trp Ile Arg Gln Pro Pro Gly Lys Gly Leu Glu Trp Ile
35 40 45

Gly Glu Ile Tyr His Ser Gly Asp Thr Asn Tyr Asn Pro Ser Leu Lys
50 55 60

Ser Arg Val Thr Ile Ser Val Asp Thr Ser Lys Asn Gln Phe Ser Leu
65 70 75 80

Lys Leu Tyr Ser Val Thr Ala Ala Asp Thr Ala Val Tyr Tyr Cys Ala
85 90 95

Arg Leu Tyr Phe Gly Ser Gly Ile Tyr Tyr Leu Asp Tyr Trp Gly Gln
100 105 110

Gly Thr Leu Val Thr Val Ser Ser
115 120

<210> 106
<211> 107
<212> PRT
<213> homo sapiens

<400> 106

Asp Ile Gln Met Thr Gln Ser Pro Ser Ser Leu Ser Ala Ser Val Gly
1 5 10 15

Asp Arg Val Thr Ile Thr Cys Arg Ala Ser Gln Gly Ile Ser Ser Trp
20 25 30

Leu Val Trp Tyr Gln Gln Lys Pro Glu Lys Ala Pro Lys Ser Leu Ile
35 40 45

```
Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser Phe Pro Pro
                85                  90                  95

Thr Phe Gly Gln Gly Thr Lys Val Glu Ile Lys
            100                 105


<210>  107
<211>  124
<212>  PRT
<213>  homo sapiens

<400>  107

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Gly Met Ser Trp Val Arg Gln Ala Pro Gly Lys Gly Leu Glu Trp Val
        35                  40                  45

Ser Ala Ile Ser Gly Ser Ala Tyr Ser Thr Tyr Tyr Ala Asp Ser Val
    50                  55                  60

Lys Gly Arg Phe Thr Ile Ser Arg Asp Asn Ser Lys Asn Thr Leu Trp
65                  70                  75                  80

Leu Gln Met Asn Ser Leu Arg Ala Glu Asp Thr Ala Val Tyr Tyr Cys
                85                  90                  95

Ala Lys Ala His Tyr His Gly Ser Gly Ser Tyr Tyr Thr Leu Phe Asp
            100                 105                 110

Tyr Trp Gly Gln Gly Thr Leu Val Thr Val Ser Ser
        115                 120


<210>  108
<211>  107
<212>  PRT
<213>  homo sapiens

<400>  108
```

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Glu Lys Ala Pro Lys Ser Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser Tyr Pro Tyr
                85                  90                  95

Thr Phe Gly Gln Gly Thr Lys Leu Glu Ile Lys
            100                 105


<210>  109
<211>  124
<212>  PRT
<213>  homo sapiens

<400>  109

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Asn Asn Tyr
            20                  25                  30

Gly Met Asn Trp Val Arg Gln Ala Pro Gly Lys Gly Leu Glu Trp Val
        35                  40                  45

Ser Ala Ile Ser Gly Thr Gly Tyr Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala His Tyr Phe Gly Ser Gly Ser Tyr Tyr Thr Leu Phe Asp
            100                 105                 110
```

```
Tyr Trp Gly Gln Gly Thr Leu Val Thr Val Ser Ser
        115                 120


<210>  110
<211>  107
<212>  PRT
<213>  homo sapiens

<400>  110

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Gly Ile Ser Ser Trp
            20                  25                  30


Leu Ala Trp Tyr Gln Gln Lys Pro Glu Lys Ala Pro Lys Ser Leu Ile
        35                  40                  45


Tyr Ala Ala Ser Ser Leu Gln Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Tyr Asn Ser Tyr Pro Tyr
                85                  90                  95


Thr Phe Gly Gln Gly Thr Lys Leu Glu Ile Lys
            100                 105


<210>  111
<211>  124
<212>  PRT
<213>  homo sapiens

<400>  111

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Thr Asp Tyr
            20                  25                  30


Ala Met Asn Trp Val Arg Gln Ala Pro Gly Lys Gly Leu Glu Trp Val
        35                  40                  45


Ser Thr Ile Ser Gly Ser Gly Tyr Ala Thr Tyr Tyr Ala Asp Ser Val
    50                  55                  60
```

Lys Gly Arg Phe Thr Ile Ser Arg Asp Asn Ser Lys Thr Thr Leu Tyr
65 70 75 80

Leu Gln Met Asn Ser Leu Arg Ala Glu Asp Thr Ala Val Tyr Tyr Cys
85 90 95

Ala Lys Gly His Thr Leu Gly Ser Gly Ser Tyr Tyr Thr Leu Phe Asp
100 105 110

Tyr Trp Gly Gln Gly Thr Leu Val Thr Val Ser Ser
115 120

<210> 112
<211> 107
<212> PRT
<213> homo sapiens

<400> 112

Asp Ile Gln Met Thr Gln Ser Pro Ser Ser Leu Ser Ala Ser Val Gly
1 5 10 15

Asp Arg Val Thr Ile Thr Cys Arg Ala Ser Gln Gly Ile Asn Ser Trp
20 25 30

Leu Ala Trp Tyr Gln Gln Lys Pro Glu Lys Ala Pro Lys Ser Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Asn Ser Tyr Pro Tyr
85 90 95

Thr Phe Gly Gln Gly Thr Lys Leu Glu Ile Lys
100 105

<210> 113
<211> 124
<212> PRT
<213> homo sapiens

<400> 113

Glu Val Gln Leu Trp Glu Ser Gly Gly Gly Ser Val Gln Pro Gly Gly
1 5 10 15

Ser Leu Arg Leu Ser Cys Ala Ala Ser Gly Phe Thr Phe Ser Ser Tyr

```
            20                  25                  30

Gly Met Ser Trp Val Arg Gln Ala Pro Gly Lys Gly Leu Glu Trp Val
        35                  40                  45

Ser Gly Ile Ser Gly Ser Gly Tyr Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Tyr Tyr His Gly Ser Gly Ser Tyr Tyr Thr Ser Phe Asp
            100                 105                 110

Tyr Trp Gly Gln Gly Thr Leu Val Thr Val Ser Ser
        115                 120


<210>  114
<211>  107
<212>  PRT
<213>  homo sapiens

<400>  114

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Glu Lys Ala Pro Lys Ser Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser Tyr Pro Leu
                85                  90                  95

Thr Phe Gly Gly Gly Thr Lys Val Glu Ile Lys
            100                 105


<210>  115
```

<211> 121
<212> PRT
<213> homo sapiens

<400> 115

Gln Val Gln Leu Val Glu Ser Gly Gly Gly Val Val Gln Thr Gly Arg
1 5 10 15

Ser Leu Arg Leu Ser Cys Ala Ala Ser Gly Phe Thr Phe Ser Ser His
20 25 30

Ala Met His Trp Val Arg Gln Ala Pro Gly Lys Gly Leu Glu Trp Val
35 40 45

Ala Ala Ile Ser Tyr Asp Gly Ser Asn Lys Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Gly Asp Tyr Ile Ser Ser Ser Gly Val Phe Asp Tyr Trp Gly
100 105 110

Gln Gly Thr Leu Val Thr Val Ser Ser
115 120

<210> 116
<211> 107
<212> PRT
<213> homo sapiens

<400> 116

Asp Ile Gln Met Thr Gln Ser Pro Ser Ser Leu Ser Ala Ser Val Gly
1 5 10 15

Asp Arg Val Thr Ile Thr Cys Arg Ala Ser Gln Gly Ile Ser Ser Trp
20 25 30

Leu Ala Trp Tyr Gln Gln Lys Pro Glu Lys Ala Pro Lys Ser Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

```
Glu Asp Phe Ala Thr Tyr Tyr Cys Gln Gln Tyr Asn Ser Tyr Pro Ile
                85                  90                  95

Thr Phe Gly Gln Gly Thr Arg Leu Glu Ile Lys
            100                 105


<210>  117
<211>  121
<212>  PRT
<213>  homo sapiens

<400>  117

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser His
            20                  25                  30

Ala Met His Trp Val Arg Gln Ala Pro Gly Lys Gly Leu Glu Trp Val
        35                  40                  45

Ala Ala Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Met Tyr
65                  70                  75                  80

Leu Gln Met Asn Ser Leu Arg Ala Glu Asp Thr Ala Met Cys Tyr Cys
                85                  90                  95

Ala Arg Gly Ser Ile Thr Gly Ser Thr Gly Val Phe Asp Tyr Trp Gly
            100                 105                 110

Gln Gly Thr Leu Val Thr Val Ser Ser
        115                 120


<210>  118
<211>  107
<212>  PRT
<213>  homo sapiens

<400>  118

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Ser Trp
            20                  25                  30
```

```
Leu Ala Trp Tyr Gln Gln Lys Pro Glu Lys Ala Pro Lys Ser Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser Tyr Pro Ile
                85                  90                  95

Thr Phe Gly Gln Gly Thr Arg Leu Glu Ile Lys
            100                 105
```

<210>  119
<211>  121
<212>  PRT
<213>  homo sapiens

<400>  119

```
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ala Val Ile Ser Tyr Asp Gly Ser Asn Glu Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Ser Ile Ile Gly Ser Thr Gly Val Phe Asp Tyr Trp Gly
            100                 105                 110

Gln Gly Thr Leu Val Thr Val Ser Ser
        115                 120
```

<210>  120
<211>  107
<212>  PRT

<213> homo sapiens

<400> 120

Asp Ile Gln Met Thr Gln Ser Pro Ser Ser Leu Ser Ala Ser Val Gly
1 5 10 15

Asp Arg Val Thr Ile Thr Cys Arg Ala Ser Gln Gly Ile Ser Asn Trp
20 25 30

Leu Ala Trp Tyr Gln Gln Lys Pro Glu Lys Ala Pro Lys Ser Leu Ile
35 40 45

Tyr Asp Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Asn Ser Tyr Pro Ile
85 90 95

Thr Phe Gly Gln Gly Thr Arg Leu Glu Ile Lys
100 105

<210> 121
<211> 121
<212> PRT
<213> homo sapiens

<400> 121

Gln Val Gln Val Val Glu Ser Gly Gly Gly Val Val Gln Pro Gly Arg
1 5 10 15

Ser Leu Arg Leu Ser Cys Ala Ala Ser Gly Phe Thr Phe Ser Ser Tyr
20 25 30

Ala Met His Trp Val Arg Gln Ala Pro Gly Lys Gly Leu Glu Trp Val
35 40 45

Ala Val Ile Ser Tyr Asp Gly Ser Tyr Lys Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Gly Ser Ile Thr Gly Ser Thr Gly Val Phe Asp Tyr Trp Gly
100 105 110

Gln Gly Thr Leu Val Thr Val Ser Ser
115 120

<210> 122
<211> 107
<212> PRT
<213> homo sapiens

<400> 122

Asp Ile Gln Met Thr Gln Ser Pro Ser Ser Leu Ser Ala Ser Val Gly
1 5 10 15

Asp Arg Val Thr Ile Thr Cys Arg Ala Ser Gln Gly Ile Asn Ser Trp
20 25 30

Leu Ala Trp Tyr Gln Gln Lys Pro Glu Lys Ala Pro Lys Ser Leu Ile
35 40 45

Tyr Asp Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asn Phe Ala Thr Tyr Tyr Cys Gln Gln Tyr Asn Ser Tyr Pro Ile
85 90 95

Thr Phe Gly Gln Gly Thr Arg Leu Glu Ile Lys
100 105

<210> 123
<211> 121
<212> PRT
<213> homo sapiens

<400> 123

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Ser Tyr
20 25 30

Ala Ile His Trp Val Arg Gln Ala Pro Gly Lys Gly Leu Glu Trp Val
35 40 45

Ala Val Ile Ser Tyr Asp Gly Ser Asn Lys Tyr Tyr Ala Asp Ser Val

```
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Ser Ile Thr Gly Ser Thr Gly Val Phe Asp Tyr Trp Gly
            100                 105                 110

Gln Gly Thr Leu Val Thr Val Ser Ser
        115                 120


<210>  124
<211>  107
<212>  PRT
<213>  homo sapiens

<400>  124

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Asn Trp
            20                  25                  30

Leu Ala Trp Tyr Gln Gln Lys Pro Glu Lys Ala Pro Lys Ser Leu Ile
        35                  40                  45

Tyr Asp Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser Tyr Pro Ile
                85                  90                  95

Thr Phe Gly Gln Gly Thr Arg Leu Glu Ile Lys
            100                 105


<210>  125
<211>  8
<212>  PRT
<213>  homo sapiens


<220>
<221>  MISC_FEATURE
<222>  (4)..(4)
```

<223> wherein Xaa=Arg or Ser, preferably Arg

<220>
<221> MISC_FEATURE
<222> (7)..(7)
<223> wherein Xaa=Thr or Ser, preferably Thr

<400> 125

Ile Ser Gly Xaa Gly Gly Xaa Thr
1 5

<210> 126
<211> 8
<212> PRT
<213> homo sapiens

<220>
<221> MISC_FEATURE
<222> (5)..(5)
<223> wherein Xaa=Arg or Ser, preferably Arg

<220>
<221> MISC_FEATURE
<222> (6)..(6)
<223> wherein Xaa=Thr or Ser, preferably Thr

<400> 126

Gly Gly Thr Phe Xaa Xaa Tyr Ala
1 5

<210> 127
<211> 8
<212> PRT
<213> homo sapiens

<220>
<221> MISC_FEATURE
<222> (2)..(2)
<223> wherein Xaa=Asn or Ile, preferably Asn

<220>
<221> MISC_FEATURE
<222> (3)..(3)
<223> wherein Xaa=Thr or Pro, preferably Thr

<220>
<221> MISC_FEATURE
<222> (4)..(4)
<223> wherein Xaa=Val or Ile, preferably Val

<220>
<221> MISC_FEATURE
<222> (8)..(8)
<223> wherein Xaa=Val or Ala, preferably Val

<400> 127

Ile Xaa Xaa Xaa Leu Gly Ile Xaa
1               5

<210>  128
<211>  13
<212>  PRT
<213>  homo sapiens

<220>
<221>  MISC_FEATURE
<222>  (12)..(12)
<223>  wherein Xaa=Ile or Met, preferably Ile

<220>
<221>  MISC_FEATURE
<222>  (13)..(13)
<223>  wherein Xaa=Glu or Asp, preferably Glu

<400>  128

Ala Arg Glu Lys Gly Val Asp Tyr Tyr Tyr Gly Xaa Xaa
1               5                   10

<210>  129
<211>  8
<212>  PRT
<213>  homo sapiens

<220>
<221>  MISC_FEATURE
<222>  (6)..(6)
<223>  wherein Xaa=Asn or Ser, preferably Ser

<400>  129

Gly Tyr Thr Phe Thr Xaa Tyr Gly
1               5

<210>  130
<211>  8
<212>  PRT
<213>  homo sapiens

<220>
<221>  MISC_FEATURE
<222>  (2)..(2)
<223>  wherein Xaa=Ser, Thr, or Ile, preferably Ser

<220>
<221>  MISC_FEATURE
<222>  (3)..(3)
<223>  wherein Xaa=Ala or Thr, preferably Ala

<220>
<221>  MISC_FEATURE
<222>  (5)..(5)
<223>  wherein Xaa=Ser or Asn, preferably Ser

<400> 130

Ile Xaa Xaa Tyr Xaa Gly Asn Thr
1 5

<210> 131
<211> 14
<212> PRT
<213> homo sapiens

<220>
<221> MISC_FEATURE
<222> (5)..(5)
<223> wherein Xaa=Ile or Val, preferably Ile

<220>
<221> MISC_FEATURE
<222> (6)..(6)
<223> wherein Xaa=Val or Ile, preferably Val

<400> 131

Ala Arg Asp Arg Xaa Xaa Val Arg Pro Asp Tyr Phe Asp Tyr
1 5 10

<210> 132
<211> 8
<212> PRT
<213> homo sapiens

<220>
<221> MISC_FEATURE
<222> (6)..(6)
<223> wherein Xaa=Asp or Gly, preferably Asp

<220>
<221> MISC_FEATURE
<222> (8)..(8)
<223> wherein Xaa=Tyr or Phe, preferably Tyr

<400> 132

Gly Gly Ser Phe Ser Xaa Tyr Xaa
1 5

<210> 133
<211> 7
<212> PRT
<213> homo sapiens

<220>
<221> MISC_FEATURE
<222> (2)..(2)
<223> wherein Xaa=His or Asn, preferably His

<220>

<221>  MISC_FEATURE
<222>  (4)..(4)
<223>  wherein Xaa=Ser or Val, preferably Ser

<220>
<221>  MISC_FEATURE
<222>  (7)..(7)
<223>  wherein Xaa=Thr or Ala, preferably Thr

<400>  133

Ile Xaa His Xaa Gly Ser Xaa
1               5

<210>  134
<211>  14
<212>  PRT
<213>  homo sapiens

<220>
<221>  MISC_FEATURE
<222>  (4)..(4)
<223>  wherein Xaa=Tyr, Asn or Leu, preferably Tyr

<220>
<221>  MISC_FEATURE
<222>  (5)..(5)
<223>  wherein Xaa=Tyr or Ile, preferably Tyr

<220>
<221>  MISC_FEATURE
<222>  (6)..(6)
<223>  wherein Xaa=Asp, Gly, or Ala, preferably Asp

<220>
<221>  MISC_FEATURE
<222>  (9)..(9)
<223>  wherein Xaa=Val or Tyr, preferably Val

<220>
<221>  MISC_FEATURE
<222>  (14)..(14)
<223>  wherein Xaa=Tyr or Leu, preferably Tyr

<400>  134

Ala Arg Gly Xaa Xaa Xaa Ser Gly Xaa Tyr Tyr Phe Asp Xaa
1               5                   10

<210>  135
<211>  8
<212>  PRT
<213>  homo sapiens

<220>
<221>  MISC_FEATURE
<222>  (6)..(6)
<223>  wherein Xaa=Gly or Asp, preferably Gly

<400> 135

Gly Gly Ser Phe Ser Xaa Tyr Tyr
1               5

<210> 136
<211> 7
<212> PRT
<213> homo sapiens

<220>
<221> MISC_FEATURE
<222> (2)..(2)
<223> wherein Xaa=Tyr, Asn or His, preferably Tyr

<220>
<221> MISC_FEATURE
<222> (6)..(6)
<223> wherein Xaa=Asp or Ser, preferably Asp

<400> 136

Ile Xaa His Ser Gly Xaa Thr
1               5

<210> 137
<211> 14
<212> PRT
<213> homo sapiens

<220>
<221> MISC_FEATURE
<222> (4)..(4)
<223> wherein Xaa=Tyr, Phe or Trp, preferably Tyr

<220>
<221> MISC_FEATURE
<222> (5)..(5)
<223> wherein Xaa=Phe or Tyr, preferably Phe

<220>
<221> MISC_FEATURE
<222> (9)..(9)
<223> wherein Xaa=Ile, Thr or Ser, preferably Ile

<220>
<221> MISC_FEATURE
<222> (12)..(12)
<223> wherein Xaa=Leu or Phe, preferably Leu

<400> 137

Ala Arg Leu Xaa Xaa Gly Ser Gly Xaa Tyr Tyr Xaa Asp Tyr
1               5                   10

<210> 138
<211> 8
<212> PRT

<213> homo sapiens

<220>
<221> MISC_FEATURE
<222> (6)..(6)
<223> wherein Xaa=Thr or Phe, preferably Thr

<220>
<221> MISC_FEATURE
<222> (7)..(7)
<223> wherein Xaa=Phe or Tyr, preferably Phe

<400> 138

Gly Phe Thr Phe Ser Xaa Xaa Ala
1               5

<210> 139
<211> 8
<212> PRT
<213> homo sapiens

<220>
<221> MISC_FEATURE
<222> (6)..(6)
<223> wherein Xaa=Gly or Ser, preferably Gly

<220>
<221> MISC_FEATURE
<222> (7)..(7)
<223> wherein Xaa=His or Asn, preferably His

<400> 139

Ile Ser Tyr Asp Gly Xaa Xaa Lys
1               5

<210> 140
<211> 12
<212> PRT
<213> homo sapiens

<220>
<221> MISC_FEATURE
<222> (9)..(9)
<223> wherein Xaa=Ala or Tyr, preferably Ala

<400> 140

Ala Arg Gly Leu Gly Val Trp Gly Xaa Phe Asp Tyr
1               5                   10

<210> 141
<211> 8
<212> PRT
<213> homo sapiens

<220>
<221> MISC_FEATURE
<222> (5)..(5)
<223> wherein Xaa=Ser, Asn or Thr, preferably Ser

<220>
<221> MISC_FEATURE
<222> (6)..(6)
<223> wherein Xaa=Asn, Asp or Ser, preferably Asn

<220>
<221> MISC_FEATURE
<222> (8)..(8)
<223> wherein Xaa=Gly or Ala, preferably Gly

<400> 141

Gly Phe Thr Phe Xaa Xaa Tyr Xaa
1               5

<210> 142
<211> 8
<212> PRT
<213> homo sapiens

<220>
<221> MISC_FEATURE
<222> (4)..(4)
<223> wherein Xaa=Ser or Thr, preferably Ser

<220>
<221> MISC_FEATURE
<222> (5)..(5)
<223> wherein Xaa=Ala or Gly, preferably Ala

<220>
<221> MISC_FEATURE
<222> (6)..(6)
<223> wherein Xaa=Tyr or Gly, preferably Tyr

<220>
<221> MISC_FEATURE
<222> (7)..(7)
<223> wherein Xaa=Ser or Ala, preferably Ser

<400> 142

Ile Ser Gly Xaa Xaa Xaa Xaa Thr
1               5

<210> 143
<211> 17
<212> PRT
<213> homo sapiens

<220>
<221> MISC_FEATURE
<222> (3)..(3)

<223> wherein Xaa=Ala or Gly, preferably Ala

<220>
<221> MISC_FEATURE
<222> (4)..(4)
<223> wherein Xaa=His or Tyr, preferably His

<220>
<221> MISC_FEATURE
<222> (5)..(5)
<223> wherein Xaa=Tyr or Thr, preferably Tyr

<220>
<221> MISC_FEATURE
<222> (6)..(6)
<223> wherein Xaa=His, Phe or Leu, preferably His

<220>
<221> MISC_FEATURE
<222> (14)..(14)
<223> wherein Xaa=Leu or Ser, preferably Leu

<400> 143

```
Ala Lys Xaa Xaa Xaa Xaa Gly Ser Gly Ser Tyr Tyr Thr Xaa Phe Asp
1               5                   10                  15


Tyr
```

<210> 144
<211> 8
<212> PRT
<213> homo sapiens

<220>
<221> MISC_FEATURE
<222> (5)..(5)
<223> wherein Xaa=Ser or Thr, preferably Ser

<220>
<221> MISC_FEATURE
<222> (6)..(6)
<223> wherein Xaa=Ile or Ser, preferably Ile

<400> 144

```
Gly Tyr Ser Phe Xaa Xaa Tyr Trp
1               5
```

<210> 145
<211> 8
<212> PRT
<213> homo sapiens

<220>
<221> MISC_FEATURE
<222> (2)..(2)

<223> wherein Xaa=Phe or Tyr, preferably Phe

<220>
<221> MISC_FEATURE
<222> (8)..(8)
<223> wherein Xaa=Ile or Thr, preferably Ile

<400> 145

Ile Xaa Pro Gly Asp Ser Asp Xaa
1 5

<210> 146
<211> 16
<212> PRT
<213> homo sapiens

<400> 146

Ala Arg Gln Pro Gly Asp Trp Ser Pro Arg His Trp Tyr Phe Asp Leu
1 5 10 15

<210> 147
<211> 8
<212> PRT
<213> homo sapiens

<220>
<221> MISC_FEATURE
<222> (3)..(3)
<223> wherein Xaa=Asn or Ser, preferably Asn

<400> 147

Gly Tyr Xaa Phe Thr Ser Tyr Trp
1 5

<210> 148
<211> 8
<212> PRT
<213> homo sapiens

<220>
<221> MISC_FEATURE
<222> (8)..(8)
<223> wherein Xaa=Ser or Thr, preferably Ser

<400> 148

Ile Ser Ala Tyr Asn Gly Asn Xaa
1 5

<210> 149
<211> 18
<212> PRT
<213> homo sapiens

<400> 149

Ala Arg Glu Tyr Ser Tyr Asp Ser Gly Thr Tyr Phe Tyr Tyr Gly Met
1 5 10 15

Asp Val

<210> 150
<211> 8
<212> PRT
<213> homo sapiens

<220>
<221> MISC_FEATURE
<222> (6)..(6)
<223> wherein Xaa=Asp or Ser, preferably Asp

<220>
<221> MISC_FEATURE
<222> (7)..(7)
<223> wherein Xaa=Tyr or His, preferably Tyr

<220>
<221> MISC_FEATURE
<222> (8)..(8)
<223> wherein Xaa=Val or Ala, preferably Val

<400> 150

Gly Phe Thr Phe Ser Xaa Xaa Xaa
1 5

<210> 151
<211> 8
<212> PRT
<213> homo sapiens

<220>
<221> MISC_FEATURE
<222> (7)..(7)
<223> wherein Xaa=Asn or Tyr, preferably Asn

<220>
<221> MISC_FEATURE
<222> (8)..(8)
<223> wherein Xaa=Lys or Glu, preferably Lys

<400> 151

Ile Ser Tyr Asp Gly Ser Xaa Xaa
1 5

<210> 152
<211> 14
<212> PRT
<213> homo sapiens

<220>
<221> MISC_FEATURE
<222> (4)..(4)
<223> wherein Xaa=Gly, Asp or Ser, preferably Gly

<220>
<221> MISC_FEATURE
<222> (5)..(5)
<223> wherein Xaa=Ile or Tyr, preferably Ile

<220>
<221> MISC_FEATURE
<222> (6)..(6)
<223> wherein Xaa=Thr or Ile, preferably Thr

<220>
<221> MISC_FEATURE
<222> (7)..(7)
<223> wherein Xaa=Gly or Ser, preferably Gly

<220>
<221> MISC_FEATURE
<222> (8)..(8)
<223> wherein Xaa=Thr or Ser, preferably Thr

<220>
<221> MISC_FEATURE
<222> (9)..(9)
<223> wherein Xaa=Thr or Ser, preferably Thr

<220>
<221> MISC_FEATURE
<222> (11)..(11)
<223> wherein Xaa=Tyr or Val, preferably Val

<400> 152

Ala Arg Gly Xaa Xaa Xaa Xaa Xaa Xaa Gly Xaa Phe Asp Tyr
1               5                   10

<210> 153
<211> 9
<212> PRT
<213> homo sapiens

<220>
<221> MISC_FEATURE
<222> (8)..(8)
<223> wherein Xaa=Ile or Leu

<400> 153

Gln Gln Ala Asn Ser Phe Pro Xaa Thr
1               5

<210> 154
<211> 9
<212> PRT

<213> homo sapiens

<220>
<221> MISC_FEATURE
<222> (5)..(5)
<223> wherein Xaa=Asn or His, preferably Asn

<400> 154

Gln Gln Arg Ser Xaa Trp Pro Arg Thr
1 5

<210> 155
<211> 6
<212> PRT
<213> homo sapiens

<220>
<221> MISC_FEATURE
<222> (5)..(5)
<223> wherein Xaa=Arg or Ser, preferably Arg

<400> 155

Gln Gly Ile Ser Xaa Trp
1 5

<210> 156
<211> 9
<212> PRT
<213> homo sapiens

<400> 156

Gln Gln Tyr Asn Ser Phe Pro Pro Thr
1 5

<210> 157
<211> 6
<212> PRT
<213> homo sapiens

<220>
<221> MISC_FEATURE
<222> (4)..(4)
<223> wherein Xaa=Ser or Asn, preferably Ser

<400> 157

Gln Gly Ile Xaa Ser Trp
1 5

<210> 158
<211> 9
<212> PRT
<213> homo sapiens

<220>
<221> MISC_FEATURE
<222> (8)..(8)
<223> wherein Xaa=Tyr or Leu, preferably Tyr

<400> 158

Gln Gln Tyr Asn Ser Tyr Pro Xaa Thr
1 5

<210> 159
<211> 6
<212> PRT
<213> homo sapiens

<220>
<221> MISC_FEATURE
<222> (4)..(4)
<223> wherein Xaa=Ser or Asn, preferably Ser

<220>
<221> MISC_FEATURE
<222> (5)..(5)
<223> wherein Xaa=Ser or Asn, preferably Ser

<400> 159

Gln Gly Ile Xaa Xaa Trp
1 5

<210> 160
<211> 9
<212> PRT
<213> homo sapiens

<220>
<221> MISC_FEATURE
<222> (4)..(4)
<223> wherein Xaa=Lys or Asn, preferably Lys

<400> 160

Gln Gln Tyr Xaa Ser Tyr Pro Ile Thr
1 5

<210> 161
<211> 7
<212> PRT
<213> homo sapiens

<400> 161

Ile Tyr His Ser Gly Asp Thr
1 5

<210> 162
<211> 9
<212> PRT
<213> homo sapiens

<220>
<221> MISC_FEATURE
<222> (6)..(6)
<223> wherein Xaa=Tyr or Phe, preferably Tyr

<400> 162

```
Gln Gln Tyr Asn Ser Xaa Pro Ile Thr
1               5
```

<210> 163
<211> 122
<212> PRT
<213> homo sapiens

<400> 163

```
Glu Val Gln Leu Val Gln Ser Gly Ala Glu Val Lys Lys Pro Gly Glu
1               5                   10                  15

Ser Leu Lys Ile Ser Cys Lys Ala Ser Gly Tyr Ser Phe His Phe Tyr
            20                  25                  30

Trp Ile Gly Trp Val Arg Gln Met Pro Gly Lys Gly Leu Glu Trp Met
        35                  40                  45

Gly Ser Ile Tyr Pro Gly Asp Ser Asp Thr Arg Tyr Arg Pro Ser Phe
    50                  55                  60

Gln Gly Gln Val Thr Ile Ser Ala Asp Lys Ser Ile Ser Thr Ala Tyr
65                  70                  75                  80

Leu Gln Trp Thr Ser Leu Lys Ala Ser Asp Thr Ala Ile Tyr Tyr Cys
                85                  90                  95

Ala Arg Gln Arg Gly Asp Tyr Tyr Tyr Phe Tyr Gly Met Asp Val Trp
            100                 105                 110

Gly Gln Gly Thr Thr Val Thr Val Ser Ser
        115                 120
```

<210> 164
<211> 8
<212> PRT
<213> homo sapiens

<400> 164

Gly Tyr Ser Phe His Phe Tyr Trp
1               5

<210>  165
<211>  8
<212>  PRT
<213>  homo sapiens

<400>  165

Ile Tyr Pro Gly Asp Ser Asp Thr
1               5

<210>  166
<211>  15
<212>  PRT
<213>  homo sapiens

<400>  166

Ala Arg Gln Arg Gly Asp Tyr Tyr Tyr Phe Tyr Gly Met Asp Val
1               5                   10                  15

<210>  167
<211>  107
<212>  PRT
<213>  homo sapiens

<400>  167

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Val Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Leu
                85                  90                  95

Thr Phe Gly Gly Gly Thr Lys Val Glu Ile Lys
            100                 105

<210>  168
<211>  7

<212> PRT
<213> homo sapiens

<400> 168

Gln Ser Val Ser Ser Ser Tyr
1               5

<210> 169
<211> 8
<212> PRT
<213> homo sapiens

<400> 169

Gln Gln Tyr Gly Ser Ser Leu Thr
1               5

<210> 170
<211> 127
<212> PRT
<213> homo sapiens

<400> 170

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Leu Ile Trp Val Arg Gln Ala Pro Gly Lys Gly Leu Glu Trp Val
        35                  40                  45

Ser Ile Ile Arg Gly Gly Ala Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala Arg Ile Trp Gly Pro Leu Phe Asp Tyr Trp Gly Gln Gly
            100                 105                 110

Thr Leu Val Thr Val Ser Ser Gly Phe Thr Phe Ser Asn Tyr Ala
        115                 120                 125

<210> 171
<211> 8
<212> PRT
<213> homo sapiens

<400> 171

Gly Phe Thr Phe Ser Asn Tyr Ala
1 5

<210> 172
<211> 8
<212> PRT
<213> homo sapiens

<400> 172

Ile Arg Gly Gly Ala Gly Ser Thr
1 5

<210> 173
<211> 12
<212> PRT
<213> homo sapiens

<400> 173

Ala Lys Ala Arg Ile Trp Gly Pro Leu Phe Asp Tyr
1 5 10

<210> 174
<211> 108
<212> PRT
<213> homo sapiens

<400> 174

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Tyr Asp Ala Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Arg Ser Asn Trp Pro Pro
85 90 95

Leu Thr Phe Gly Gly Gly Thr Lys Val Glu Ile Lys
100 105

<210> 175
<211> 6
<212> PRT
<213> homo sapiens

<400> 175

Gln Ser Val Ser Ser Tyr
1 5

<210> 176
<211> 10
<212> PRT
<213> homo sapiens

<400> 176

Gln Gln Arg Ser Asn Trp Pro Pro Leu Thr
1 5 10

<210> 177
<211> 122
<212> PRT
<213> homo sapiens

<400> 177

Gln Val Gln Leu Val Gln Ser Gly Ala Glu Val Lys Lys Pro Gly Ala
1 5 10 15

Ser Val Arg Val Pro Cys Lys Ala Ser Gly Tyr Thr Phe Thr Arg Tyr
20 25 30

Gly Ile Ser Trp Val Arg Gln Ala Pro Gly Gln Gly Leu Glu Trp Met
35 40 45

Gly Trp Ile Ser Ala Tyr Asn Gly Lys Thr Tyr Tyr Ala Gln Lys Leu
50 55 60

Gln Gly Arg Val Thr Met Thr Thr Asp Thr Ser Thr Ser Thr Ala Tyr
65 70 75 80

Met Glu Leu Arg Ser Leu Arg Ser Asp Asp Thr Ala Val Tyr Tyr Cys
85 90 95

Ala Arg Ser Pro Leu Leu Trp Phe Glu Glu Leu Tyr Phe Asp Tyr Trp
100 105 110

Gly Gln Gly Thr Leu Val Thr Val Ser Ser
115 120

<210> 178

<211> 8
<212> PRT
<213> homo sapiens

<400> 178

Gly Tyr Thr Phe Thr Arg Tyr Gly
1 5

<210> 179
<211> 8
<212> PRT
<213> homo sapiens

<400> 179

Ile Ser Ala Tyr Asn Gly Lys Thr
1 5

<210> 180
<211> 15
<212> PRT
<213> homo sapiens

<400> 180

Ala Arg Ser Pro Leu Leu Trp Phe Glu Glu Leu Tyr Phe Asp Tyr
1 5 10 15

<210> 181
<211> 108
<212> PRT
<213> homo sapiens

<400> 181

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Thr
20 25 30

Tyr Leu Ala Trp Tyr Gln Gln Lys Pro Gly Gln Ala Pro Arg Leu Leu
35 40 45

Ile Tyr Gly Ala Ser Ser Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Tyr Gly Thr Ser Leu
85 90 95

Phe Thr Phe Gly Pro Gly Thr Lys Val Asp Ile Lys
100 105

<210> 182
<211> 7
<212> PRT
<213> homo sapiens

<400> 182

Gln Ser Val Ser Ser Thr Tyr
1 5

<210> 183
<211> 9
<212> PRT
<213> homo sapiens

<400> 183

Gln Gln Tyr Gly Thr Ser Leu Phe Thr
1 5

<210> 184
<211> 122
<212> PRT
<213> homo sapiens

<400> 184

Glu Val Gln Leu Val Gln Ser Gly Ala Glu Val Lys Lys Pro Gly Glu
1 5 10 15

Ser Leu Lys Ile Ser Cys Lys Gly Ser Gly Tyr Arg Phe Thr Ser Tyr
20 25 30

Trp Ile Gly Trp Val Arg Gln Met Pro Gly Lys Gly Leu Glu Trp Met
35 40 45

Gly Ser Ile Tyr Pro Gly Asp Ser Tyr Thr Arg Asn Ser Pro Ser Phe
50 55 60

Gln Gly Gln Val Thr Ile Ser Ala Asp Lys Ser Ile Ala Thr Ala Tyr
65 70 75 80

Leu Gln Trp Asn Ser Leu Lys Ala Ser Asp Thr Ala Met Tyr Tyr Cys
85 90 95

Ala Arg His Ala Gly Asp Phe Tyr Tyr Phe Asp Gly Leu Asp Val Trp
100 105 110

Gly Gln Gly Thr Thr Val Thr Val Ser Ser
115 120

<210> 185
<211> 9
<212> PRT
<213> homo sapiens

<400> 185

Gly Tyr Arg Phe Thr Thr Ser Tyr Trp
1 5

<210> 186
<211> 8
<212> PRT
<213> homo sapiens

<400> 186

Ile Tyr Pro Gly Asp Ser Tyr Thr
1 5

<210> 187
<211> 15
<212> PRT
<213> homo sapiens

<400> 187

Ala Arg His Ala Gly Asp Phe Tyr Tyr Phe Asp Gly Leu Asp Val
1 5 10 15

<210> 188
<211> 109
<212> PRT
<213> homo sapiens

<400> 188

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

Ile Tyr Gly Ala Ser Ser Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Tyr Gly Ser Ser Pro

85 90 95

Pro Ile Thr Phe Gly Gln Gly Thr Arg Leu Glu Ile Lys
100 105

<210> 189
<211> 7
<212> PRT
<213> homo sapiens

<400> 189

Gln Ser Val Ser Ser Ser Tyr
1 5

<210> 190
<211> 10
<212> PRT
<213> homo sapiens

<400> 190

Gln Gln Tyr Gly Ser Ser Pro Pro Ile Thr
1 5 10

<210> 191
<211> 132
<212> PRT
<213> homo sapiens

<400> 191

Glu Val Gln Leu Val Gln Ser Gly Ala Glu Val Lys Lys Pro Gly Glu
1 5 10 15

Ser Leu Lys Ile Ser Cys Lys Gly Ser Gly Tyr Ser Phe Thr Arg Tyr
20 25 30

Trp Ile Gly Trp Val Arg Gln Met Pro Gly Lys Gly Leu Glu Trp Met
35 40 45

Gly Ile Ile Tyr Pro Gly Asp Ser Asp Thr Arg Tyr Ser Pro Ser Phe
50 55 60

Gln Gly Gln Val Thr Ile Ser Ala Asp Lys Ser Ile Ser Thr Ala Tyr
65 70 75 80

Leu Gln Trp Ser Ser Leu Lys Ala Ser Asp Thr Ala Met Tyr Tyr Cys
85 90 95

Ala Arg Leu Thr Gly Asp Arg Gly Phe Asp Tyr Tyr Ser Gly Met Asp
100 105 110

Val Trp Gly Gln Gly Thr Thr Val Thr Val Ser Ser Gly Tyr Ser Phe
115 120 125

Thr Arg Tyr Trp
130

<210> 192
<211> 8
<212> PRT
<213> homo sapiens

<400> 192

Gly Tyr Ser Phe Thr Arg Tyr Trp
1 5

<210> 193
<211> 8
<212> PRT
<213> homo sapiens

<400> 193

Ile Tyr Pro Gly Asp Ser Asp Thr
1 5

<210> 194
<211> 17
<212> PRT
<213> homo sapiens

<400> 194

Ala Arg Leu Thr Gly Asp Arg Gly Phe Asp Tyr Tyr Ser Gly Met Asp
1 5 10 15

Val

<210> 195
<211> 107
<212> PRT
<213> homo sapiens

<400> 195

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

```
Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Phe
                85                  90                  95

Thr Phe Gly Pro Gly Thr Lys Val Asp Ile Lys
            100                 105


<210>  196
<211>  7
<212>  PRT
<213>  homo sapiens

<400>  196

Gln Ser Val Ser Ser Ser Tyr
1               5


<210>  197
<211>  8
<212>  PRT
<213>  homo sapiens

<400>  197

Gln Gln Tyr Gly Ser Ser Phe Thr
1               5


<210>  198
<211>  119
<212>  PRT
<213>  homo sapiens

<400>  198

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Gly Ile Ser Trp Val Arg Gln Ala Pro Gly Pro Gly Leu Glu Trp Met
        35                  40                  45

Gly Arg Ile Ile Pro Ile Leu Gly Ile Ala Asn Tyr Ala Gln Lys Phe
    50                  55                  60
```

Gln Gly Arg Val Thr Ile Thr Ala Asp Lys Ser Thr Asn Thr Ala Tyr
65 70 75 80

Met Glu Leu Ser Ser Leu Arg Ser Glu Asp Thr Ala Val Tyr Tyr Cys
85 90 95

Ala Arg Asp Gln Glu Tyr Ser Ser Asn Trp Tyr Tyr Trp Gly Gln Gly
100 105 110

Thr Leu Val Thr Val Ser Ser
115

<210> 199
<211> 8
<212> PRT
<213> homo sapiens

<400> 199

Gly Gly Thr Phe Ser Ser Tyr Gly
1 5

<210> 200
<211> 8
<212> PRT
<213> homo sapiens

<400> 200

Ile Ile Pro Ile Leu Gly Ile Ala
1 5

<210> 201
<211> 12
<212> PRT
<213> homo sapiens

<400> 201

Ala Arg Asp Gln Glu Tyr Ser Ser Asn Trp Tyr Tyr
1 5 10

<210> 202
<211> 107
<212> PRT
<213> homo sapiens

<400> 202

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Arg Ser Ser
20 25 30

```
Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Leu Tyr Gly Ser Ser Pro
                85                  90                  95

Thr Phe Gly Pro Gly Thr Lys Val Asp Ile Lys
            100                 105
```

<210> 203
<211> 7
<212> PRT
<213> homo sapiens

<400> 203

```
Gln Ser Val Arg Ser Ser Tyr
1               5
```

<210> 204
<211> 8
<212> PRT
<213> homo sapiens

<400> 204

```
Gln Leu Tyr Gly Ser Ser Pro Thr
1               5
```

<210> 205
<211> 122
<212> PRT
<213> homo sapiens

<400> 205

```
Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Gly Trp Val Arg Gln Met Pro Gly Lys Gly Leu Glu Trp Met
        35                  40                  45

Gly Ser Ile Tyr Pro Gly Asp Ser His Thr Arg Tyr Arg Pro Ser Phe
```

```
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gln Lys Gly Asp Phe Tyr Tyr Phe Phe Gly Leu Asp Val Trp
            100                 105                 110

Gly Gln Gly Thr Ala Ile Thr Val Ser Ser
        115                 120


<210>  206
<211>  107
<212>  PRT
<213>  homo sapiens

<400>  206

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Leu
                85                  90                  95

Thr Phe Gly Gly Gly Thr Lys Val Glu Ile Lys
            100                 105


<210>  207
<211>  122
<212>  PRT
<213>  homo sapiens

<400>  207

Glu Val Gln Leu Val Gln Ser Gly Ala Glu Val Lys Lys Pro Gly Glu
1               5                   10                  15
```

Ser Leu Lys Ile Ser Cys Lys Gly Ser Gly Tyr Ser Phe Thr Ser Tyr
20 25 30

Trp Ile Gly Trp Val Arg Gln Met Pro Gly Lys Gly Leu Glu Trp Met
35 40 45

Gly Ser Ile Tyr Pro Gly Asp Ser His Thr Arg Tyr Arg Pro Ser Phe
50 55 60

Gln Gly Gln Val Thr Ile Ser Ala Asp Lys Ser Ile Ser Thr Ala Tyr
65 70 75 80

Leu Gln Trp Ser Ser Leu Lys Ala Ser Asp Thr Ala Met Tyr Tyr Cys
85 90 95

Ala Arg Gln Ala Gly Asp Tyr Tyr Tyr Tyr Asn Gly Met Asp Val Trp
100 105 110

Gly Gln Gly Thr Thr Val Thr Val Ser Ser
115 120

<210> 208
<211> 107
<212> PRT
<213> homo sapiens

<400> 208

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Thr Trp Tyr Gln Gln Lys Pro Gly Gln Ala Pro Arg Leu Leu
35 40 45

Ile Tyr Gly Ala Ser Ser Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Tyr Gly Ser Ser Leu
85 90 95

Thr Phe Gly Gly Gly Thr Lys Val Glu Ile Lys
100 105

<210> 209
<211> 122
<212> PRT
<213> homo sapiens

<400> 209

```
Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Gly Trp Val Arg Gln Met Pro Gly Lys Gly Leu Glu Trp Met
        35                  40                  45

Gly Ile Ile Tyr Pro Gly Asp Ser Asp Thr Arg Tyr Ser Pro Ser Phe
    50                  55                  60

Gln Gly Gln Val Thr Ile Ser Val Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gln Lys Gly Asp Tyr Tyr Tyr His Tyr Gly Leu Asp Val Trp
            100                 105                 110

Gly Gln Gly Thr Thr Val Thr Val Ser Ser
        115                 120
```

<210> 210
<211> 109
<212> PRT
<213> homo sapiens

<400> 210

```
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60
```

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Tyr Gly Ser Ser Pro
85 90 95

Arg Leu Thr Phe Gly Gly Gly Thr Lys Val Glu Ile Lys
100 105

<210> 211
<211> 122
<212> PRT
<213> homo sapiens

<400> 211

Glu Val Gln Leu Val Gln Ser Gly Ala Glu Val Lys Lys Pro Gly Glu
1 5 10 15

Ser Leu Lys Ile Ser Cys Lys Gly Ser Gly Tyr Ser Phe Thr Ser Tyr
20 25 30

Trp Ile Gly Trp Val Arg Gln Met Pro Gly Lys Gly Leu Glu Trp Met
35 40 45

Gly Ile Ile Tyr Pro Gly Asp Ser Asp Thr Arg Tyr Ser Pro Ser Phe
50 55 60

Gln Gly Gln Val Thr Ile Ser Ala Asp Lys Ser Ile Ser Thr Ala Tyr
65 70 75 80

Leu Gln Trp Ser Ser Leu Lys Ala Ser Asp Thr Ala Met Tyr Tyr Cys
85 90 95

Ala Arg Gln Lys Gly Asp Tyr Tyr Tyr Phe Asn Gly Leu Asp Val Trp
100 105 110

Gly Gln Gly Thr Thr Val Thr Val Ser Ser
115 120

<210> 212
<211> 109
<212> PRT
<213> homo sapiens

<400> 212

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

Ile Tyr Gly Ala Ser Ser Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Tyr Gly Ser Ser Pro
85 90 95

Arg Leu Thr Phe Gly Gly Gly Thr Lys Val Glu Ile Lys
100 105

<210> 213
<211> 122
<212> PRT
<213> homo sapiens

<400> 213

Glu Val Gln Leu Val Gln Ser Gly Ala Glu Val Lys Lys Pro Gly Glu
1 5 10 15

Ser Leu Lys Ile Ser Cys Gln Gly Ser Gly Tyr Arg Phe Ile Ser Tyr
20 25 30

Trp Ile Gly Trp Val Arg Gln Met Pro Gly Lys Gly Leu Glu Trp Met
35 40 45

Gly Arg Ile Tyr Pro Gly Asp Ser Asp Thr Arg Tyr Ser Pro Ser Phe
50 55 60

Gln Gly Gln Val Thr Ile Ser Val Asp Lys Ser Ile Ser Thr Ala Tyr
65 70 75 80

Leu Gln Trp Ser Ser Leu Lys Ala Ser Asp Thr Ala Met Tyr Tyr Cys
85 90 95

Ala Arg Gln Arg Gly Asp Tyr Tyr Tyr Phe Asn Gly Leu Asp Val Trp
100 105 110

Gly Gln Gly Thr Thr Val Thr Val Ser Ser
115 120

<210> 214
<211> 107
<212> PRT
<213> homo sapiens

<400> 214

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

Ile Tyr Gly Ala Ser Ser Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Tyr Gly Ser Ser Leu
85 90 95

Thr Phe Gly Gly Gly Thr Lys Val Glu Ile Lys
100 105

<210> 215
<211> 122
<212> PRT
<213> homo sapiens

<400> 215

Glu Val Gln Leu Val Gln Ser Gly Ala Glu Val Lys Lys Pro Gly Glu
1 5 10 15

Ser Leu Lys Ile Ser Cys Lys Gly Ser Gly Tyr Ser Phe Thr Ser Tyr
20 25 30

Trp Ile Gly Trp Val Arg Gln Met Pro Gly Lys Gly Leu Glu Trp Met
35 40 45

Gly Arg Ile Tyr Pro Gly Asp Ser Asp Thr Arg Tyr Ser Pro Ser Phe
50 55 60

Gln Gly Gln Val Thr Ile Ser Ala Asp Lys Ser Ile Thr Thr Ala Tyr
65 70 75 80

Leu Gln Trp Ser Ser Leu Arg Ala Ser Asp Thr Ala Met Tyr Tyr Cys

85 90 95

Ala Arg Gln Arg Gly Asp Tyr Tyr Tyr Phe Phe Gly Leu Asp Ile Trp
100 105 110

Gly Gln Gly Thr Thr Val Thr Val Ser Leu
115 120

<210> 216
<211> 107
<212> PRT
<213> homo sapiens

<400> 216

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

Ile Tyr Gly Ala Ser Ser Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Tyr Gly Ser Ser Leu
85 90 95

Thr Phe Gly Gly Gly Thr Lys Val Glu Ile Lys
100 105

<210> 217
<211> 122
<212> PRT
<213> homo sapiens

<400> 217

Glu Val Gln Leu Val Gln Ser Gly Ala Glu Val Lys Lys Pro Gly Glu
1 5 10 15

Ser Leu Lys Ile Ser Cys Lys Gly Ser Gly Tyr Arg Phe Ser Ser Tyr
20 25 30

Trp Ile Gly Trp Val Arg Gln Met Pro Gly Lys Gly Leu Glu Trp Met
35 40 45

Gly Ser Ile Phe Pro Gly Asp Ser Asp Thr Arg Tyr Ser Pro Ser Phe
    50                  55                  60

Gln Gly Gln Val Thr Ile Ser Ala Asp Lys Ser Ile Thr Thr Ala Tyr
65                  70                  75                  80

Leu Gln Trp Ser Ser Leu Lys Ala Ser Asp Thr Ala Met Tyr Tyr Cys
                85                  90                  95

Ala Arg Gln Ala Gly Asp Tyr Tyr Tyr Tyr Asn Gly Met Asp Val Trp
            100                 105                 110

Gly Gln Gly Thr Thr Val Thr Val Ser Ser
        115                 120

<210> 218
<211> 107
<212> PRT
<213> homo sapiens

<400> 218

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Leu
                85                  90                  95

Thr Phe Gly Gly Gly Thr Lys Val Glu Ile Lys
            100                 105

<210> 219
<211> 8
<212> PRT
<213> homo sapiens

<220>
<221> MISC_FEATURE
<222> (3)..(3)
<223> wherein Xaa=Ser or Arg

<220>
<221> MISC_FEATURE
<222> (5)..(5)
<223> wherein Xaa=Ser, Thr, His or Ile, preferably His or Thr

<220>
<221> MISC_FEATURE
<222> (6)..(6)
<223> wherein Xaa=Ser, Arg or Phe

<400> 219

Gly Tyr Xaa Phe Xaa Xaa Tyr Trp
1               5

<210> 220
<211> 8
<212> PRT
<213> homo sapiens

<220>
<221> MISC_FEATURE
<222> (2)..(2)
<223> wherein Xaa=Tyr or Phe

<220>
<221> MISC_FEATURE
<222> (7)..(7)
<223> wherein Xaa=Asp, Tyr or His, preferably Asp or Tyr

<400> 220

Ile Xaa Pro Gly Asp Ser Xaa Thr
1               5

<210> 221
<211> 17
<212> PRT
<213> homo sapiens

<220>
<221> MISC_FEATURE
<222> (3)..(3)
<223> wherein Xaa=Gln, His or leu, preferably Gln

<220>
<221> MISC_FEATURE
<222> (4)..(4)
<223> wherein Xaa=Arg, Ala, Thr or Lys, preferably Arg or Ala

<220>
<221> MISC_FEATURE
<222> (5)..(5)
<223> wherein Xaa=Gly

<220>
<221> MISC_FEATURE
<222> (6)..(6)
<223> wherein Xaa=Asp

<220>
<221> MISC_FEATURE
<222> (7)..(7)
<223> wherein Xaa=Arg or none, preferably none

<220>
<221> MISC_FEATURE
<222> (8)..(8)
<223> wherein Xaa=Gly or none, preferably none

<220>
<221> MISC_FEATURE
<222> (9)..(9)
<223> wherein Xaa=Tyr or Phe

<220>
<221> MISC_FEATURE
<222> (10)..(10)
<223> wherein Xaa=Tyr or Asp, preferably Tyr

<220>
<221> MISC_FEATURE
<222> (12)..(12)
<223> wherein Xaa=Tyr, Phe or His, preferably Phe

<220>
<221> MISC_FEATURE
<222> (13)..(13)
<223> wherein Xaa=Tyr, Asp, Ser, Phe or Asn, preferably Tyr

<220>
<221> MISC_FEATURE
<222> (15)..(15)
<223> wherein Xaa=Met or Leu

<220>
<221> MISC_FEATURE
<222> (17)..(17)
<223> wherein Xaa=Val or Ile, preferably Val

<400> 221

```
Ala Arg Xaa Xaa Xaa Xaa Xaa Xaa Xaa Xaa Tyr Xaa Xaa Gly Xaa Asp
1               5                   10                  15


Xaa
```

<210> 222
<211> 8
<212> PRT
<213> homo sapiens

<220>

<221> MISC_FEATURE
<222> (6)..(6)
<223> wherein Xaa=Asn or Ser, preferably Asn

<400> 222

Gly Phe Thr Phe Ser Xaa Tyr Ala
1               5

<210> 223
<211> 8
<212> PRT
<213> homo sapiens

<220>
<221> MISC_FEATURE
<222> (2)..(2)
<223> wherein Xaa=Arg or Ser, preferably Arg

<220>
<221> MISC_FEATURE
<222> (4)..(4)
<223> wherein Xaa=Gly or Ser, preferably Gly

<220>
<221> MISC_FEATURE
<222> (5)..(5)
<223> wherein Xaa=Ala or Gly, preferably Ala

<400> 223

Ile Xaa Gly Xaa Xaa Gly Ser Thr
1               5

<210> 224
<211> 11
<212> PRT
<213> homo sapiens

<220>
<221> MISC_FEATURE
<222> (8)..(8)
<223> wherein Xaa=Leu or Tyr, preferably Leu

<400> 224

Ala Lys Arg Ile Trp Gly Pro Xaa Phe Asp Tyr
1               5                   10

<210> 225
<211> 8
<212> PRT
<213> homo sapiens

<220>
<221> MISC_FEATURE
<222> (6)..(6)

<223> wherein Xaa=Arg or Ser, preferably Arg

<400> 225

Gly Tyr Thr Phe Thr Xaa Tyr Gly
1 5

<210> 226
<211> 8
<212> PRT
<213> homo sapiens

<220>
<221> MISC_FEATURE
<222> (7)..(7)
<223> wherein Xaa=Lys or Asn, preferably Lys

<400> 226

Ile Ser Ala Tyr Asn Gly Xaa Thr
1 5

<210> 227
<211> 15
<212> PRT
<213> homo sapiens

<400> 227

Ala Arg Ser Pro Leu Leu Trp Phe Glu Glu Leu Tyr Phe Asp Tyr
1 5 10 15

<210> 228
<211> 8
<212> PRT
<213> homo sapiens

<220>
<221> MISC_FEATURE
<222> (8)..(8)
<223> wherein Xaa=Gly or Ala, preferably Gly

<400> 228

Gly Gly Thr Phe Ser Ser Tyr Xaa
1 5

<210> 229
<211> 11
<212> PRT
<213> homo sapiens

<220>
<221> MISC_FEATURE
<222> (9)..(9)
<223> wherein Xaa=Asn or Tyr, preferably Asn

<220>
<221> MISC_FEATURE
<222> (10)..(10)
<223> wherein Xaa=Trp or Phe, preferably Trp

<220>
<221> MISC_FEATURE
<222> (11)..(11)
<223> wherein Xaa=Tyr or Asp, preferably Tyr

<400> 229

Ala Arg Asp Gln Glu Tyr Ser Ser Xaa Xaa Xaa
1 5 10

<210> 230
<211> 7
<212> PRT
<213> homo sapiens

<220>
<221> MISC_FEATURE
<222> (4)..(4)
<223> wherein Xaa=Ser or Arg

<220>
<221> MISC_FEATURE
<222> (6)..(6)
<223> wherein Xaa=Ser or Thr

<400> 230

Gln Ser Val Xaa Ser Xaa Tyr
1 5

<210> 231
<211> 10
<212> PRT
<213> homo sapiens

<220>
<221> MISC_FEATURE
<222> (2)..(2)
<223> wherein Xaa=Gln or Leu

<220>
<221> MISC_FEATURE
<222> (5)..(5)
<223> wherein Xaa=Ser or Thr

<220>
<221> MISC_FEATURE
<222> (7)..(7)
<223> wherein Xaa=Pro or none

<220>
<221> MISC_FEATURE
<222> (8)..(8)

<223> wherein Xaa=Pro, Leu, Arg or none, preferably Pro, Leu or none

<220>
<221> MISC_FEATURE
<222> (9)..(9)
<223> wherein Xaa=Leu, Phe, Ile or none

<400> 231

Gln Xaa Tyr Gly Xaa Ser Xaa Xaa Xaa Thr
1               5                   10

<210> 232
<211> 119
<212> PRT
<213> homo sapiens

<400> 232

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Phe
            20                  25                  30

Pro Met Ala Trp Val Arg Gln Ala Pro Gly Lys Gly Leu Glu Trp Val
        35                  40                  45

Ser Thr Ile Ser Thr Ser Gly Gly Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Phe Arg Gln Tyr Ser Gly Gly Phe Asp Tyr Trp Gly Gln Gly
            100                 105                 110

Thr Leu Val Thr Val Ser Ser
        115

<210> 233
<211> 110
<212> PRT
<213> homo sapiens

<400> 233

Asp Ile Gln Leu Thr Gln Pro Asn Ser Val Ser Thr Ser Leu Gly Ser
1               5                   10                  15

Thr Val Lys Leu Ser Cys Thr Leu Ser Ser Gly Asn Ile Glu Asn Asn
20 25 30

Tyr Val His Trp Tyr Gln Leu Tyr Glu Gly Arg Ser Pro Thr Thr Met
35 40 45

Ile Tyr Asp Asp Asp Lys Arg Pro Asp Gly Val Pro Asp Arg Phe Ser
50 55 60

Gly Ser Ile Asp Arg Ser Ser Asn Ser Ala Phe Leu Thr Ile His Asn
65 70 75 80

Val Ala Ile Glu Asp Glu Ala Ile Tyr Phe Cys His Ser Tyr Val Ser
85 90 95

Ser Phe Asn Val Phe Gly Gly Gly Thr Lys Leu Thr Val Leu
100 105 110

<210> 234
<211> 120
<212> PRT
<213> homo sapiens

<400> 234

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Ile Ser Tyr
20 25 30

Thr Met His Trp Val Arg Gln Ala Pro Gly Gln Gly Leu Glu Trp Met
35 40 45

Gly Tyr Ile Asn Pro Arg Ser Gly Tyr Thr His Tyr Asn Gln Lys Leu
50 55 60

Lys Asp Lys Ala Thr Leu Thr Ala Asp Lys Ser Ala Ser Thr Ala Tyr
65 70 75 80

Met Glu Leu Ser Ser Leu Arg Ser Glu Asp Thr Ala Val Tyr Tyr Cys
85 90 95

Ala Arg Ser Ala Tyr Tyr Asp Tyr Asp Gly Phe Ala Tyr Trp Gly Gln
100 105 110

Gly Thr Leu Val Thr Val Ser Ser
115 120

<210> 235
<211> 106
<212> PRT
<213> homo sapiens

<400> 235

```
Asp Ile Gln Met Thr Gln Ser Pro Ser Ser Leu Ser Ala Ser Val Gly
1               5                   10                  15

Asp Arg Val Thr Ile Thr Cys Ser Ala Ser Ser Ser Val Ser Tyr Met
            20                  25                  30

Asn Trp Tyr Gln Gln Lys Pro Gly Lys Ala Pro Lys Arg Leu Ile Tyr
        35                  40                  45

Asp Thr Ser Lys Leu Ala Ser Gly Val Pro Ser Arg Phe Ser Gly Ser
    50                  55                  60

Gly Ser Gly Thr Asp Phe Thr Leu Thr Ile Ser Ser Leu Gln Pro Glu
65                  70                  75                  80

Asp Phe Ala Thr Tyr Tyr Cys Gln Gln Trp Ser Ser Asn Pro Pro Thr
                85                  90                  95

Phe Gly Gly Gly Thr Lys Val Glu Ile Lys
            100                 105
```

<210> 236
<211> 119
<212> PRT
<213> homo sapiens

<400> 236

```
Gln Val Gln Leu Val Gln Ser Gly Gly Gly Val Val Gln Pro Gly Arg
1               5                   10                  15

Ser Leu Arg Leu Ser Cys Lys Ala Ser Gly Tyr Thr Phe Thr Arg Tyr
            20                  25                  30

Thr Met His Trp Val Arg Gln Ala Pro Gly Lys Gly Leu Glu Trp Ile
        35                  40                  45

Gly Tyr Ile Asn Pro Ser Arg Gly Tyr Thr Asn Tyr Asn Gln Lys Val
    50                  55                  60

Lys Asp Arg Phe Thr Ile Ser Arg Asp Asn Ser Lys Asn Thr Ala Phe
65                  70                  75                  80

Leu Gln Met Asp Ser Leu Arg Pro Glu Asp Thr Gly Val Tyr Phe Cys
```

85 90 95

Ala Arg Tyr Tyr Asp Asp His Tyr Ser Leu Asp Tyr Trp Gly Gln Gly
100 105 110

Thr Pro Val Thr Val Ser Ser
115

<210> 237
<211> 106
<212> PRT
<213> homo sapiens

<400> 237

Asp Ile Gln Met Thr Gln Ser Pro Ser Ser Leu Ser Ala Ser Val Gly
1 5 10 15

Asp Arg Val Thr Ile Thr Cys Ser Ala Ser Ser Ser Val Ser Tyr Met
20 25 30

Asn Trp Tyr Gln Gln Thr Pro Gly Lys Ala Pro Lys Arg Trp Ile Tyr
35 40 45

Asp Thr Ser Lys Leu Ala Ser Gly Val Pro Ser Arg Phe Ser Gly Ser
50 55 60

Gly Ser Gly Thr Asp Tyr Thr Phe Thr Ile Ser Ser Leu Gln Pro Glu
65 70 75 80

Asp Ile Ala Thr Tyr Tyr Cys Gln Gln Trp Ser Ser Asn Pro Phe Thr
85 90 95

Phe Gly Gln Gly Thr Lys Leu Gln Ile Thr
100 105

<210> 238
<211> 119
<212> PRT
<213> homo sapiens

<400> 238

Glu Val Gln Leu Val Glu Ser Gly Gly Gly Leu Val Lys Pro Gly Gly
1 5 10 15

Ser Leu Arg Leu Ser Cys Ala Ala Ser Gly Phe Thr Phe Ser Ser Tyr
20 25 30

Gly Met Phe Trp Val Arg Gln Ala Pro Gly Lys Gly Leu Glu Trp Val
35 40 45

```
Ala Thr Ile Ser Arg Tyr Ser Arg Tyr Ile Tyr Tyr Pro Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Arg Pro Leu Tyr Gly Ser Ser Pro Asp Tyr Trp Gly Gln Gly
            100                 105                 110

Thr Leu Val Thr Val Ser Ser
        115
```

<210> 239
<211> 106
<212> PRT
<213> homo sapiens

<400> 239

```
Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Thr Tyr Val
            20                  25                  30

His Trp Tyr Gln Gln Lys Pro Gly Gln Ala Pro Arg Leu Leu Ile Tyr
        35                  40                  45

Asp Thr Ser Lys Leu Ala Ser Gly Ile Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Phe Ala Val Tyr Tyr Cys Phe Gln Gly Ser Gly Tyr Pro Leu Thr
                85                  90                  95

Phe Gly Ser Gly Thr Lys Leu Glu Met Arg
            100                 105
```

<210> 240
<211> 8
<212> PRT
<213> homo sapiens

<400> 240

Gly Phe Thr Phe Ser Ser Tyr Gly
1               5

<210>  241
<211>  8
<212>  PRT
<213>  homo sapiens

<400>  241

Ile Ser Arg Tyr Ser Arg Tyr Ile
1               5

<210>  242
<211>  12
<212>  PRT
<213>  homo sapiens

<400>  242

Ala Arg Arg Pro Leu Tyr Gly Ser Ser Pro Asp Tyr
1               5                   10

<210>  243
<211>  5
<212>  PRT
<213>  homo sapiens

<400>  243

Ser Ser Val Thr Tyr
1               5

<210>  244
<211>  9
<212>  PRT
<213>  homo sapiens

<400>  244

Phe Gln Gly Ser Gly Tyr Pro Leu Thr
1               5

<210>  245
<211>  330
<212>  PRT
<213>  homo sapiens

<400>  245

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

```
Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285
```

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 246
<211> 330
<212> PRT
<213> homo sapiens

<400> 246

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Arg Val Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Leu
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 247
<211> 330
<212> PRT
<213> homo sapiens

<400> 247

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Arg Val Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Arg Leu Thr Val Asp Lys Ser Arg Trp Gln Gln Gly Asn
290 295 300

Val Phe Ser Cys Ser Val Met His Glu Ala Leu His Asn His Tyr Thr

305 310 315 320

Gln Lys Ser Leu Ser Leu Ser Pro Gly Lys
325 330

<210> 248
<211> 330
<212> PRT
<213> homo sapiens

<400> 248

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Arg Val Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Gln Ser Thr Tyr Arg Val Val Ser Val Leu Thr Val Leu
180 185 190

His Gln Asp Trp Leu Asn Gly Lys Glu Tyr Lys Cys Lys Val Ser Asn

195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 249
<211> 330
<212> PRT
<213> homo sapiens

<400> 249

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys

85 90 95

Arg Val Glu Pro Lys Ser Cys Asp Lys Thr His Thr Cys Pro Pro Cys
100 105 110

Pro Ala Pro Glu Phe Glu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Ala Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Gln Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Ser Ile Glu Lys Thr Ile Ser Lys Ala Lys Gly
210 215 220

Gln Pro Arg Glu Pro Gln Val Tyr Thr Leu Pro Pro Ser Arg Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 250
<211> 330
<212> PRT
<213> homo sapiens

<400> 250

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Gln Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220
```

Gln Pro Arg Glu Pro Gln Val Tyr Thr Leu Pro Pro Ser Arg Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Leu
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 251
<211> 330
<212> PRT
<213> homo sapiens

<400> 251

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Arg Val Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Gln Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Arg Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 252
<211> 330
<212> PRT
<213> homo sapiens

<400> 252

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Phe Glu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Ala Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Gln Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Ser Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255
```

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Leu
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210> 253
<211> 330
<212> PRT
<213> homo sapiens

<400> 253

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Phe Glu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Ala Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Gln Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Ser Ile Glu Lys Thr Ile Ser Lys Ala Lys Gly
210 215 220

Gln Pro Arg Glu Pro Gln Val Tyr Thr Leu Pro Pro Ser Arg Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Arg Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 254
<211> 127
<212> PRT
<213> homo sapiens

<400> 254

Gln Val Gln Leu Val Gln Ser Gly Ala Glu Val Lys Lys Pro Gly Ala
1 5 10 15

Ser Val Lys Val Ser Cys Gln Ala Ser Gly Tyr Arg Phe Ser Asn Phe
20 25 30

```
Val Ile His Trp Val Arg Gln Ala Pro Gly Gln Arg Phe Glu Trp Met
        35                  40                  45

Gly Trp Ile Asn Pro Tyr Asn Gly Asn Lys Glu Phe Ser Ala Lys Phe
    50                  55                  60

Gln Asp Arg Val Thr Phe Thr Ala Asp Thr Ser Ala Asn Thr Ala Tyr
65                  70                  75                  80

Met Glu Leu Arg Ser Leu Arg Ser Ala Asp Thr Ala Val Tyr Tyr Cys
                85                  90                  95

Ala Arg Val Gly Pro Tyr Ser Trp Asp Asp Ser Pro Gln Asp Asn Tyr
            100                 105                 110

Tyr Met Asp Val Trp Gly Lys Gly Thr Thr Val Ile Val Ser Ser
        115                 120                 125


<210>  255
<211>  108
<212>  PRT
<213>  homo sapiens

<400>  255

Glu Ile Val Leu Thr Gln Ser Pro Gly Thr Leu Ser Leu Ser Pro Gly
1               5                   10                  15

Glu Arg Ala Thr Phe Ser Cys Arg Ser Ser His Ser Ile Arg Ser Arg
            20                  25                  30

Arg Val Ala Trp Tyr Gln His Lys Pro Gly Gln Ala Pro Arg Leu Val
        35                  40                  45

Ile His Gly Val Ser Asn Arg Ala Ser Gly Ile Ser Asp Arg Phe Ser
    50                  55                  60

Gly Ser Gly Ser Gly Thr Asp Phe Thr Leu Thr Ile Thr Arg Val Glu
65                  70                  75                  80

Pro Glu Asp Phe Ala Leu Tyr Tyr Cys Gln Val Tyr Gly Ala Ser Ser
                85                  90                  95

Tyr Thr Phe Gly Gln Gly Thr Lys Leu Glu Arg Lys
            100                 105
```

## Claims

1. An agent for suppressing expression of NPC1L1 and/or LIPG mRNA, comprising, as an active ingredient, a compound having activity of inhibiting cholesteryl ester transfer protein, or a salt thereof, or a solvate thereof.

2. The agent for suppressing expression according to claim 1, wherein the compound having activity of inhibiting cholesteryl ester transfer protein, or a salt thereof, or a solvate thereof is a compound represented by the following formula (I) or (II), or a salt thereof, or a solvate thereof:

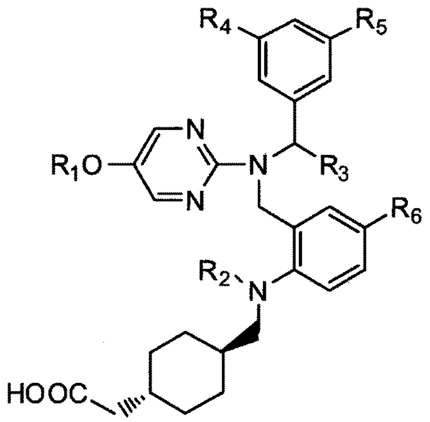

(I)

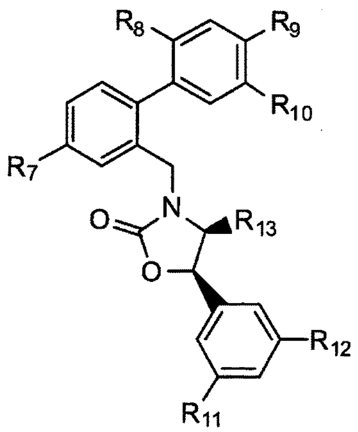

(II)

wherein, in the formula (I),

$R_1$ represents a $C_{1-6}$ alkylthio $C_{1-6}$ alkyl group, a $C_{1-6}$ alkylsulfinyl $C_{1-6}$ alkyl group or a $C_{1-6}$ alkylsulfonyl $C_{1-6}$ alkyl group,
$R_2$ represents a hydrogen atom or an optionally halogen atom-substituted $C_{1-6}$ alkyl group,
$R_3$ represents an optionally halogen atom-substituted $C_{1-6}$ alkyl group,
$R_4$ and $R_5$, which are the same or different, each represent a hydrogen atom, a halogen atom, an optionally halogen atom-substituted $C_{1-6}$ alkyl group, an optionally halogen atom-substituted $C_{1-6}$ alkoxy group, or a cyano group, and
$R_6$ represents a hydrogen atom, a halogen atom, an optionally halogen atom-substituted $C_{1-6}$ alkyl group, or an optionally halogen atom-substituted $C_{1-6}$ alkoxy group, and
in the formula (II),
$R_7$, $R_8$, $R_9$, $R_{10}$, $R_{11}$, $R_{12}$ and $R_{13}$, which are the same or different, each represent a hydrogen atom, a hydroxy group, a halogen atom, an optionally halogen atom-substituted $C_{1-6}$ alkyl group, or an optionally halogen atom-substituted $C_{1-6}$ alkoxy group.

3. The agent for suppressing expression according to claim 1, comprising, as an active ingredient, at least one compound selected from the group consisting of the following compounds, or a salt thereof, or a solvate thereof: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

4. The agent for suppressing expression according to claim 1, comprising, as an active ingredient, (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one, or a salt thereof, or a solvate thereof.

5. An agent for suppressing NPC1L1 and/or LIPG protein production, comprising, as an active ingredient, a compound having activity of inhibiting cholesteryl ester transfer protein, or a salt thereof, or a solvate thereof.

**6.** The agent for suppressing protein production according to claim 5, wherein the compound having activity of inhibiting cholesteryl ester transfer protein, or a salt thereof, or a solvate thereof is a compound represented by the following formula (I) or (II), or a salt thereof, or a solvate thereof:

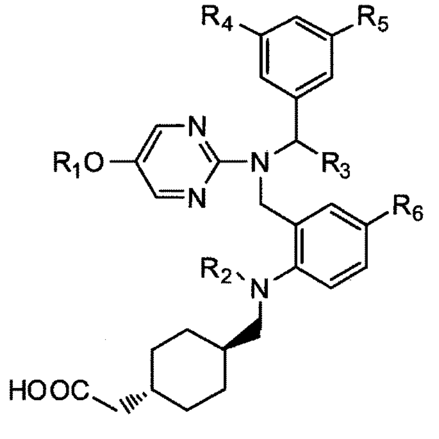

(I)

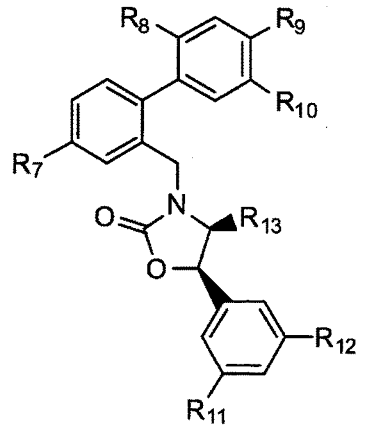

(II)

wherein, in the formula (I),

$R_1$ represents a $C_{1-6}$ alkylthio $C_{1-6}$ alkyl group, a $C_{1-6}$ alkylsulfinyl $C_{1-6}$ alkyl group or a $C_{1-6}$ alkylsulfonyl $C_{1-6}$ alkyl group,
$R_2$ represents a hydrogen atom or an optionally halogen atom-substituted $C_{1-6}$ alkyl group,
$R_3$ represents an optionally halogen atom-substituted $C_{1-6}$ alkyl group,
$R_4$ and $R_5$, which are the same or different, each represent a hydrogen atom, a halogen atom, an optionally halogen atom-substituted $C_{1-6}$ alkyl group, an optionally halogen atom-substituted $C_{1-6}$ alkoxy group, or a cyano group, and
$R_6$ represents a hydrogen atom, a halogen atom, an optionally halogen atom-substituted $C_{1-6}$ alkyl group, or an optionally halogen atom-substituted $C_{1-6}$ alkoxy group, and
in the formula (II),
$R_7$, $R_8$, $R_9$, $R_{10}$, $R_{11}$, $R_{12}$ and $R_{13}$, which are the same or different, each represent a hydrogen atom, a hydroxy group, a halogen atom, an optionally halogen atom-substituted $C_{1-6}$ alkyl group, or an optionally halogen atom-substituted $C_{1-6}$ alkoxy group.

**7.** The agent for suppressing protein production according to claim 5, comprising, as an active ingredient, at least one compound selected from the group consisting of the following compounds, or a salt thereof, or a solvate thereof: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

**8.** The agent for suppressing protein production according to claim 5, comprising, as an active ingredient, (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one, or a salt thereof, or a solvate thereof.

**9.** An agent for preventing and/or treating obesity, comprising, as an active ingredient, a compound represented by the following formula (I), or a salt thereof, or a solvate thereof:

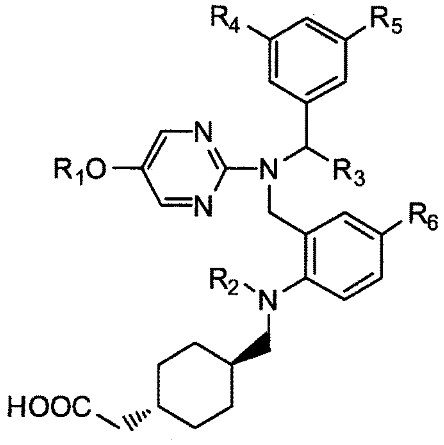

(I)

wherein, in the formula (I),

$R_1$ represents a $C_{1-6}$ alkylthio $C_{1-6}$ alkyl group, a $C_{1-6}$ alkylsulfinyl $C_{1-6}$ alkyl group or a $C_{1-6}$ alkylsulfonyl $C_{1-6}$ alkyl group,
$R_2$ represents a hydrogen atom or an optionally halogen atom-substituted $C_{1-6}$ alkyl group,
$R_3$ represents an optionally halogen atom-substituted $C_{1-6}$ alkyl group,
$R_4$ and $R_5$, which are the same or different, each represent a hydrogen atom, a halogen atom, an optionally halogen atom-substituted $C_{1-6}$ alkyl group, an optionally halogen atom-substituted $C_{1-6}$ alkoxy group, or a cyano group, and
$R_6$ represents a hydrogen atom, a halogen atom, an optionally halogen atom-substituted $C_{1-6}$ alkyl group, or an optionally halogen atom-substituted $C_{1-6}$ alkoxy group.

**10.** The agent for preventing and/or treating obesity according to claim 9, comprising, as an active ingredient, at least one compound selected from the group consisting of the following compounds, or a salt thereof, or a solvate thereof: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

**11.** A compound having activity of inhibiting cholesteryl ester transfer protein, or a salt thereof, or a solvate thereof, for suppressing expression of NPC1L1 and/or LIPG mRNA.

**12.** The compound according to claim 11, or a salt thereof, or a solvate thereof, wherein the compound having activity of inhibiting cholesteryl ester transfer protein, or a salt thereof, or a solvate thereof is a compound represented by the following formula (I) or (II), or a salt thereof, or a solvate thereof:

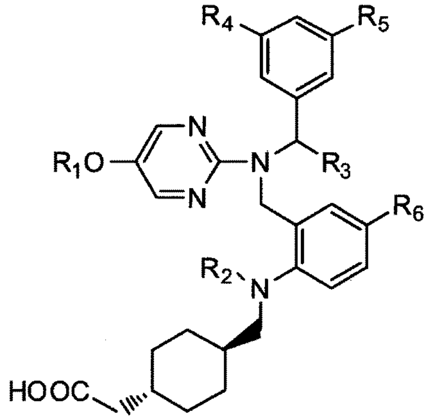

(I)

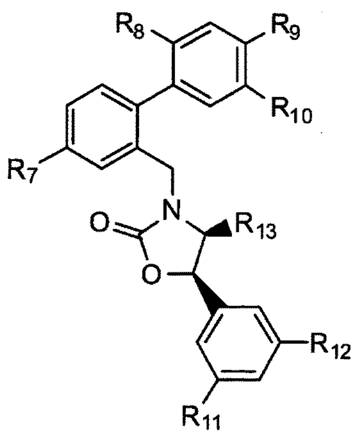

(II)

wherein, in the formula (I),

$R_1$ represents a $C_{1-6}$ alkylthio $C_{1-6}$ alkyl group, a $C_{1-6}$ alkylsulfinyl $C_{1-6}$ alkyl group or a $C_{1-6}$ alkylsulfonyl $C_{1-6}$ alkyl group,
$R_2$ represents a hydrogen atom or an optionally halogen atom-substituted $C_{1-6}$ alkyl group,
$R_3$ represents an optionally halogen atom-substituted $C_{1-6}$ alkyl group,
$R_4$ and $R_5$, which are the same or different, each represent a hydrogen atom, a halogen atom, an optionally halogen atom-substituted $C_{1-6}$ alkyl group, an optionally halogen atom-substituted $C_{1-6}$ alkoxy group, or a cyano group, and
$R_6$ represents a hydrogen atom, a halogen atom, an optionally halogen atom-substituted $C_{1-6}$ alkyl group, or an optionally halogen atom-substituted $C_{1-6}$ alkoxy group, and
in the formula (II),
$R_7$, $R_8$, $R_9$, $R_{10}$, $R_{11}$, $R_{12}$ and $R_{13}$, which are the same or different, each represent a hydrogen atom, a hydroxy group, a halogen atom, an optionally halogen atom-substituted $C_{1-6}$ alkyl group, or an optionally halogen atom-substituted $C_{1-6}$ alkoxy group.

**13.** The compound according to claim 11, or a salt thereof, or a solvate thereof, wherein the compound having activity of inhibiting cholesteryl ester transfer protein is at least one compound selected from the group consisting of: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

**14.** The compound according to claim 11, or a salt thereof, or a solvate thereof, wherein the compound having activity of inhibiting cholesteryl ester transfer protein is (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one.

**15.** A compound having activity of inhibiting cholesteryl ester transfer protein, or a salt thereof, or a solvate thereof, for suppressing NPC1L1 and/or LIPG protein production.

**16.** The compound according to claim 15, or a salt thereof, or a solvate thereof, wherein the compound having activity of inhibiting cholesteryl ester transfer protein, or a salt thereof, or a solvate thereof is a compound represented by the following formula (I) or (II), or a salt thereof, or a solvate thereof:

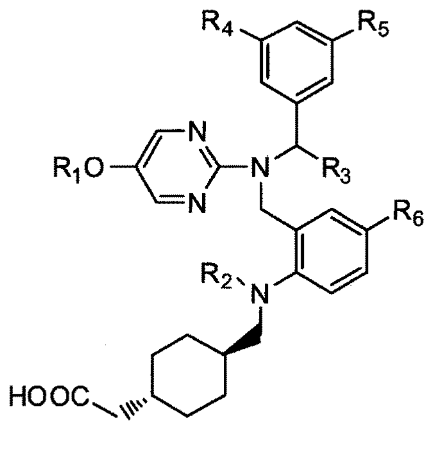

(I)

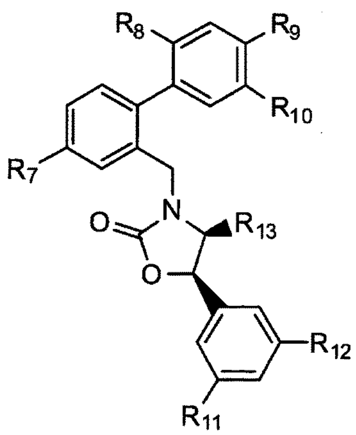

(II)

wherein, in the formula (I),

$R_1$ represents a $C_{1-6}$ alkylthio $C_{1-6}$ alkyl group, a $C_{1-6}$ alkylsulfinyl $C_{1-6}$ alkyl group or a $C_{1-6}$ alkylsulfonyl $C_{1-6}$ alkyl group,
$R_2$ represents a hydrogen atom or an optionally halogen atom-substituted $C_{1-6}$ alkyl group,
$R_3$ represents an optionally halogen atom-substituted $C_{1-6}$ alkyl group,
$R_4$ and $R_5$, which are the same or different, each represent a hydrogen atom, a halogen atom, an optionally halogen atom-substituted $C_{1-6}$ alkyl group, an optionally halogen atom-substituted $C_{1-6}$ alkoxy group, or a cyano group, and
$R_6$ represents a hydrogen atom, a halogen atom, an optionally halogen atom-substituted $C_{1-6}$ alkyl group, or an optionally halogen atom-substituted $C_{1-6}$ alkoxy group, and
in the formula (II),
$R_7$, $R_8$, $R_9$, $R_{10}$, $R_{11}$, $R_{12}$ and $R_{13}$, which are the same or different, each represent a hydrogen atom, a hydroxy group, a halogen atom, an optionally halogen atom-substituted $C_{1-6}$ alkyl group, or an optionally halogen atom-substituted $C_{1-6}$ alkoxy group.

**17.** The compound according to claim 15, or a salt thereof, or a solvate thereof, wherein the compound having activity of inhibiting cholesteryl ester transfer protein is at least one compound selected from the group consisting of: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

**18.** The compound according to claim 15, or a salt thereof, or a solvate thereof, wherein the compound having activity of inhibiting cholesteryl ester transfer protein is (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one.

**19.** A compound represented by the following formula (I), or a salt thereof, or a solvate thereof, for preventing and/or treating obesity:

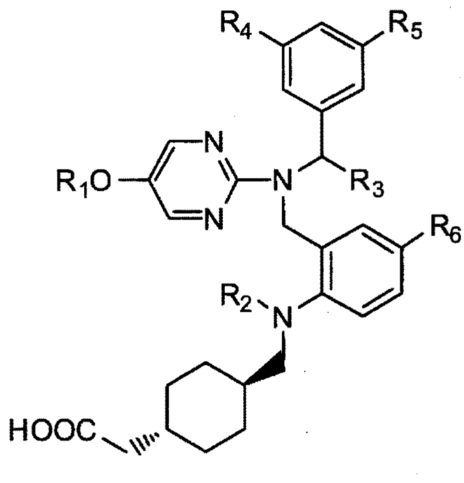

(I)

wherein $R^1$ to $R^6$ are the same as those described above.

**20.** The compound according to claim 19, or a salt thereof, or a solvate thereof, wherein a compound having activity of inhibiting cholesteryl ester transfer protein is at least one compound selected from the group consisting of the following compounds, or a salt thereof, or a solvate thereof: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

**21.** Use of a compound having activity of inhibiting cholesteryl ester transfer protein, or a salt thereof, or a solvate thereof, for manufacturing an agent for suppressing expression of NPC1L1 and/or LIPG mRNA.

**22.** The use according to claim 21, wherein the compound having activity of inhibiting cholesteryl ester transfer protein, or a salt thereof, or a solvate thereof is a compound represented by the following formula (I) or (II), or a salt thereof, or a solvate thereof:

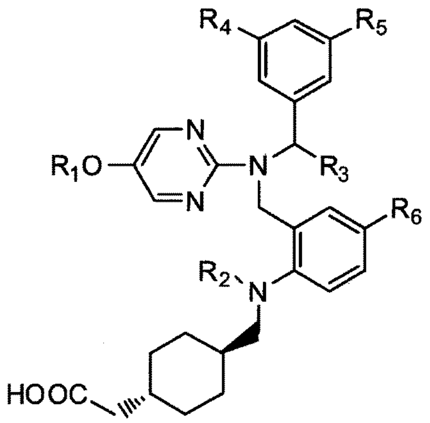

(I)

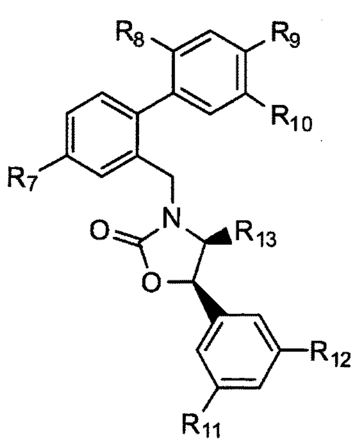

(II)

wherein, in the formula (I),

$R_1$ represents a $C_{1-6}$ alkylthio $C_{1-6}$ alkyl group, a $C_{1-6}$ alkylsulfinyl $C_{1-6}$ alkyl group or a $C_{1-6}$ alkylsulfonyl $C_{1-6}$ alkyl group,
$R_2$ represents a hydrogen atom or an optionally halogen atom-substituted $C_{1-6}$ alkyl group,
$R_3$ represents an optionally halogen atom-substituted $C_{1-6}$ alkyl group,
$R_4$ and $R_5$, which are the same or different, each represent a hydrogen atom, a halogen atom, an optionally halogen atom-substituted $C_{1-6}$ alkyl group, an optionally halogen atom-substituted $C_{1-6}$ alkoxy group, or a cyano group, and
$R_6$ represents a hydrogen atom, a halogen atom, an optionally halogen atom-substituted $C_{1-6}$ alkyl group, or an optionally halogen atom-substituted $C_{1-6}$ alkoxy group, and
in the formula (II),
$R_7$, $R_8$, $R_9$, $R_{10}$, $R_{11}$, $R_{12}$ and $R_{13}$, which are the same or different, each represent a hydrogen atom, a hydroxy group, a halogen atom, an optionally halogen atom-substituted $C_{1-6}$ alkyl group, or an optionally halogen atom-substituted $C_{1-6}$ alkoxy group.

**23.** The use according to claim 21, wherein the compound having activity of inhibiting cholesteryl ester transfer protein is at least one compound selected from the group consisting of: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

**24.** The use according to claim 21, wherein the compound having activity of inhibiting cholesteryl ester transfer protein is (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one.

**25.** Use of a compound having activity of inhibiting cholesteryl ester transfer protein, or a salt thereof, or a solvate thereof, for manufacturing an agent for suppressing NPC1L1 and/or LIPG protein production.

**26.** The use according to claim 25, wherein the compound having activity of inhibiting cholesteryl ester transfer protein, or a salt thereof, or a solvate thereof is a compound represented by the following formula (I) or (II), or a salt thereof, or a solvate thereof:

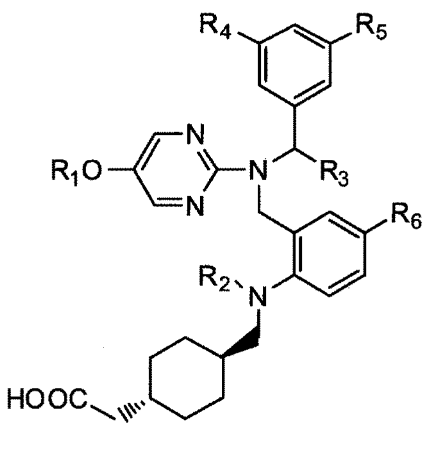

(I)

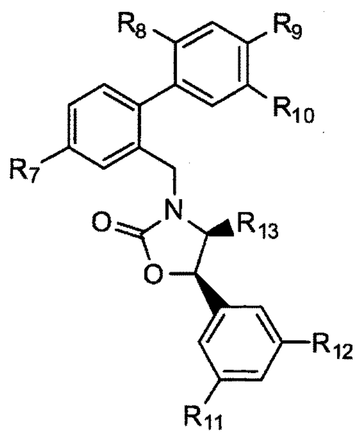

(II)

wherein, in the formula (I),

$R_1$ represents a $C_{1-6}$ alkylthio $C_{1-6}$ alkyl group, a $C_{1-6}$ alkylsulfinyl $C_{1-6}$ alkyl group or a $C_{1-6}$ alkylsulfonyl $C_{1-6}$ alkyl group,

$R_2$ represents a hydrogen atom or an optionally halogen atom-substituted $C_{1-6}$ alkyl group,
$R_3$ represents an optionally halogen atom-substituted $C_{1-6}$ alkyl group,
$R_4$ and $R_5$, which are the same or different, each represent a hydrogen atom, a halogen atom, an optionally halogen atom-substituted $C_{1-6}$ alkyl group, an optionally halogen atom-substituted $C_{1-6}$ alkoxy group, or a cyano group, and
$R_6$ represents a hydrogen atom, a halogen atom, an optionally halogen atom-substituted $C_{1-6}$ alkyl group, or an optionally halogen atom-substituted $C_{1-6}$ alkoxy group, and
in the formula (II),
$R_7$, $R_8$, $R_9$, $R_{10}$, $R_{11}$, $R_{12}$ and $R_{13}$, which are the same or different, each represent a hydrogen atom, a hydroxy group, a halogen atom, an optionally halogen atom-substituted $C_{1-6}$ alkyl group, or an optionally halogen atom-substituted $C_{1-6}$ alkoxy group.

**27.** The use according to [25] above, wherein the compound having activity of inhibiting cholesteryl ester transfer protein is at least one compound selected from the group consisting of: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

**28.** The use according to claim 25, wherein the compound having activity of inhibiting cholesteryl ester transfer protein is (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one.

**29.** Use of a compound represented by the following formula (I), or a salt thereof, or a solvate thereof, for manufacturing an agent for preventing and/or treating obesity:

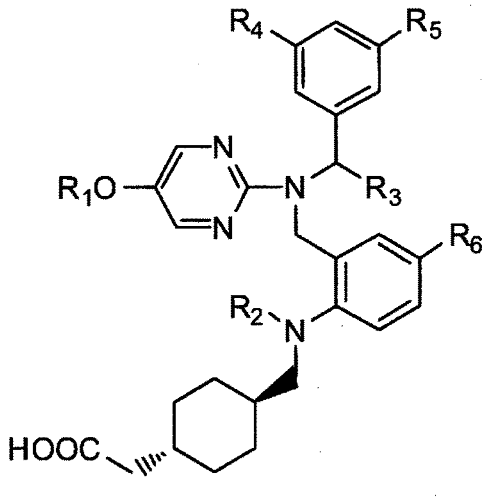

(I)

wherein $R_1$ to $R_6$ are the same as those described above.

**30.** The use according to claim 29, wherein the compound represented by the formula (I) is at least one compound selected from the group consisting of: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

**31.** A method for suppressing expression of NPC1L1 and/or LIPG mRNA, comprising administering a compound having activity of inhibiting cholesteryl ester transfer protein, or a salt thereof, or a solvate thereof.

**32.** The method for suppressing expression according to claim 31, wherein the compound having activity of inhibiting cholesteryl ester transfer protein or a salt thereof, or a solvate thereof is a compound represented by the following formula (I) or (II), or a salt thereof, or a solvate thereof:

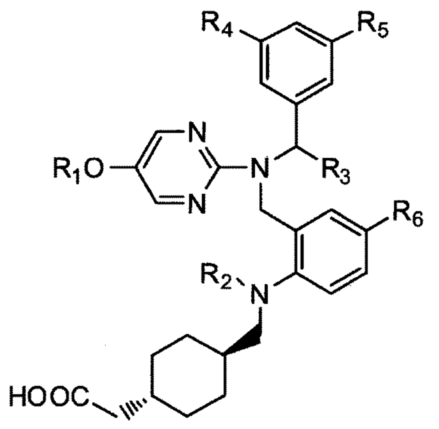

(I)

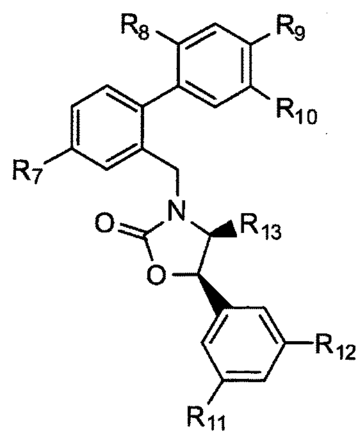

(II)

wherein, in the formula (I),

$R_1$ represents a $C_{1-6}$ alkylthio $C_{1-6}$ alkyl group, a $C_{1-6}$ alkylsulfinyl $C_{1-6}$ alkyl group or a $C_{1-6}$ alkylsulfonyl $C_{1-6}$ alkyl group,
$R_2$ represents a hydrogen atom or an optionally halogen atom-substituted $C_{1-6}$ alkyl group,
$R_3$ represents an optionally halogen atom-substituted $C_{1-6}$ alkyl group,
$R_4$ and $R_5$, which are the same or different, each represent a hydrogen atom, a halogen atom, an optionally halogen atom-substituted $C_{1-6}$ alkyl group, an optionally halogen atom-substituted $C_{1-6}$ alkoxy group, or a cyano group, and
$R_6$ represents a hydrogen atom, a halogen atom, an optionally halogen atom-substituted $C_{1-6}$ alkyl group, or an optionally halogen atom-substituted $C_{1-6}$ alkoxy group, and
in the formula (II),
$R_7$, $R_8$, $R_9$, $R_{10}$, $R_{11}$, $R_{12}$ and $R_{13}$, which are the same or different, each represent a hydrogen atom, a hydroxy group, a halogen atom, an optionally halogen atom-substituted $C_{1-6}$ alkyl group, or an optionally halogen atom-substituted $C_{1-6}$ alkoxy group.

**33.** The method for suppressing expression according to claim 31, comprises administering at least one compound selected from the group consisting of the following compounds, or a salt thereof, or a solvate thereof: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

**34.** The method for suppressing expression according to claim 31, wherein the method comprises administering (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one, or a salt thereof, or a solvate thereof.

**35.** A method for suppressing NPC1L1 and/or LIPG protein production, comprising administering a compound having activity of inhibiting cholesteryl ester transfer protein, or a salt thereof, or a solvate thereof.

**36.** The method for suppressing protein production according to claim 35, wherein the compound having activity of

inhibiting cholesteryl ester transfer protein, or a salt thereof, or a solvate thereof is a compound represented by the following formula (I) or (II), or a salt thereof, or a solvate thereof:

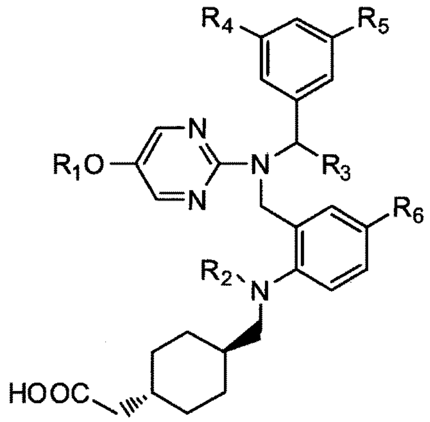

(I)

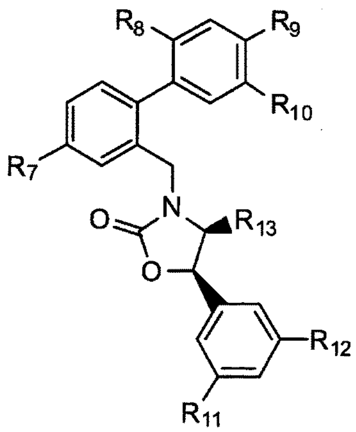

(II)

wherein, in the formula (I),

$R_1$ represents a $C_{1-6}$ alkylthio $C_{1-6}$ alkyl group, a $C_{1-6}$ alkylsulfinyl $C_{1-6}$ alkyl group or a $C_{1-6}$ alkylsulfonyl $C_{1-6}$ alkyl group,
$R_2$ represents a hydrogen atom or an optionally halogen atom-substituted $C_{1-6}$ alkyl group,
$R_3$ represents an optionally halogen atom-substituted $C_{1-6}$ alkyl group,
$R_4$ and $R_5$, which are the same or different, each represent a hydrogen atom, a halogen atom, an optionally halogen atom-substituted $C_{1-6}$ alkyl group, an optionally halogen atom-substituted $C_{1-6}$ alkoxy group, or a cyano group, and
$R_6$ represents a hydrogen atom, a halogen atom, an optionally halogen atom-substituted $C_{1-6}$ alkyl group, or an optionally halogen atom-substituted $C_{1-6}$ alkoxy group, and
in the formula (II),
$R_7$, $R_8$, $R_9$, $R_{10}$, $R_{11}$, $R_{12}$ and $R_{13}$, which are the same or different, each represent a hydrogen atom, a hydroxy group, a halogen atom, an optionally halogen atom-substituted $C_{1-6}$ alkyl group, or an optionally halogen atom-substituted $C_{1-6}$ alkoxy group.

**37.** The method for suppressing protein production according to claim 35, wherein the method comprises administering at least one compound selected from the group consisting of the following compounds, or a salt thereof, or a solvate thereof: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

**38.** The method for suppressing protein production according to claim 35, wherein the method comprises administering (4S,5R)-5-[3,5-bis(trifluoromethyl)phenyl]-3-((4'-fluoro-5'-isopropyl-2'-methoxy-4-(trifluoromethyl)-[1,1'-biphenyl]-2-yl)methyl)-4-methyloxazolidin-2-one, or a salt thereof, or a solvate thereof.

**39.** A method for preventing and/or treating obesity, comprising administering a compound represented by the following formula (I), or a salt thereof, or a solvate thereof:

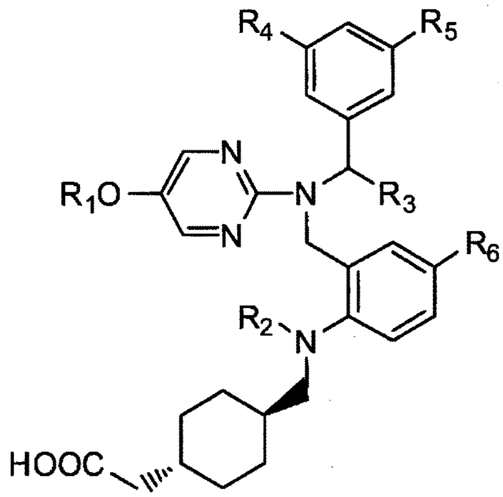

(I)

wherein $R_1$ to $R_6$ are the same as those described above.

**40.** The method for preventing and/or treating obesity according to claim 39, wherein the method comprises administering at least one compound selected from the group consisting of the following compounds, or a salt thereof, or a solvate thereof: trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, (S)-(-)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid, and (R)-(+)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl}(ethyl)amino)methyl]cyclohexyl}ac etic acid.

[Figure 1]

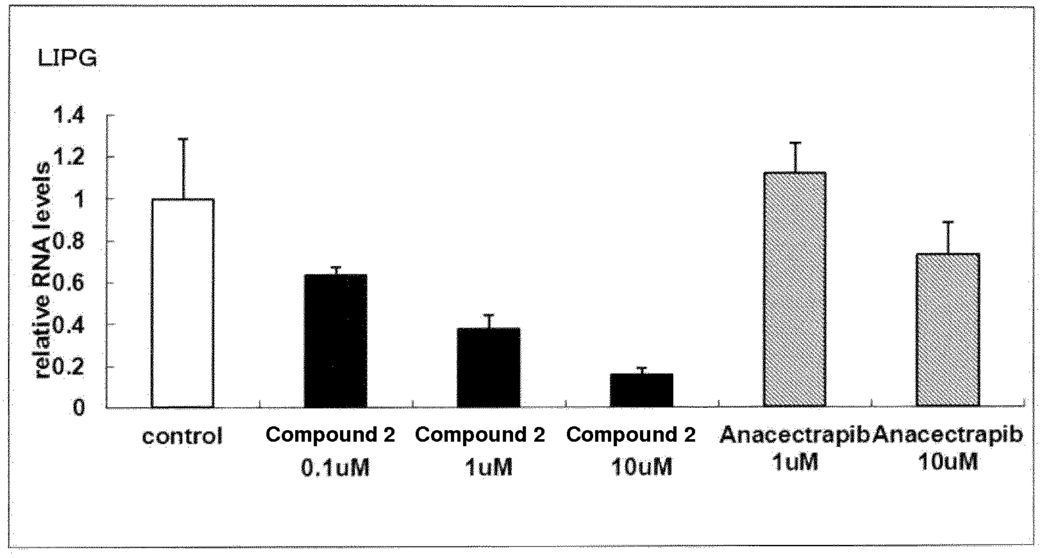


[Figure 2]

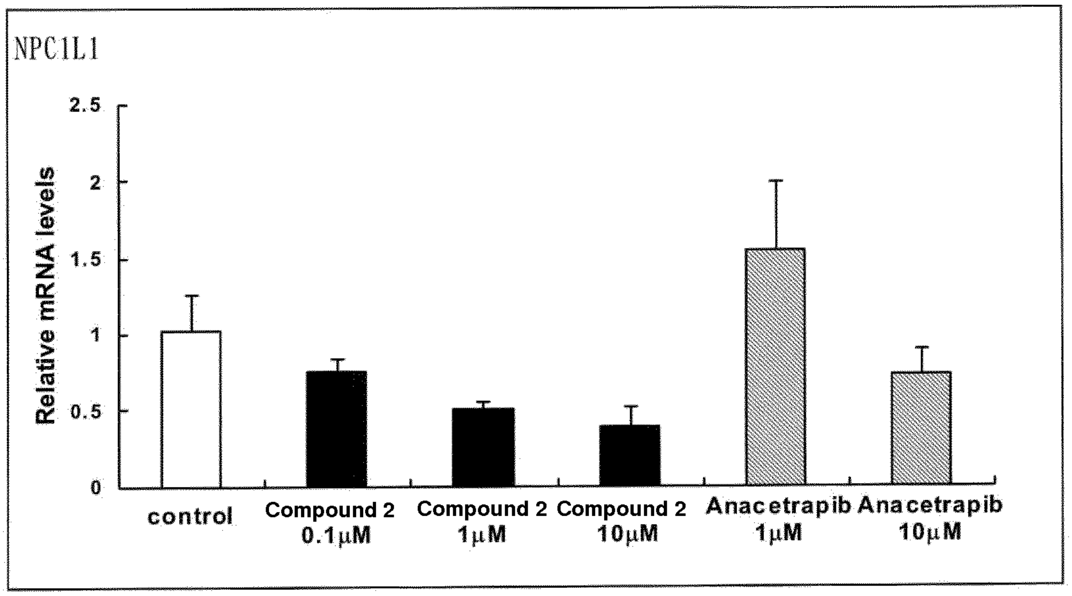

Sequence Listing

<110>  KOWA COMPANY, LTD.

<120>  An expression inhibitor of NPC1L1 and/or LIPG mRNA, and preventive and/or therapeutic agent for obesity

<130>  KW0263

<150>  JP2011-260334
<151>  2011-11-29

<160>  4

<170>  PatentIn version 3.1

<210>  1
<211>  22
<212>  DNA
<213>  Artificial sequence

<220>
<223>  Designed DNA based on human NPC1L1 gene

<400>  1
caggtatggt cgcccgaagc ac
22

<210> 2

<211> 22

<212> DNA

<213> Artificial sequence

<220>

<223> Designed DNA based on human NPC1L1 gene

<400> 2

tgcggttgtt ctggaaatac tg

22

<210> 3

<211> 20

<212> DNA

<213> Artificial sequence

<220>

<223> Designed DNA based on human L1PG gene

<400> 3

tcaacgatgt cttgggatca

20

<210> 4

<211> 20

<212> DNA

<213> Artificial sequence

<220>

<223> Designed DNA based on human L1PG gene

<400> 4

tgaagcgatt ggagtcagtg

20

**INTERNATIONAL SEARCH REPORT**

International application No.
PCT/JP2012/080731

A. CLASSIFICATION OF SUBJECT MATTER
*A61K45/00*(2006.01)i, *A61K31/421*(2006.01)i, *A61K31/505*(2006.01)i, *A61P3/04*(2006.01)i, *A61P43/00*(2006.01)i, *C07D239/47*(2006.01)n, *C07D263/24*(2006.01)n, *C12N15/09*(2006.01)n

According to International Patent Classification (IPC) or to both national classification and IPC

B. FIELDS SEARCHED

Minimum documentation searched (classification system followed by classification symbols)
A61K45/00, A61K31/421, A61K31/505, A61P3/04, A61P43/00, C07D239/47, C07D263/24, C12N15/09

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X | WO 2008/129951 A1 (Kowa Co., Ltd.), 30 October 2008 (30.10.2008), entire text & US 2009/0082352 A1 & EP 2149563 A1 & CN 101679309 A & CA 2683534 A & KR 10-2010-0016332 A & TW 200906804 A | 1-30 |
| X | WO 2006/014357 A1 (MERK & CO., INC.), 09 February 2006 (09.02.2006), entire text & JP 2008-505120 A & US 2006/0040999 A1 & EP 1765793 A & CA 2570717 A & KR 10-2007-0039523 A & CN 1980904 A | 1-30 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 February, 2013 (21.02.13) | 05 March, 2013 (05.03.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/080731

C (Continuation). DOCUMENTS CONSIDERED TO BE RELEVANT

| Category* | Citation of document, with indication, where appropriate, of the relevant passages | Relevant to claim No. |
|---|---|---|
| A | WO 2008/018529 A1 (Kowa Co., Ltd.), 14 February 2008 (14.02.2008), entire text & US 2009/0054474 A1 & EP 2055703 A1 | 1-30 |
| P,X | WO 2012/046681 A1 (Kowa Co., Ltd.), 12 April 2012 (12.04.2012), entire text (Family: none) | 1-30 |

Form PCT/ISA/210 (continuation of second sheet) (July 2009)

**INTERNATIONAL SEARCH REPORT**

International application No.
PCT/JP2012/080731

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 31-40
because they relate to subject matter not required to be searched by this Authority, namely:
Claims 31-40 involve a step of administering a compound or the like and therefore relate to a method for the treatment of the human body by therapy. Thus, the inventions of these claims relate to a subject matter for which the international search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004505637 A
- WO 2007037299 A
- WO 2000017164 A
- WO 2006014357 A
- WO 1998035937 A
- WO 2004055162 A
- WO 99041237 A
- WO 00018721 A
- WO 05097805 A
- WO 06073973 A
- WO 08079427 A
- WO 08129951 A
- WO 08156718 A
- WO 09007259 A
- WO 06014357 A
- WO 2008129951 A


### Non-patent literature cited in the description


- *Himan to Yobo no Keizaigaku,* 23 September 2010, http://www.oecdtokyo2.org/pdf/theme_pdf/health_pdf/201009 23fit.pdf
- *EMBO J.,* 1993, vol. 12, 2219-28
- *J. Cell. Biol.,* 1993, vol. 120, 1123-35
- *J. Cell. Biol.,* 1994, vol. 125, 253-268
- *Science,* 1996, vol. 272, 227-34
- *Science,* 1997, vol. 277, 228-31
- *Genomics,* 2000, vol. 65 (2), 137
- *J. Atheroscler. Thromb.,* 2010, vol. 17 (4), 356
- *J. Lipid Res.,* 2010, vol. 51 (10), 3024-33
- *J. Biol. Chem.,* 1999, vol. 274, 14170
- *Nat. Genet.,* 1999, vol. 21, 424
- *Biochem. Biophys. Res. Commun.,* 2000, vol. 272, 90
- *Proc Natl Acad Sci USA,* 2003, vol. 100 (5), 2748
- *J Clin Invest,* 2003, vol. 111 (3), 347
- *Atherosclerosis,* 2009, vol. 10 (2), 612
- *J. Lipid Res.,* 2011, vol. 52 (1), 57
- *Bioorganic & Medicinal Chemistry Letters,* 1996, vol. 6, 1951-1954
- *Bioorganic & Medicinal Chemistry Letters,* 2005, vol. 15, 3611-3614
- *Bioorganic & Medicinal Chemistry Letters,* 2007, vol. 17, 2608-2613
- Progress in Medicine. *Lifescience Medica,* 1985, vol. 5, 2157-2161
- Iyakuhin no Kaihatsu. Bunshi Sekkei. Hirokawa Shoten, 1990, vol. 7, 163-198